(19) Europäisches Patentamt
European Patent Office
Office européen des brevets

(11) EP 3 430 016 B1

(12) EUROPEAN PATENT SPECIFICATION

(45) Date of publication and mention of the grant of the patent:
**05.01.2022 Bulletin 2022/01**

(21) Application number: **17709111.3**

(22) Date of filing: **10.03.2017**

(51) Int Cl.:
*C07D 513/04* (2006.01) *C08G 61/12* (2006.01)
*C08L 65/00* (2006.01) *C09D 165/00* (2006.01)
*H01L 51/00* (2006.01) *H01L 51/42* (2006.01)

(86) International application number:
**PCT/EP2017/055638**

(87) International publication number:
**WO 2017/157782 (21.09.2017 Gazette 2017/38)**

# (54) ORGANIC SEMICONDUCTORS

ORGANISCHE HALBLEITER

SEMICONDUCTEURS ORGANIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2016 EP 16160490**

(43) Date of publication of application:
**23.01.2019 Bulletin 2019/04**

(73) Proprietor: **Raynergy Tek Inc.**
**Hsinchu 30075 (TW)**

(72) Inventors:
- **NANSON, Lana**
  **Southampton SO16 3TZ (GB)**
- **PRON, Agnieszka**
  **Eastleigh SO50 9LG (GB)**
- **KROMPIEC, Michal**
  **Southampton SO15 4GY (GB)**

(74) Representative: **Lawrie IP Limited**
**310 St. Vincent Street**
**Glasgow G2 5RG (GB)**

(56) References cited:
**WO-A1-2015/122321 WO-A1-2016/121589**
**WO-A1-2016/125822 WO-A2-2011/098113**
**JP-A- 2007 238 530**

- **KIM I T ET AL: "Synthesis, characterization, and properties of a new thiophene-benzobisthiazole copolymer", SYNTHETIC METALS, ELSEVIER SEQUOIA, LAUSANNE, CH, vol. 156, no. 1, 5 January 2006 (2006-01-05), pages 38-41, XP027940190, ISSN: 0379-6779 [retrieved on 2006-01-05]**
- **IN TAE KIM ET AL: "Synthesis, characterization and properties of poly(p-phenylene) derivative", POLYMER PREPRINTS, vol. 44, no. 1, 18 March 2003 (2003-03-18) , pages 931-932, XP055377676, US ISSN: 0032-3934**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 430 016 B1

## Description

### Technical Field

The invention relates to novel compounds containing one or more benzo[1,2-d;4,5-d']bisthiazole-4,8-diyl ("BBT") units, to methods for their preparation and educts or intermediates used therein, to mixtures and formulations containing them, to the use of the compounds, mixtures and formulations as organic semiconductors in organic electronic (OE) devices, especially in organic photovoltaic (OPV) devices and organic photodetectors (OPD), and to OE, OPV and OPD devices comprising these compounds, mixtures or formulations.

### Background

In recent years, there has been development of organic semiconducting (OSC) materials in order to produce more versatile, lower cost electronic devices. Such materials find application in a wide range of devices or apparatus, including organic field effect transistors (OFETs), organic light emitting diodes (OLEDs), organic photodetectors (OPDs), organic photovoltaic (OPV) cells, sensors, memory elements and logic circuits to name just a few. The organic semiconducting materials are typically present in the electronic device in the form of a thin layer.

The performance of OFET devices is principally based upon the charge carrier mobility of the semiconducting material and the current on/off ratio, so the ideal semiconductor should have a low conductivity in the off state, combined with a high charge carrier mobility (> $1 \times 10^{-3}$ $cm^2$ $V^{-1}$ $s^{-1}$). In addition, it is important that the semiconducting material is relatively stable to oxidation i.e. it has a high ionisation potential, as oxidative doping leads to reduced device performance, for example increased off current and threshold voltage shift. Further requirements for the semiconducting material to have include good processability, especially for large-scale production of thin-film layers and desired patterns, and high stability, thin-film uniformity and integrity of the organic semiconductor layer.

Another particular area of importance is organic photovoltaics (OPV). Conjugated polymers have found use in OPVs as they allow devices to be manufactured by solution-processing techniques such as spin casting, dip coating or ink jet printing. Solution processing can be carried out cheaper and on a larger scale compared to the evaporative techniques used to make inorganic thin film devices. Currently, polymer based photovoltaic devices are achieving efficiencies over 10%.

The conjugated polymer serves as the main absorber of the solar energy in the bulk-heterojunction blend layer and therefore a low band gap is a basic requirement of the ideal polymer design to absorb the maximum of the solar spectrum.

A commonly used strategy to narrow the band gap of polymers is to utilize an alternating copolymer consisting of both electron rich donor units and electron deficient acceptor units within the polymer backbone. However, the ideal polymer which combines high efficiency, facile synthesis and scalable has yet to be found.

Thus there is still a need for organic semiconducting (OSC) polymers which are easy to synthesize, especially by methods suitable for mass production, show good structural organization and film-forming properties, exhibit good electronic properties, especially a high charge carrier mobility, a good processibility, especially a high solubility in organic solvents, and high stability in air. Especially for use in OPV cells, there is a need for OSC materials having a low bandgap, which enable improved light harvesting by the photoactive layer and can lead to higher cell efficiencies, compared to the polymers from prior art.

It was an aim of the present invention to provide compounds for use as organic semiconducting materials in OE devices like OFETs, OPDs and OPV devices, which are easy to synthesize, especially by methods suitable for mass production, which show especially good processibility, high stability, good solubility in organic solvents, high charge carrier mobility, and a low bandgap. Another aim of the invention was to extend the pool of OSC materials available to the expert. Other aims of the present invention are immediately evident to the expert from the following detailed description.

The inventors of the present invention have found that one or more of the above aims can be achieved by providing compounds having a divalent unit derived from benzo[1,2-d;4,5-d']bisthiazole-4,8-diyl (hereinafter also referred to as "BBT").

JP2007238530 discloses a pi-conjugated organic compound which contains BBT units connected by ethynylene groups, and its use in electronic materials.

KR2010088764 discloses a conjugated trimer containing an alkylated BBT group and two ethylenedioxythiophene groups, and its use in OLEDs.

WO2011/098113 A1 discloses a polymer comprising units of the following formula

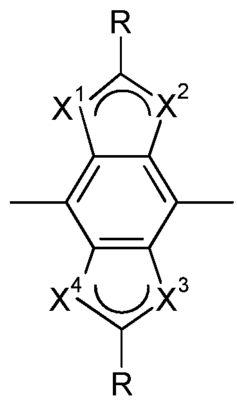

wherein one of $X^1$ and $X^2$ is selected from S, Se and O, and the other is selected from CH, $CR^x$ and N, and one of $X^3$ and $X^4$ is selected from S, Se and O, and the other is selected from CH, $CR^x$ and N.

WO2012/156022 A1 and WO2013/013765 A1 disclose copolymers with co-units that can be selected from a list of preferred donor units including also benzobisthiazole. Kim et al., Synth. Met. 2006, 156(1), 38-41, WO 2015/122321 A1, Kim et al., Pol. Preprints 2003, 44(1), 931-932, WO 2016/125822 A1 and WO 2016/121589 A1 do also disclose small molecules or polymers comprising a benzobisthiazole unit.

However, the specific compounds as disclosed and claimed hereinafter and their use as organic semiconductors have not been explicitly disclosed or suggested in prior art so far.

## Summary

The invention relates to a compound comprising one or more divalent heteroarylene units of formula I

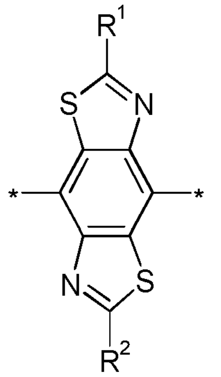

I

wherein the individual radicals, independently of each other and on each occurrence identically or differently, have the following meanings

$R^1$, $R^2$ are selected from the group consisting of -C(=O)-R, -C(=O)-OR,-OC(=O)-R, -C(=O)-NHR and -C(=O)-NRR$^n$, wherein R and R$^n$ are independently of each other straight-chain or branched alkyl with 1 to 20 C atoms, which is unsubstituted or substituted by one or more F atoms

L F, Cl, -CN, -NC, -NCO, -NCS, -OCN, -SCN, $-R^0$, $-OR^0$, $-SR^0$, $-C(=O)X^0$,$-C(=O)R^0$, $-C(=O)-OR^0$, $-O-C(=O)R^0$, $-NH_2$, $-NHR^0$, $-NR^0R^{00}$, $-C(=O)NHR^0$,$-C(=O)NR^0R^{00}$, -SOsH, $-SO_2R^0$, -OH, $-NO_2$, $-CF_3$, $-SF_5$, or optionally substituted silyl, or carbyl or hydrocarbyl with 1 to 20 C atoms that is optionally substituted and optionally comprises one or more hetero atoms, preferably F, -CN, R, -OR, -SR, -C(=O)-R, -C(=O)-OR, -O-C(=O)-R, -O-C(=O)-OR, -C(=O)-NHR, -C(=O)-NRR$^n$,

$R^0$, $R^{00}$ H or straight-chain or branched alkyl with 1 to 20, preferably 1 to 12 C atoms that is optionally fluorinated,

and further comprising one or more arylene or heteroarylene units that have from 5 to 20 ring atoms, are mono- or polycyclic, do optionally contain fused rings, are unsubstituted or substituted by one or more identical or different groups

L, and are either selected of formula I or are structurally different from formula I, wherein all the aforementioned units are directly connected to each other, and wherein the compound is a conjugated polymer.

The invention relates to a compound as described above and below which is a conjugated polymer. The conjugated polymer comprises, preferably consists of, one or more units of formula I and one or more arylene or heteroarylene units that have from 5 to 20 ring atoms, are mono- or polycyclic, do optionally contain fused rings, are unsubstituted or substituted by one or more identical or different groups L, and are either selected of formula I or are structurally different from formula I, and wherein all the aforementioned units are directly connected to each other.

The invention further relates to a compound as described above and below which is an oligomer.

The invention further relates to a conjugated polymer comprising one or more electron acceptor repeating units comprising a unit of formula I, and preferably further comprising one or more repeating units having electron donor property.

The invention further relates to the use of a compound as described above and below as electron acceptor or n-type semiconductor.

The invention further relates to the use of a conjugated polymer as described above and below as semiconductor, preferably as electron donor or p-type semiconductor.

The invention further relates to the use of a compound as described above and below as electron donor or electron acceptor component in a semiconducting material, formulation, polymer blend, device or component of a device.

The invention further relates to a semiconducting material, formulation, polymer blend, device or component of a device comprising a compound as described above and below as electron donor component, and preferably further comprising one or more compounds having electron acceptor properties.

The invention further relates to a mixture, which may also be a polymer blend, comprising one or more compounds as described above and below, and further comprising one or more additional compounds selected from compounds having one or more of semiconducting, charge transport, hole or electron transport, hole or electron blocking, electrically conducting, photoconducting or light emitting properties.

The invention further relates to a mixture comprising one or more compounds as described above and below, and further comprising one or more n-type organic semiconductors, preferably selected from fullerenes or substituted fullerenes.

The invention further relates to a formulation comprising one or more compounds or mixtures as described above and below, and further comprising one or more solvents, preferably selected from organic solvents.

The invention further relates to an organic semiconducting formulation comprising one or more compounds as described above and below, and further comprising one or more organic binders or precursors thereof, preferably having a permittivity ε at 1,000 Hz and 20°C of 3.3 or less, and optionally one or more solvents preferably selected from organic solvents.

The invention further relates to an optical, electrooptical, electronic, electroluminescent or photoluminescent device, or a component thereof, or an assembly comprising it, which is prepared using a formulation according to the present invention.

The invention further relates to the use of a compound or mixture as described above and below as semiconducting, charge transport, electrically conducting, photoconducting or light emitting material, or in an optical, electrooptical, electronic, electroluminescent or photoluminescent device, or in a component of such a device or in an assembly comprising such a device or component

The invention further relates to a semiconducting, charge transport, electrically conducting, photoconducting or light emitting material comprising a compound or mixture as described above and below.

The invention further relates to an optical, electrooptical, electronic, electroluminescent or photoluminescent device, or a component thereof, or an assembly comprising it, which comprises a compound or mixture as described above and below, or comprises a semiconducting, charge transport, electrically conducting, photoconducting or light emitting material as described above and below.

The optical, electrooptical, electronic, electroluminescent and photoluminescent device includes, without limitation, organic field effect transistors (OFET), organic thin film transistors (OTFT), organic light emitting diodes (OLED), organic light emitting transistors (OLET), organic photovoltaic devices (OPV), organic photodetectors (OPD), organic solar cells, dye-sensitized solar cells (DSSC), perovskite-based solar cells, laser diodes, Schottky diodes, photoconductors and photodetectors.

Preferred devices are OFETs, OTFTs, OPVs, OPDs and OLEDs, in particular bulk heterojunction (BHJ) OPVs or inverted BHJ OPVs.

Further preferred is the use of a compound or mixture as described above and below as dye in a DSSC or a perovskite-based solar cell. Further preferred is a DSSC or perovskite-based solar cells comprising a compound or mixture as described above and below.

The component of the above devices includes, without limitation, charge injection layers, charge transport layers, interlayers, planarising layers, antistatic films, polymer electrolyte membranes (PEM), conducting substrates and

conducting patterns.

The assembly comprising such a device or component includes, without limitation, integrated circuits (IC), radio frequency identification (RFID) tags or security markings or security devices containing them, flat panel displays or backlights thereof, electrophotographic devices, electrophotographic recording devices, organic memory devices, sensor devices, biosensors and biochips.

In addition the compounds, mixtures and formulations of the present invention can be used as electrode materials in batteries and in components or devices for detecting and discriminating DNA sequences.

The invention further relates to a bulk heterojunction which comprises, or is being formed from, a mixture comprising one or more compounds according to the present invention and one or more n-type organic semiconductors that are preferably selected from fullerenes or substituted fullerenes. The invention further relates to a bulk heterojunction (BHJ) OPV device or inverted BHJ OPV device, comprising such a bulk heterojunction.

## Terms and Definitions

As used herein, the term "polymer" will be understood to mean a molecule of high relative molecular mass, the structure of which essentially comprises multiple repetitions of units derived, actually or conceptually, from molecules of low relative molecular mass (Pure Appl. Chem., 1996, 68, 2291). The term "oligomer" will be understood to mean a molecule of intermediate relative molecular mass, the structure of which essentially comprises a small plurality of units derived, actually or conceptually, from molecules of lower relative molecular mass (Pure Appl. Chem., 1996, 68, 2291). In a preferred meaning as used herein present invention a polymer will be understood to mean a compound having $>1$, i.e. at least 2 repeat units, preferably $\geq 5$ repeat units, and an oligomer will be understood to mean a compound with $>1$ and $<10$, preferably $<5$, repeat units.

Further, as used herein, the term "polymer" will be understood to mean a molecule that encompasses a backbone (also referred to as "main chain") of one or more distinct types of repeat units (the smallest constitutional unit of the molecule) and is inclusive of the commonly known terms "oligomer", "copolymer", "homopolymer", "random polymer" and the like. Further, it will be understood that the term polymer is inclusive of, in addition to the polymer itself, residues from initiators, catalysts and other elements attendant to the synthesis of such a polymer, where such residues are understood as not being covalently incorporated thereto. Further, such residues and other elements, while normally removed during post polymerization purification processes, are typically mixed or co-mingled with the polymer such that they generally remain with the polymer when it is transferred between vessels or between solvents or dispersion media.

As used herein, in a formula showing a polymer or a repeat unit, like for example a unit of formula I or a polymer of formula III or IV or their subformulae, an asterisk (*) will be understood to mean a chemical linkage to an adjacent unit or to a terminal group in the polymer backbone. In a ring, like for example a benzene or thiophene ring, an asterisk (*) will be understood to mean a C atom that is fused to an adjacent ring.

As used herein, the terms "repeat unit", "repeating unit" and "monomeric unit" are used interchangeably and will be understood to mean the constitutional repeating unit (CRU), which is the smallest constitutional unit the repetition of which constitutes a regular macromolecule, a regular oligomer molecule, a regular block or a regular chain (Pure Appl. Chem., 1996, 68, 2291). As further used herein, the term "unit" will be understood to mean a structural unit which can be a repeating unit on its own, or can together with other units form a constitutional repeating unit.

As used herein, a "terminal group" will be understood to mean a group that terminates a polymer backbone. The expression "in terminal position in the backbone" will be understood to mean a divalent unit or repeat unit that is linked at one side to such a terminal group and at the other side to another repeat unit. Such terminal groups include endcap groups, or reactive groups that are attached to a monomer forming the polymer backbone which did not participate in the polymerisation reaction, like for example a group having the meaning of $R^5$ or $R^6$ as defined below.

As used herein, the term "endcap group" will be understood to mean a group that is attached to, or replacing, a terminal group of the polymer backbone. The endcap group can be introduced into the polymer by an endcapping process. Endcapping can be carried out for example by reacting the terminal groups of the polymer backbone with a monofunctional compound ("endcapper") like for example an alkyl- or arylhalide, an alkyl- or arylstannane or an alkyl- or arylboronate. The endcapper can be added for example after the polymerisation reaction. Alternatively the endcapper can be added in situ to the reaction mixture before or during the polymerisation reaction. In situ addition of an endcapper can also be used to terminate the polymerisation reaction and thus control the molecular weight of the forming polymer. Typical endcap groups are for example H, phenyl and lower alkyl.

As used herein, the term "small molecule" will be understood to mean a monomeric compound which typically does not contain a reactive group by which it can be reacted to form a polymer, and which is designated to be used in monomeric form. In contrast thereto, the term "monomer" unless stated otherwise will be understood to mean a monomeric compound that carries one or more reactive functional groups by which it can be reacted to form a polymer.

As used herein, the terms "donor" or "donating" and "acceptor" or "accepting" will be understood to mean an electron donor or electron acceptor, respectively. "Electron donor" will be understood to mean a chemical entity that

donates electrons to another compound or another group of atoms of a compound. "Electron acceptor" will be understood to mean a chemical entity that accepts electrons transferred to it from another compound or another group of atoms of a compound. See also International Union of Pure and Applied Chemistry, Compendium of Chemical Technology, Gold Book, Version 2.3.2, 19. August 2012, pages 477 and 480.

As used herein, the term "n-type" or "n-type semiconductor" will be understood to mean an extrinsic semiconductor in which the conduction electron density is in excess of the mobile hole density, and the term "p-type" or "p-type semiconductor" will be understood to mean an extrinsic semiconductor in which mobile hole density is in excess of the conduction electron density (see also, J. Thewlis, Concise Dictionary of Physics, Pergamon Press, Oxford, 1973).

As used herein, the term "leaving group" will be understood to mean an atom or group (which may be charged or uncharged) that becomes detached from an atom in what is considered to be the residual or main part of the molecule taking part in a specified reaction (see also Pure Appl. Chem., 1994, 66, 1134).

As used herein, the term "conjugated" will be understood to mean a compound (for example a polymer) that contains mainly C atoms with $sp^2$-hybridisation (or optionally also sp-hybridisation), and wherein these C atoms may also be replaced by hetero atoms. In the simplest case this is for example a compound with alternating C-C single and double (or triple) bonds, but is also inclusive of compounds with aromatic units like for example 1,4-phenylene. The term "mainly" in this connection will be understood to mean that a compound with naturally (spontaneously) occurring defects, or with defects included by design, which may lead to interruption of the conjugation, is still regarded as a conjugated compound.

As used herein, unless stated otherwise the molecular weight is given as the number average molecular weight $M_n$ or weight average molecular weight Mw, which is determined by gel permeation chromatography (GPC) against polystyrene standards in eluent solvents such as tetrahydrofuran, trichloromethane (TCM, chloroform), chlorobenzene or 1,2,4-trichlorobenzene. Unless stated otherwise, 1,2,4-trichlorobenzene is used as solvent. The degree of polymerization, also referred to as total number of repeat units, n, will be understood to mean the number average degree of polymerization given as $n = M_n/M_U$, wherein $M_n$ is the number average molecular weight and Mu is the molecular weight of the single repeat unit, see J. M. G. Cowie, Polymers: Chemistry & Physics of Modern Materials, Blackie, Glasgow, 1991.

As used herein, the term "carbyl group" will be understood to mean any monovalent or multivalent organic moiety which comprises at least one carbon atom either without any non-carbon atoms (like for example -C≡C-), or optionally combined with at least one non-carbon atom such as B, N, O, S, P, Si, Se, As, Te or Ge (for example carbonyl etc.).

As used herein, the term "hydrocarbyl group" will be understood to mean a carbyl group that does additionally contain one or more H atoms and optionally contains one or more hetero atoms like for example B, N, O, S, P, Si, Se, As, Te or Ge.

As used herein, the term "hetero atom" will be understood to mean an atom in an organic compound that is not a H- or C-atom, and preferably will be understood to mean B, N, O, S, P, Si, Se, As, Te or Ge.

A carbyl or hydrocarbyl group comprising a chain of 3 or more C atoms may be straight-chain, branched and/or cyclic, and may include spiro-connected and/or fused rings.

Preferred carbyl and hydrocarbyl groups include alkyl, alkoxy, thioalkyl, alkylcarbonyl, alkoxycarbonyl, alkylcarbonyloxy and alkoxycarbonyloxy, each of which is optionally substituted and has 1 to 40, preferably 1 to 25, very preferably 1 to 18 C atoms, furthermore optionally substituted aryl or aryloxy having 6 to 40, preferably 6 to 25 C atoms, furthermore alkylaryloxy, arylcarbonyl, aryloxycarbonyl, arylcarbonyloxy and aryloxycarbonyloxy, each of which is optionally substituted and has 6 to 40, preferably 7 to 40 C atoms, wherein all these groups do optionally contain one or more hetero atoms, preferably selected from B, N, O, S, P, Si, Se, As, Te and Ge.

Further preferred carbyl and hydrocarbyl group include for example: a $C_1$-$C_{40}$ alkyl group, a $C_1$-$C_{40}$ fluoroalkyl group, a $C_1$-$C_{40}$ alkoxy or oxaalkyl group, a $C_2$-$C_{40}$ alkenyl group, a $C_2$-$C_{40}$ alkynyl group, a $C_3$-$C_{40}$ allyl group, a $C_4$-$C_{40}$ alkyldienyl group, a $C_4$-$C_{40}$ polyenyl group, a $C_2$-$C_{40}$ ketone group, a $C_2$-$C_{40}$ ester group, a $C_6$-$C_{18}$ aryl group, a $C_6$-$C_{40}$ alkylaryl group, a $C_6$-$C_{40}$ arylalkyl group, a $C_4$-$C_{40}$ cycloalkyl group, a $C_4$-$C_{40}$ cycloalkenyl group, and the like. Preferred among the foregoing groups are a $C_1$-$C_{20}$ alkyl group, a $C_1$-$C_{20}$ fluoroalkyl group, a $C_2$-$C_{20}$ alkenyl group, a $C_2$ -$C_{20}$ alkynyl group, a $C_3$-$C_{20}$ allyl group, a $C_4$-$C_{20}$ alkyldienyl group, a $C_2$-$C_{20}$ ketone group, a $C_2$-$C_{20}$ ester group, a $C_6$-$C_{12}$ aryl group, and a $C_4$-$C_{20}$ polyenyl group, respectively.

Also included are combinations of groups having carbon atoms and groups having hetero atoms, like e.g. an alkynyl group, preferably ethynyl, that is substituted with a silyl group, preferably a trialkylsilyl group.

The carbyl or hydrocarbyl group may be an acyclic group or a cyclic group. Where the carbyl or hydrocarbyl group is an acyclic group, it may be straight-chain or branched. Where the carbyl or hydrocarbyl group is a cyclic group, it may be a non-aromatic carbocyclic or heterocyclic group, or an aryl or heteroaryl group.

A non-aromatic carbocyclic group as referred to above and below is saturated or unsaturated and preferably has 4 to 30 ring C atoms. A non-aromatic heterocyclic group as referred to above and below preferably has 4 to 30 ring C atoms, wherein one or more of the C ring atoms are optionally replaced by a hetero atom, preferably selected from N, O, S, Si and Se, or by a -S(O)- or -$S(O)_2$- group. The non-aromatic carbo- and heterocyclic groups are mono- or

polycyclic, may also contain fused rings, preferably contain 1, 2, 3 or 4 fused or unfused rings, and are optionally substituted with one or more groups L, wherein

L is selected from F, Cl, -CN, -NC, -NCO, -NCS, -OCN, -SCN, $-R^0$, $-OR^0$,$-SR^0$, $-C(=O)X^0$, $-C(=O)R^0$, $-C(=O)-OR^0$, -O-$C(=O)R^0$, $-NH_2$, $-NHR^0$,$-NR^0R^{00}$, $-C(=O)NHR^0$, $-C(=O)NR^0R^{00}$, $-SO_3H$, $-SO_2R^0$, -OH, $-NO_2$, $-CF_3$,$-SF_5$, or optionally substituted silyl, or carbyl or hydrocarbyl with 1 to 20 C atoms that is optionally substituted and optionally comprises one or more hetero atoms, wherein $X^0$ is halogen, preferably F or Cl, and $R^0$, $R^{00}$ denote H or straight-chain or branched alkyl with 1 to 20, preferably 1 to 12 C atoms that is optionally fluorinated.

Preferably L is selected from F, -CN, R, -OR, -SR, -C(=O)-R, -C(=O)-OR,-O-C(=O)-R, -O-C(=O)-OR, -C(=O)-NHR, $-C(=O)-NRR^n$, wherein R and $R^n$ are independently of each other straight-chain or branched alkyl with 1 to 20 C atoms that is optionally fluorinated.

Further preferred substituents L are selected from F or alkyl, alkoxy, oxaalkyl, thioalkyl, fluoroalkyl and fluoroalkoxy with 1 to 12 C atoms, or alkylcarbonyl, alkylcarbonyloxy, alkxoycarbonyl, alkenyl or alkynyl with 2 to 12 C atoms (including the carbonyl-C-atom).

Preferred non-aromatic carbocyclic or heterocyclic groups are tetrahydrofuran, indane, pyran, pyrrolidine, piperidine, cyclopentane, cyclohexane, cycloheptane, cyclopentanone, cyclohexanone, dihydro-furan-2-one, tetrahydropyran-2-one and oxepan-2-one.

An aryl group as referred to above and below preferably has 4 to 30 ring C atoms, is mono- or polycyclic and may also contain fused rings, preferably contains 1, 2, 3 or 4 fused or unfused rings, and is optionally substituted with one or more groups L as defined above.

A heteroaryl group as referred to above and below preferably has 4 to 30 ring C atoms, wherein one or more of the C ring atoms are replaced by a hetero atom, preferably selected from N, O, S, Si and Se, is mono- or polycyclic and may also contain fused rings, preferably contains 1, 2, 3 or 4 fused or unfused rings, and is optionally substituted with one or more groups L as defined above.

An arylalkyl or heteroarylalkyl group as referred to above and below preferably denotes $-(CH_2)_a$-aryl or $-(CH_2)_a$-heteroaryl, wherein a is an integer from 1 to 6, preferably 1, and "aryl" and "heteroaryl" have the meanings given above and below. A preferred arylalkyl group is benzyl which is optionally substituted by L.

As used herein, "arylene" will be understood to mean a divalent aryl group, and "heteroarylene" will be understood to mean a divalent heteroaryl group, including all preferred meanings of aryl and heteroaryl as given above and below.

Preferred aryl and heteroaryl groups are phenyl in which, in addition, one or more CH groups may be replaced by N, naphthalene, thiophene, selenophene, thienothiophene, dithienothiophene, fluorene and oxazole, all of which can be unsubstituted, mono- or polysubstituted with L as defined above. Very preferred rings are selected from pyrrole, preferably N-pyrrole, furan, pyridine, preferably 2- or 3-pyridine, pyrimidine, pyridazine, pyrazine, triazole, tetrazole, pyrazole, imidazole, isothiazole, thiazole, thiadiazole, isoxazole, oxazole, oxadiazole, thiophene, preferably 2-thiophene, selenophene, preferably 2-selenophene, thieno[3,2-b]thiophene, thieno[2,3-b]thiophene, furo[3,2-b]furan, furo[2,3-b]furan, seleno[3,2-b]selenophene, seleno[2,3-b]selenophene, thieno[3,2-b]selenophene, thieno[3,2-b]furan, indole, isoindole, benzo[b]furan, benzo[b]thiophene, benzo[1,2-b;4,5-b']dithiophene, benzo[2,1-b;3,4-b']dithiophene, quinole, 2-methylquinole, isoquinole, quinoxaline, quinazoline, benzotriazole, benzimidazole, benzothiazole, benzisothiazole, benzisoxazole, benzoxadiazole, benzoxazole, benzothiadiazole, 4H-cyclopenta[2,1-b;3,4-b']dithiophene, 7H-3,4-dithia-7-sila-cyclopenta[a]pentalene, all of which can be unsubstituted, mono- or polysubstituted with L as defined above. Further examples of aryl and heteroaryl groups are those selected from the groups shown hereinafter.

An alkyl group or an alkoxy group, i.e., where the terminal $CH_2$ group is replaced by -O-, can be straight-chain or branched. It is preferably straight-chain, has 2, 3, 4, 5, 6, 7, 8, 12 or 16 carbon atoms and accordingly is preferably ethyl, propyl, butyl, pentyl, hexyl, heptyl, octyl, dodecyl or hexadecyl, ethoxy, propoxy, butoxy, pentoxy, hexoxy, heptoxy, octoxy, dodecoxy or hexadecoxy, furthermore methyl, nonyl, decyl, undecyl, tridecyl, tetradecyl, pentadecyl, nonoxy, decoxy, undecoxy, tridecoxy or tetradecoxy, for example.

An alkenyl group, i.e., wherein one or more $CH_2$ groups are replaced by-CH=CH- can be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are $C_2$-$C_7$-1E-alkenyl, $C_4$-$C_7$-3E-alkenyl, $C_5$-$C_7$-4-alkenyl, $C_6$-$C_7$-5-alkenyl and $C_7$-6-alkenyl, in particular $C_2$-$C_7$-1E-alkenyl, $C_4$-$C_7$-3E-alkenyl and $C_5$-$C_7$-4-alkenyl. Examples for particularly preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C atoms are generally preferred.

An oxaalkyl group, i.e., where one $CH_2$ group is replaced by -O-, is preferably straight-chain 2-oxapropyl (=methoxymethyl), 2-(=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

In an alkyl group wherein one $CH_2$ group is replaced by -O- and one $CH_2$ group is replaced by -C(O)-, these radicals are preferably neighboured. Accordingly these radicals together form a carbonyloxy group -C(O)-O- or an oxycarbonyl group -O-C(O)-. Preferably this group is straight-chain and has 2 to 6 C atoms. It is accordingly preferably acetyloxy, propionyloxy, butyryloxy, pentanoyloxy, hexanoyloxy, acetyloxymethyl, propionyloxymethyl, butyryloxymethyl, pentanoyloxymethyl, 2-acetyloxyethyl, 2-propionyloxyethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxycarbonyl)ethyl, 2-(ethoxycarbonyl)ethyl, 2-(propoxycarbonyl)ethyl, 3-(methoxycarbonyl)propyl, 3-(ethoxycarbonyl)propyl, 4-(methoxycarbonyl)-butyl.

An alkyl group wherein two or more $CH_2$ groups are replaced by -O- and/or -C(O)O- can be straight-chain or branched. It is preferably straight-chain and has 3 to 12 C atoms. Accordingly it is preferably bis-carboxy-methyl, 2,2-bis-carboxy-ethyl, 3,3-bis-carboxy-propyl, 4,4-bis-carboxy-butyl, 5,5-bis-carboxy-pentyl, 6,6-bis-carboxy-hexyl, 7,7-bis-carboxy-heptyl, 8,8-bis-carboxy-octyl, 9,9-bis-carboxy-nonyl, 10,10-bis-carboxy-decyl, bis-(methoxycarbonyl)-methyl, 2,2-bis-(methoxycarbonyl)-ethyl, 3,3-bis-(methoxycarbonyl)-propyl, 4,4-bis-(methoxycarbonyl)-butyl, 5,5-bis-(methoxycarbonyl)-pentyl, 6,6-bis-(methoxycarbonyl)-hexyl, 7,7-bis-(methoxycarbonyl)-heptyl, 8,8-bis-(methoxycarbonyl)-octyl, bis-(ethoxycarbonyl)-methyl, 2,2-bis-(ethoxycarbonyl)-ethyl, 3,3-bis-(ethoxycarbonyl)-propyl, 4,4-bis-(ethoxycarbonyl)-butyl, 5,5-bis-(ethoxycarbonyl)-hexyl.

A thioalkyl group, i.e., where one $CH_2$ group is replaced by -S-, is preferably straight-chain thiomethyl ($-SCH_3$), 1-thioethyl ($-SCH_2CH_3$), 1-thiopropyl (= $-SCH_2CH_2CH_3$), 1- (thiobutyl), 1-(thiopentyl), 1-(thiohexyl), 1-(thioheptyl), 1-(thiooctyl), 1-(thiononyl), 1-(thiodecyl), 1-(thioundecyl) or 1-(thiododecyl), wherein preferably the $CH_2$ group adjacent to the $sp^2$ hybridised vinyl carbon atom is replaced.

A fluoroalkyl group is perfluoroalkyl $C_iF_{2i+i}$, wherein i is an integer from 1 to 15, in particular $CF_3$, $C_2F_5$, $C_3F_7$, $C_4F_9$, $C_5F_{11}$, $C_6F_{13}$, $C_7F_{15}$ or $C_8F_{17}$, very preferably $C_6F_{13}$, or partially fluorinated alkyl, preferably with 1 to 15 C atoms, in particular 1,1-difluoroalkyl, all of the aforementioned being straight-chain or branched.

Preferably "fluoroalkyl" means a partially fluorinated (i.e. not perfluorinated) alkyl group.

Alkyl, alkoxy, alkenyl, oxaalkyl, thioalkyl, carbonyl and carbonyloxy groups can be achiral or chiral groups. Particularly preferred chiral groups are 2-butyl (=1-methylpropyl), 2-methylbutyl, 2-methylpentyl, 3-methylpentyl, 2-ethylhexyl, 2-butyloctyl, 2-hexyldecyl, 2-octyldodecyl, 2-propylpentyl, in particular 2-methylbutyl, 2-methylbutoxy, 2-methylpentoxy, 3-methylpentoxy, 2-ethyl-hexoxy, 2-butyloctoxyo, 2-hexyldecoxy, 2-octyldodecoxy, 1-methylhexoxy, 2-octyloxy, 2-oxa-3-methylbutyl, 3-oxa-4-methyl-pentyl, 4-methylhexyl, 2-hexyl, 2-octyl, 2-nonyl, 2-decyl, 2-dodecyl, 6-methoxy-octoxy, 6-methyloctoxy, 6-methyloctanoyloxy, 5-methylheptyloxy-carbonyl, 2-methylbutyryloxy, 3-methylvaleroyloxy, 4-methylhexanoyloxy, 2-chloro-propionyloxy, 2-chloro-3-methylbutyryloxy, 2-chloro-4-methyl-valeryl-oxy, 2-chloro-3-methylvaleryloxy, 2-methyl-3-oxapentyl, 2-methyl-3-oxa-hexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1 -trifluoro-2-octyl, 2-fluoromethyloctyloxy for example. Very preferred are 2-ethylhexyl, 2-butyloctyl, 2-hexyldecyl, 2-octyldodecyl, 2-hexyl, 2-octyl, 2-octyloxy, 1,1,1 -trifluoro-2-hexyl, 1,1,1-trifluoro-2-octyl and 1,1,1-trifluoro-2-octyloxy.

Preferred achiral branched groups are isopropyl, isobutyl (=methylpropyl), isopentyl (=3-methylbutyl), tert. butyl, isopropoxy, 2-methyl-propoxy and 3-methylbutoxy.

In a preferred embodiment, the alkyl groups are independently of each other selected from primary, secondary or tertiary alkyl or alkoxy with 1 to 30 C atoms, wherein one or more H atoms are optionally replaced by F, or aryl, aryloxy, heteroaryl or heteroaryloxy that is optionally alkylated or alkoxylated and has 4 to 30 ring atoms. Very preferred groups of this type are selected from the group consisting of the following formulae

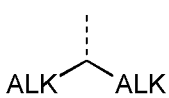
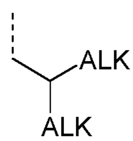
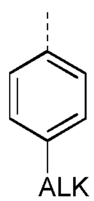
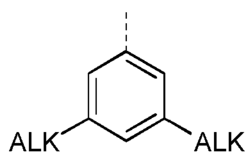

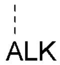
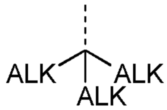
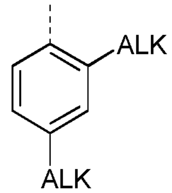
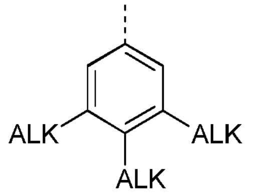

wherein "ALK" denotes optionally fluorinated, preferably linear, alkyl or alkoxy with 1 to 20, preferably 1 to 12 C-atoms, in case of tertiary groups very preferably 1 to 9 C atoms, and the dashed line denotes the link to the ring to which these groups are attached. Especially preferred among these groups are those wherein all ALK subgroups are identical.

As used herein, if an aryl(oxy) or heteroaryl(oxy) group is "alkylated or alkoxylated", this means that it is substituted with one or more alkyl or alkoxy groups having from 1 to 20 C-atoms and being straight-chain or branched and wherein one or more H atoms are optionally substituted by an F atom.

Above and below, $Y^1$ and $Y^2$ are independently of each other H, F, Cl or CN.

As used herein, -CO-, -C(=O)- and -C(O)- will be understood to mean a carbonyl group, i.e. a group having the structure

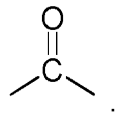

As used herein $C=CR^1R^2$ will be understood to mean a group having the structure

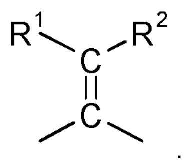

As used herein, "halogen" includes F, Cl, Br or I, preferably F, Cl or Br. A halogen atom that represents a substituent on a ring or chain is preferably F or Cl, very preferably F. A halogen atom that represents a reactive group in a monomer is preferably Cl, Br or I, very preferably Br or I.

Detailed Description

The compounds of the present invention are easy to synthesize and exhibit advantageous properties. They show good processability for the device manufacture process, high solubility in organic solvents, and are especially suitable for large scale production using solution processing methods.

Co-polymers derived from monomers of the present invention and electron donor monomers show low bandgaps, high charge carrier mobilities, high external quantum efficiencies in BHJ solar cells, good morphology when used in p/n-type blends e.g. with fullerenes, high oxidative stability, a long lifetime in electronic devices, and are promising materials for organic electronic OE devices, especially for OPV devices with high power conversion efficiency.

The compounds of the present invention are especially suitable as p-type semiconductors for the preparation of blends of p-type and n-type semiconductors which are suitable for use in BHJ photovoltaic devices.

Besides, the compounds of the present invention show the following advantageous properties:

i) Additional solubility can be introduced into the compound by inclusion of various solubilizing groups in $R^1$ and $R^2$ positions.

ii) Additional fine-tuning of the electronic energies (HOMO/LUMO levels) by co-polymerisation with appropriate comonomer(s) can afford attractive candidate materials for OPV applications.

iii) The BBT units have planar structures that enable strong pi-pi stacking in the solid state leading to improved charge transport properties in the form of higher charge carrier mobility.

iv) An incorporation of various $R^1$ and $R^2$ substituents into the BBT core results in monomers with either electron donating property, like for example dialkyl-BBT, or electron accepting property, like for example dialkyl ester-BBT.

v) The BBT unit can thus be altered from a donor unit to an acceptor unit by switching the nature of the solubilising groups, for example from electron donating groups like alkyl or alkoxy to electron withdrawing groups like ketone or ester, thus allowing a much broader applicability of the core for use in OPV or OPD polymers

vi) Using both BBT units substituted by electron donating groups like alkyl or alkoxy and BBT units substituted by to electron withdrawing groups like ketone or ester, it is possible to create a donor-acceptor polymer from BBT units,

optionally with spacer units like thiophene, dithiophene or thienothiophene to keep the backbone flat, but without further donor or acceptor units. Such polymers are expected to have a high Voc.

Very preferably $R^1$ and $R^2$ in the units of formula I denote R or -C(=O)-OR, wherein R is straight-chain or branched alkyl with 1 to 20 C atoms that is optionally fluorinated.

The compounds according to the present invention include oligomers and polymers.

A preferred embodiment of the present invention relates to a conjugated polymer comprising, preferably consisting of, one or more repeating units of formula II1 or II2, and optionally one or more repeating units of formula II3:

$$-(Ar^1)_a-U-(Ar^2)_b-(Ar^3)_c-(Ar^4)_d- \qquad \text{II1}$$

$$-(Ar^1)_a-(Ar^2)_b-U-(Ar^3)_c-(Ar^4)_d- \qquad \text{II2}$$

$$-(Ar^1)_a-(Ar^2)_b-(Ar^3)_c-(Ar^4)_d- \qquad \text{II3}$$

wherein the individual radicals, independently of each other and on each occurrence identically or differently, have the following meanings

U a unit of formula I as defined above and below,

$Ar^{1-4}$ arylene or heteroarylene that has 5 to 20 ring atoms, is mono- or polycyclic, does optionally contain fused rings, is unsubstituted or substituted by one or more identical or different groups L, and is different from formula I,

a, b, c, d 0 or 1, wherein in formula II3 $a+b+c+d\geq 1$.

Preferably the conjugated polymer comprises one or more repeating units of formula II1 or II2 wherein $a+b+c+d\geq 1$.

Further preferably the conjugated polymer comprises one or more repeating units of formula II1 wherein b=1 and a=c=d=0 and one or more repeating units of formula II3 wherein a=b=1 and c=d=0.

Further preferably the conjugated polymer comprises two or more distinct repeating units of formula II1 wherein b=1 and a=c=d=0.

Further preferably at least one of $Ar^1$, $Ar^2$, $Ar^3$ and $Ar^4$ is an arylene or heteroarylene group as being defined in formula II1 and having electron donor property.

Preferably L denotes F or is selected from the following groups

- the group consisting of R, -OR and -SR wherein R is straight-chain or branched alkyl with 1 to 20 C atoms which is unsubstituted or substituted by one or more F atoms,

- the group consisting of -C(=O)-R, -C(=O)-OR, -OC(=O)-R, -C(=O)-NHR and -C(=O)-NRR$^n$, wherein R and R$^n$ are independently of each other straight-chain or branched alkyl with 1 to 20 C atoms that is optionally fluorinated,

Further preferably the conjugated polymer according to the present invention is selected of formula III:

$$*\!-\!\!\left[(A)_x\!-\!(B)_y\right]_n\!\!-\!* \qquad \text{III}$$

wherein

A is a unit of formula I, II1 or II2 as defined above and below,
B is a unit of formula I, 111, II2 or II3 as defined above and below,
x is > 0 and ≤ 1,
y is ≥ 0 and < 1,
x+y is 1, and
n is an integer ≥5.

Preferred polymers of formula III are selected from the following formulae

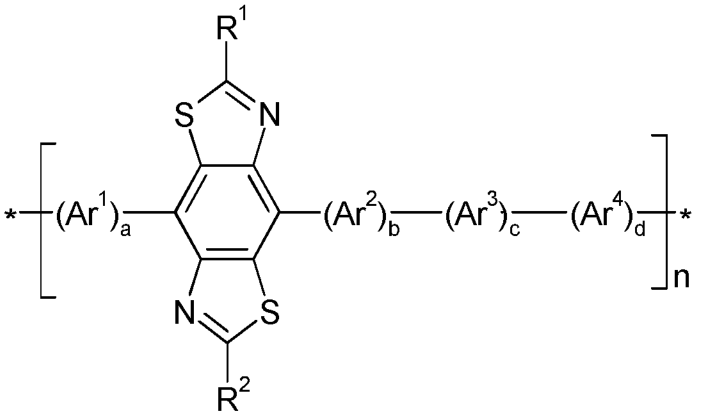

III1

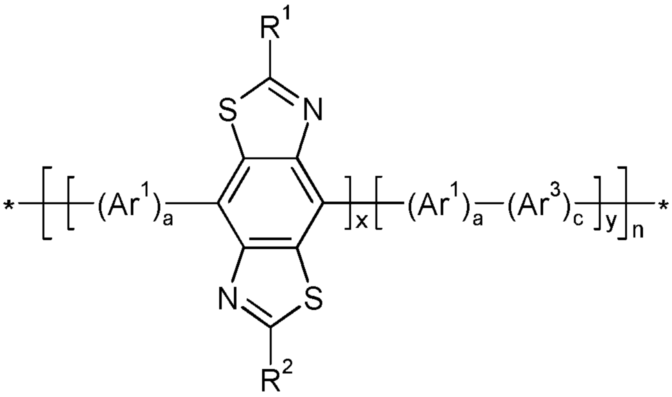

III2

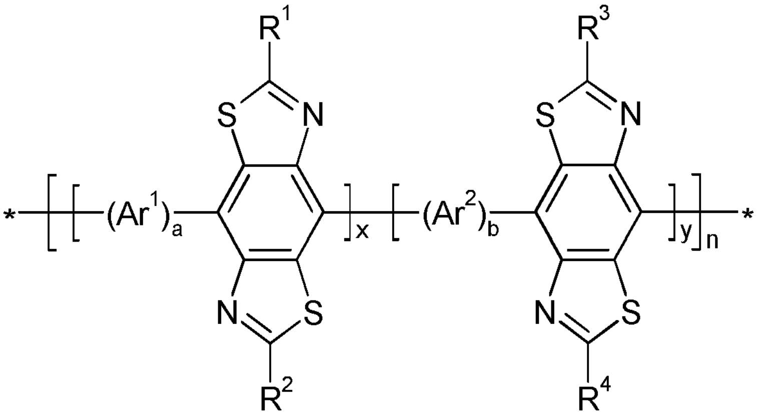

III3

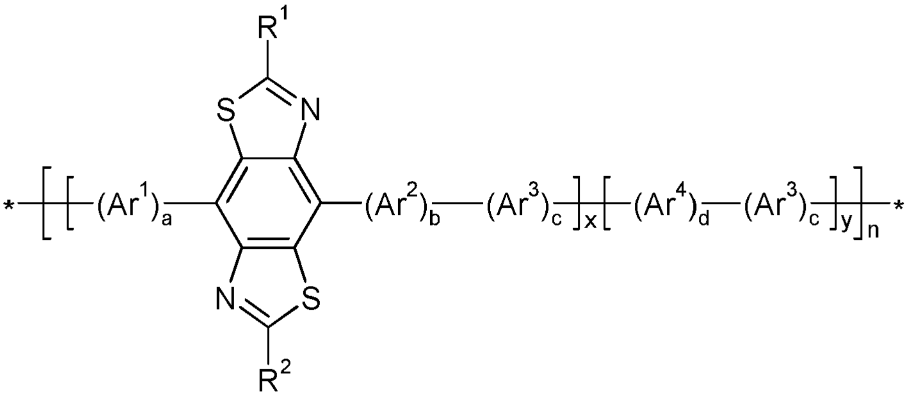

III4

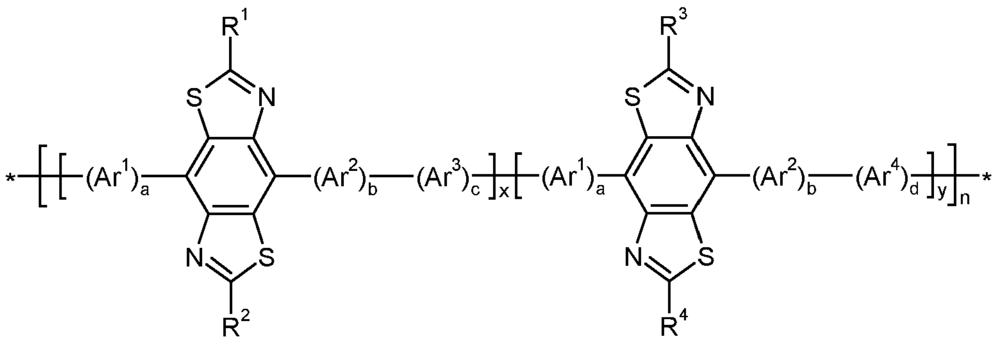 III5

wherein

$R^1$ and $R^2$ have the meanings of formula I or one of the preferred meanings given above and below,
$R^3$ and $R^4$ have one of the meanings of $R^1$ or its preferred meanings given above and below,
$Ar^1$, $Ar^2$, $Ar^3$, $Ar^4$, a, b, c and d have the meanings of formula II1 or one of the preferred meanings given above and below,
x, y and n have the meanings of formula III or one of the preferred meanings given above and below, and
preferably $Ar^3$ is selected from arylene or heteroarylene units as described above and below having electron donor properties, and
preferably at least one of the meanings of $R^1$ and $R^2$ is different from at least one of the meanings of $R^3$ and $R^4$.

In the polymers of formula III and III1-III5, x and y denote the mole fraction of repeating units A and B, respectively, and n denotes the degree of polymerisation or total number of repeating units A and B. These formulae include block copolymers, random or statistical copolymers and alternating copoymers of A and B, as well as homopolymers of A for the case when x>0 and y=0.

In the polymers of formula III and III1- III5, x is preferably from 0.1 to 0.9, very preferably from 0.3 to 0.7.

In the polymers of formula III and III1-III5, y is preferably from 0.1 to 0.9, very preferably from 0.3 to 0.7.

In the polymers according to the present invention, the total number of repeating units n is preferably from 2 to 10,000. The total number of repeating units n is preferably $\geq 5$, very preferably $\geq 10$, most preferably $\geq 50$, and preferably $\leq 500$, very preferably $\leq 1,000$, most preferably $\leq 2,000$, including any combination of the aforementioned lower and upper limits of n.

The polymers of the present invention include homopolymers and copolymers, like statistical or random copolymers, alternating copolymers and block copolymers, as well as combinations thereof.

Further preferably the conjugated polymer is selected of formula IV

$R^5$-chain-$R^6$ IV

wherein "chain" denotes a polymer chain selected of formulae III and III1-III5, and $R^5$ and $R^6$ have independently of each other one of the meanings of L as defined above, or denote, independently of each other, H, F, Br, Cl, I, $-CH_2Cl$, -CHO, $-CR'=CR''_2$, -SiR′R″R‴, -SiR′X′X″, -SiR′R″X′, -SnR′R″R‴, -BR′R″, -B(OR′)(OR″), $-B(OH)_2$, $-O-SO_2-R'$, -C≡CH, $-C{\equiv}C-SiR'_3$, -ZnX′ or an endcap group, X′ and X″ denote halogen, R′, R″ and R‴ have independently of each other one of the meanings of $R^0$ given in formula I, and preferably denote alkyl with 1 to 12 C atoms, and two of R′, R″ and R‴ may also form a cyclosilyl, cyclostannyl, cycloborane or cycloboronate group with 2 to 20 C atoms together with the respective hetero atom to which they are attached.

Preferred endcap groups $R^5$ and $R^6$ are H, $C_{1-20}$ alkyl, or optionally substituted $C_{6-12}$ aryl or $C_{2-10}$ heteroaryl, very preferably H or phenyl.

The polymers of the present invention can be prepared from a monomer of formula V1 or V2

$R^7-(Ar^1)_a-U-(Ar^2)_b-(Ar^3)_c-(Ar^4)_d-R^8$ V1

$R^7-(Ar^1)_a-(Ar^2)_b-U-(Ar^3)_c-(Ar^4)_d-R^8$ V2

wherein U, $Ar^{1-4}$, a, b, c and d have the meanings of formula 111, or one of the preferred meanings as described above and below, and $R^7$ and $R^8$ are independently of each other selected from the group consisting of H, which is preferably an activated C-H bond, Cl, Br, I, O-tosylate, O-triflate, O-mesylate, O-nonaflate, $-SiMe_2F$, $-SiMeF_2$, $-O-SO_2Z^1$, $-B(OZ^2)_2$, $-CZ^3=C(Z^3)_2$, -C≡CH, $-C{\equiv}CSi(Z^1)_3$, $-ZnX^0$ and $-Sn(Z^4)_3$, wherein $X^0$ is halogen, $Z^{1-4}$ are selected from the group consisting

of alkyl and aryl, preferably $C_{1-10}$ alkyl and $C_{6-12}$ aryl, each being optionally substituted, and two groups $Z^2$ may also form a cycloboronate group having 2 to 20 C atoms together with the B- and O-atoms, and wherein at least one of $R^7$ and $R^8$ is different from H, and preferably both of $R^1$ and $R^2$ are different from H,
with the proviso that a+b+c+d≥1 or $R^7$ and/or $R^8$ are different from Br, preferably different from halogen.

Very preferred are monomers of formula V1 and V2 and their subformulae wherein a+b+c+d ≥1.

Further preferred are monomers of formula V1 and its subformulae wherein a+b+c+d=0.

Further preferred are monomers of formula V1 and V2 and their subformulae wherein $R^7$ and $R^8$ are selected from $-B(OZ^2)_2$ and $Sn(Z^4)_3$.

Further preferred are monomers selected from the following formulae

$R^7$-$Ar^1$-U-$Ar^2$-$R^8$ V1a

$R^7$-U-$R^8$ V1b

$R^7$-$Ar^1$-U-$R^8$ V1c

$R^7$-U-$Ar^2$-$R^8$ V1d

wherein U, $Ar^1$, $Ar^2$, $R^7$ and $R^8$ are as defined in formula V1.

Very preferred are monomers of formula V1 and V2 and their subformulae wherein $R^7$ and $R^8$ are selected from Br, $B(OZ^2)_2$ and $Sn(Z^4)_3$.

A further preferred embodiment of the present invention relates to an oligomer of formula VI

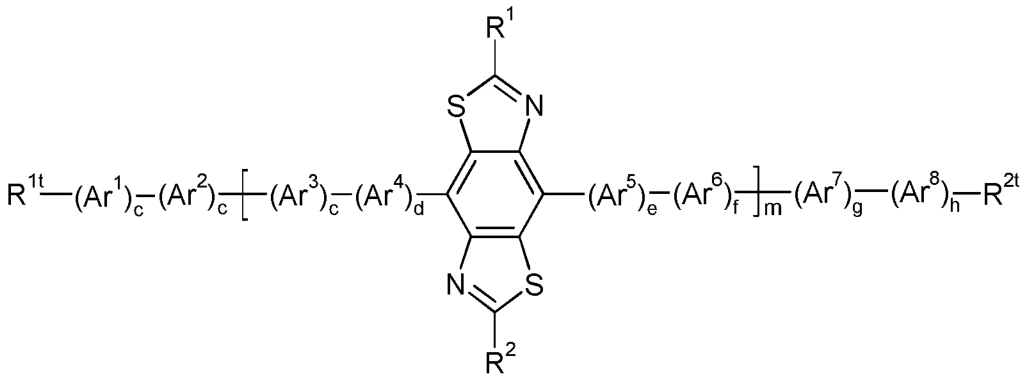

VI

wherein the individual radicals, independently of each other and on each occurrence identically or differently, have the following meanings

$R^1$, $R^2$ one of the meanings given above and below,

$Ar^{1-8}$ one of the meanings given for $Ar^1$ in formula II1 or one of its preferred meanings given above and below, or a unit of formula I as defined above and below, or $-CY^1=CY^2-$,

$Y^1$, $Y^2$ H, F, Cl or CN,

$R^{1t, 2t}$ H, F, Cl, Br, -CN, $-CF_3$, R*, $-CF_2$-R*, -O-R*, -S-R*, $-SO_2$-R*,$-SO_3$-R*,-C(=O)-R*, -C(=S)-R*, -C(=O)-$CF_2$-R*, -C(=O)-OR*,-C(=S)-OR*, -O-C(=O)-R*, -O-C(=S)-R*, -C(=O)-SR*, -S-C(=O)-R*, -C(=O)NR*R**, -NR*-C(=O)-R*, -NHR*,-NR*R**,-CR*=CR*R**, -C≡C-R*, -C≡C-SiR*R**R***, -SiR*R**R***,-CH=C(CN)-C(=O)-OR*, -CH=C(CO-OR*)$_2$, -CH=C(CO-NR*R**)$_2$, -CH=C(CN)($Ar^9$),

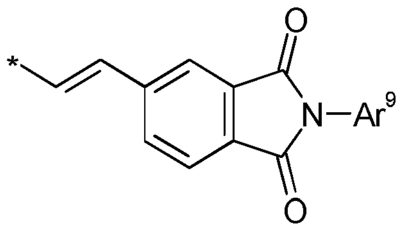,
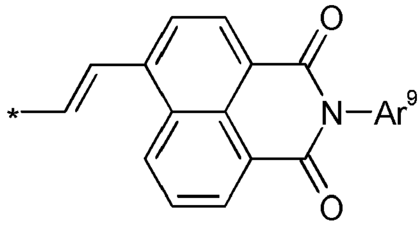,

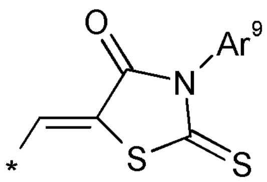,
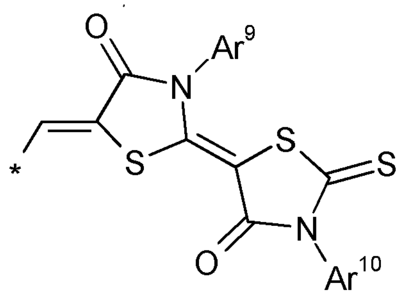,
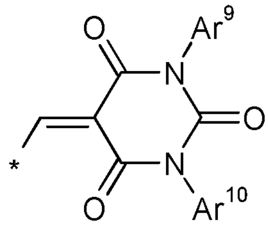,

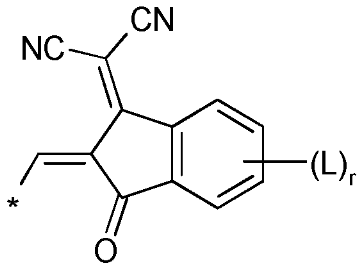,
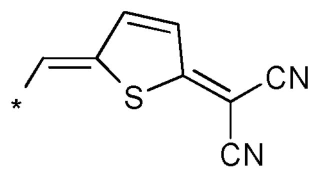

$Ar^{9,10}$ aryl or heteroaryl, each having from 4 to 30 ring atoms, optionally containing fused rings and being unsubstituted or substituted with one or more groups L as defined in formula I,

R*, R**, R*** alkyl with 1 to 20 C atoms which is straight-chain, branched or cyclic, and is unsubstituted, or substituted with one or more F or Cl atoms or CN groups, or perfluorinated, and in which one or more C atoms are optionally replaced by -O-, -S-, -C(O)-,-C(S)-, $-SiR^0R^{00}-$, $-NR^0R^{00}-$, $-CHR^0=CR^{00}-$ or -C=C- such that O-and/or S-atoms are not directly linked to each other,

$R^0$, $R^{00}$ H or or straight-chain or branched alkyl with 1 to 20, preferably 1 to 12 C atoms that is optionally fluorinated,

a-h 0 or 1, preferably with at least one of a-h being 1,

m 1, 2 or 3,

with the proviso that m>1.

Especially preferred are repeating units, oligomers, and polymers of formulae II1, II2, III, III1-III5, IV, V1, V2, V1a-V1d, VI and their subformulae wherein one or more of $Ar^1$, $Ar^2$, $Ar^3$ and $Ar^4$ denote arylene or heteroarylene, preferably having electron donor properties, selected from the group consisting of the following formulae

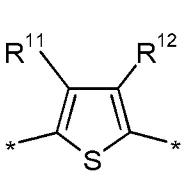
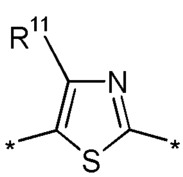
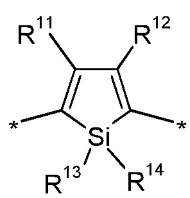
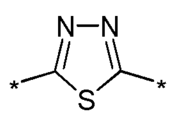

(D1) (D2) (D3) (D4)

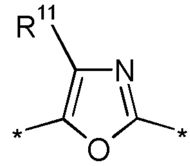 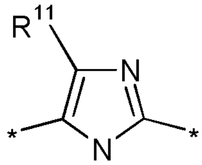 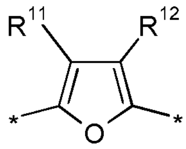

(D5) (D6) (D7)

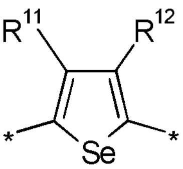 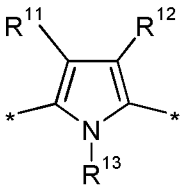 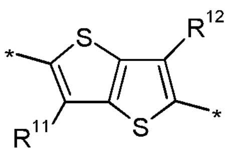

(D8) (D9) (D10)

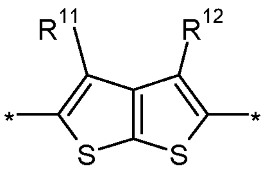 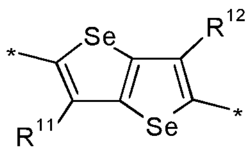 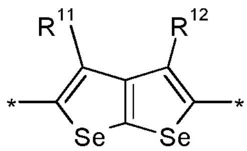

(D11) (D12) (D13)

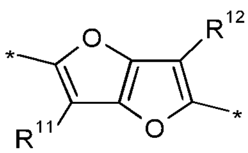 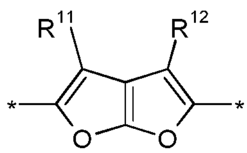 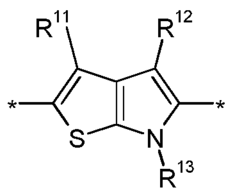

(D14) (D15) (D16)

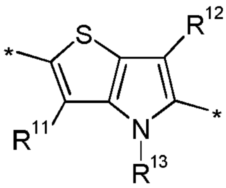 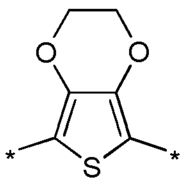 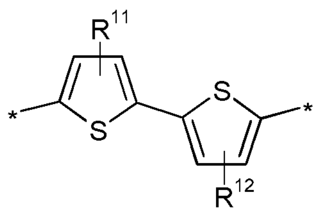

(D17) (D18) (D19)

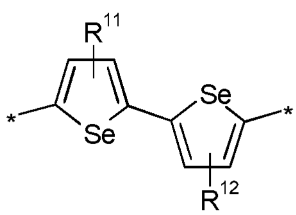 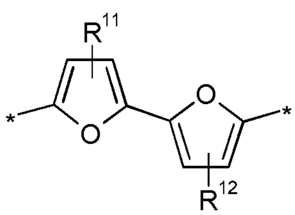 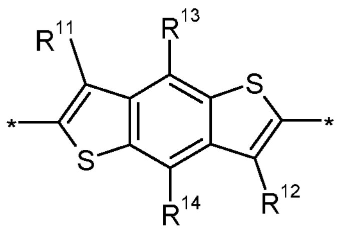

(D20) (D21) (D22)

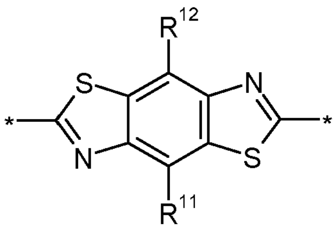
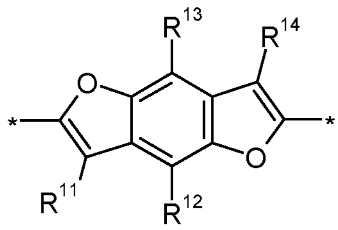
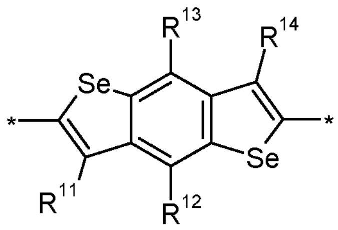

(D23) (D24) (D25)

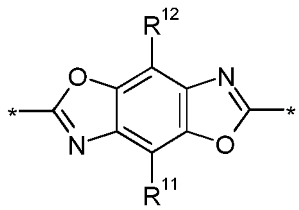
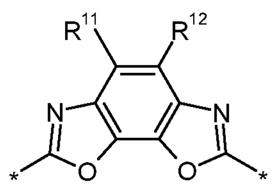
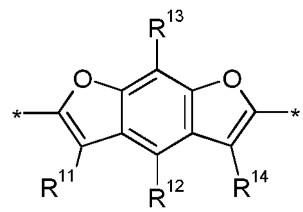

(D26) (D27) (D28)

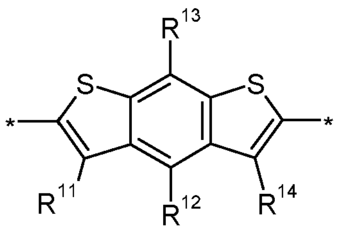
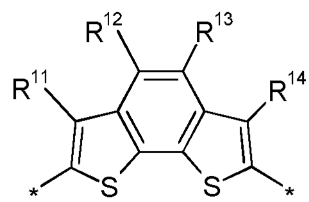
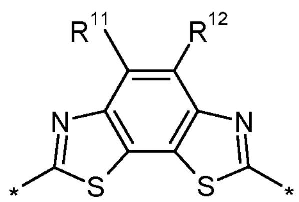

(D29) (D30) (D31)

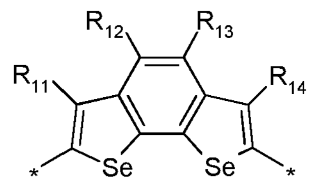
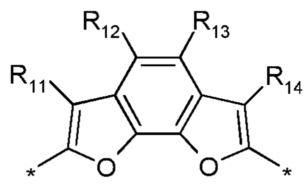
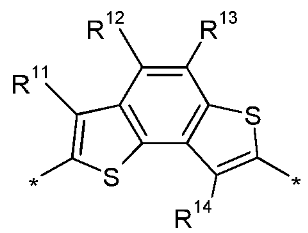

(D32) (D33) (D34)

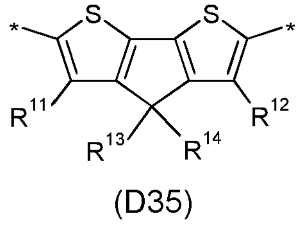
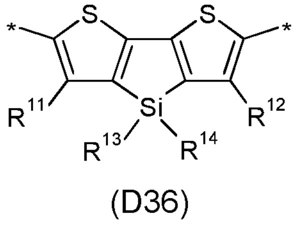
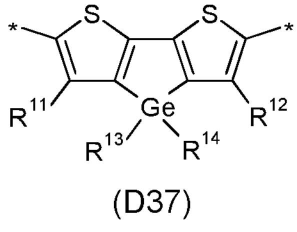

(D35) (D36) (D37)

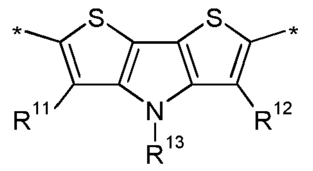
(D38)

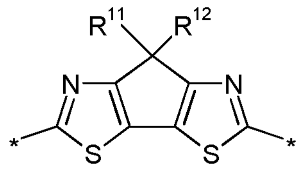
(D39)

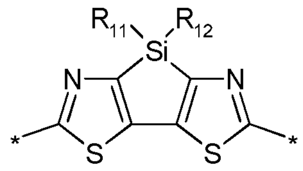
(D40)

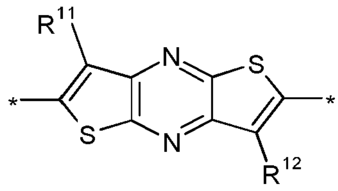
(D41)

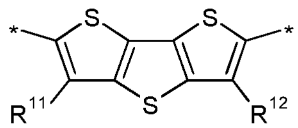
(D42)

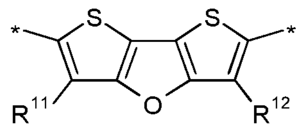
(D43)

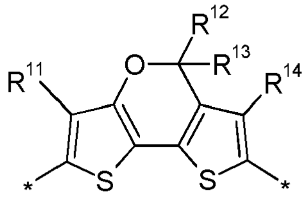
(D44)

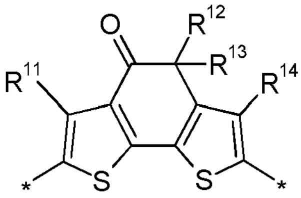
(D45)

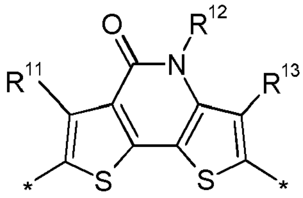
(D46)

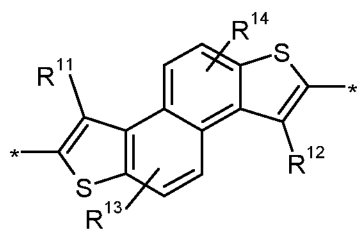
(D47)

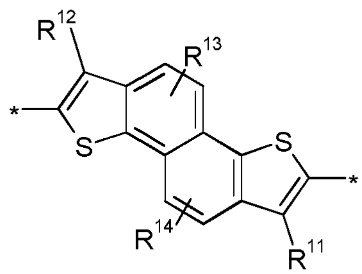
(D48)

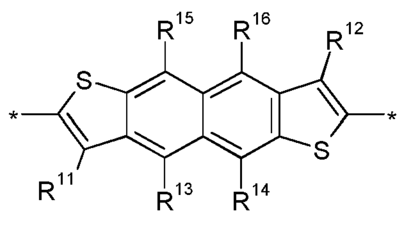
(D49)

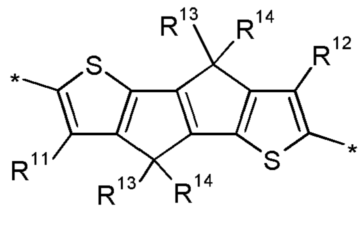
(D50)

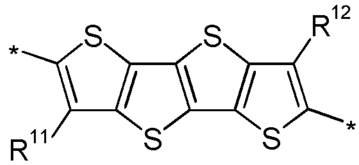

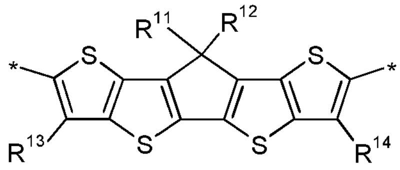

(D51) (D52)

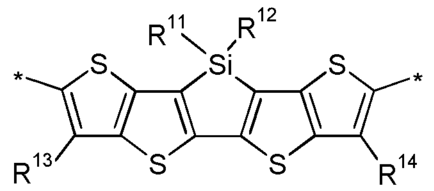

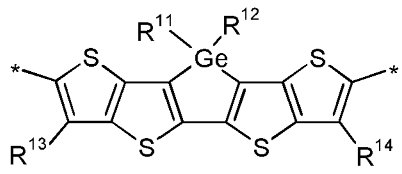

(D53) (D54)

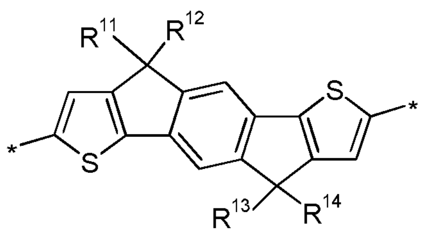

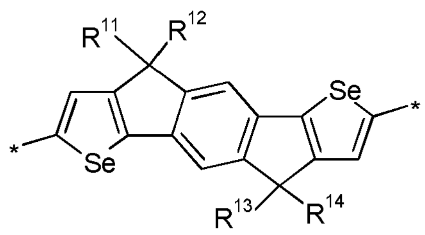

(D55) (D56)

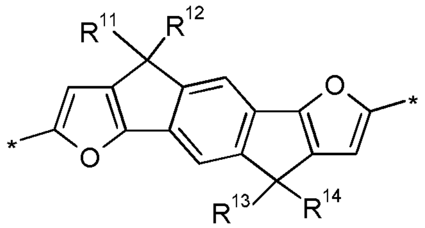

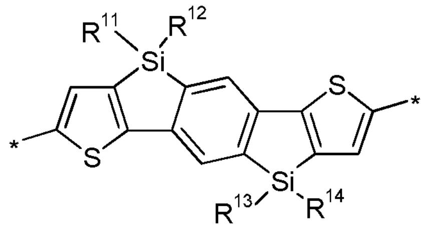

(D57) (D58)

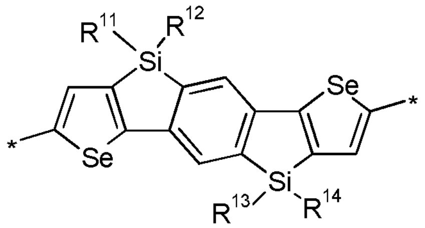

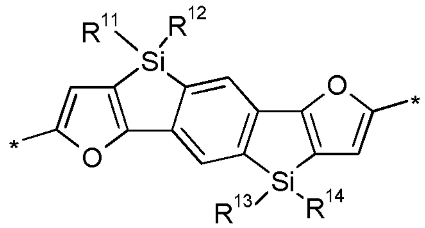

(D59) (D60)

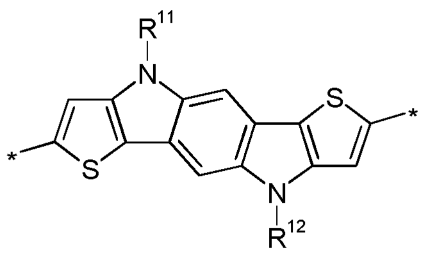

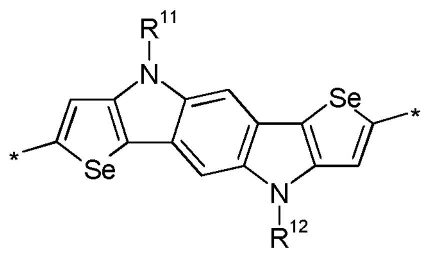

(D61) (D62)

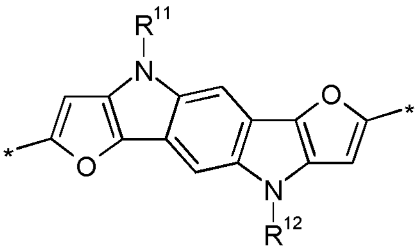

(D63)

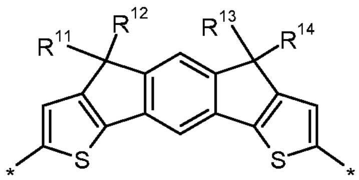

(D64)

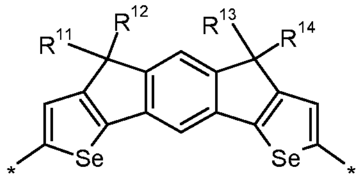

(D65)

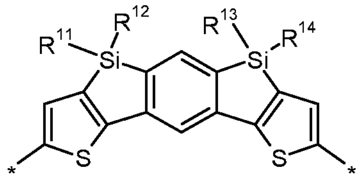

(D66)

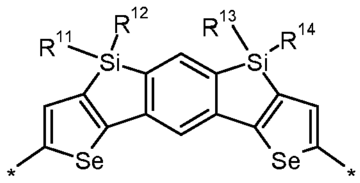

(D67)

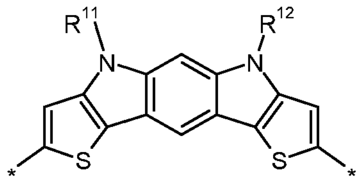

(D68)

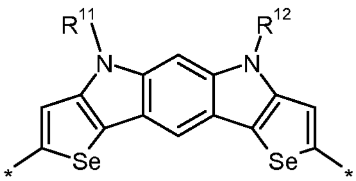

(D69)

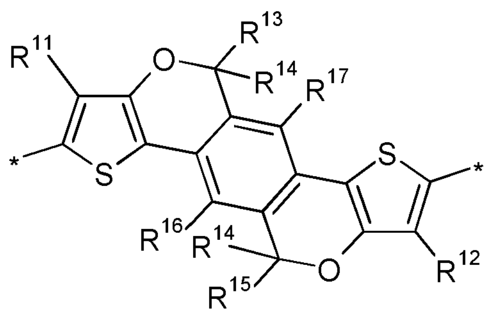

(D70)

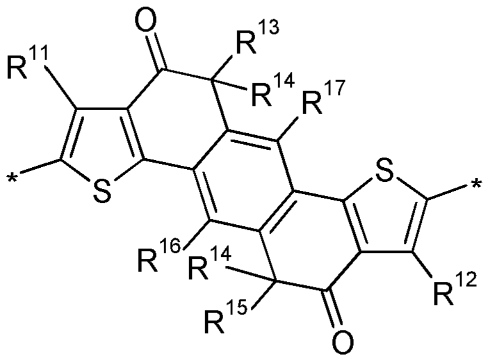

(D71)

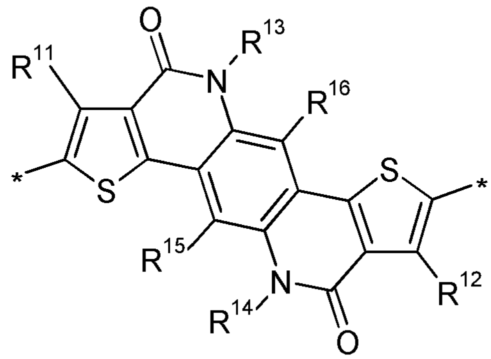

(D72)

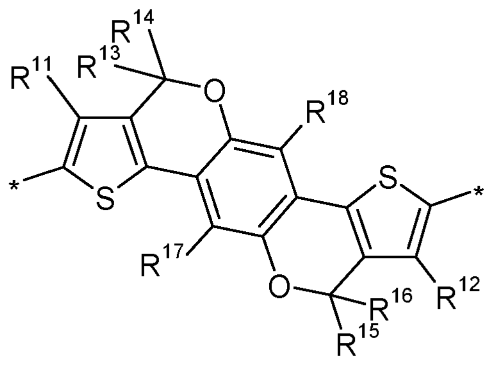

(D73)

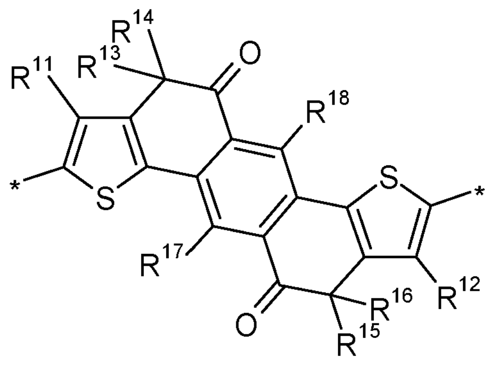

(D74)

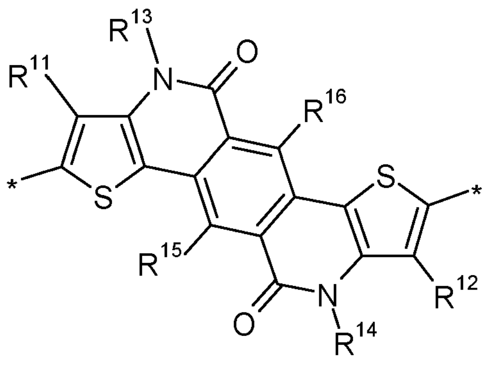

(D75)

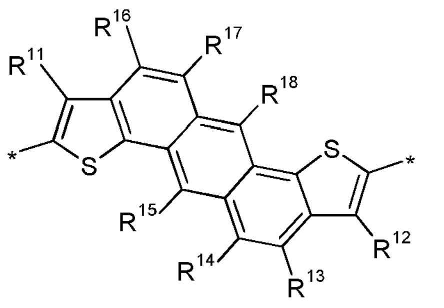

(76)

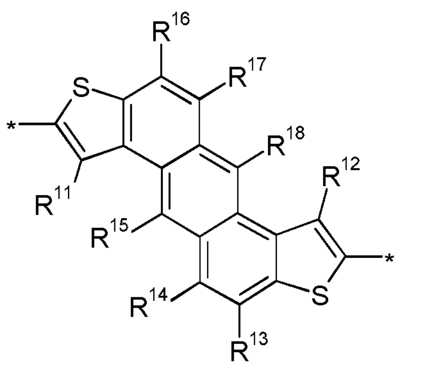

(D77)

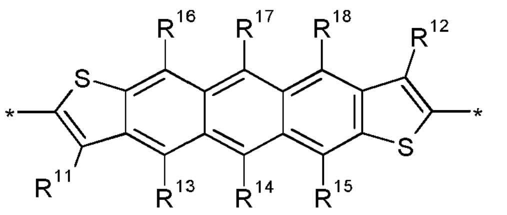

(D78)

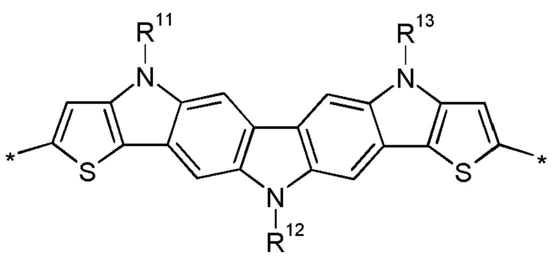

(D79)

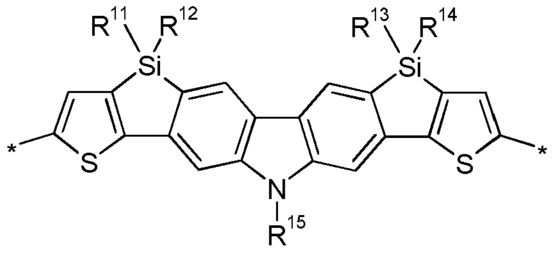

(D80)

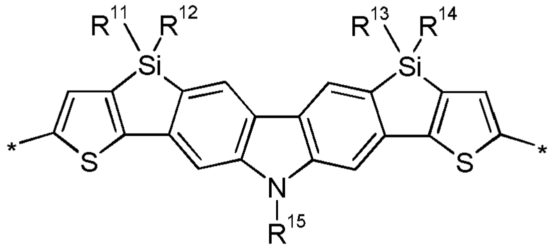

(D81)

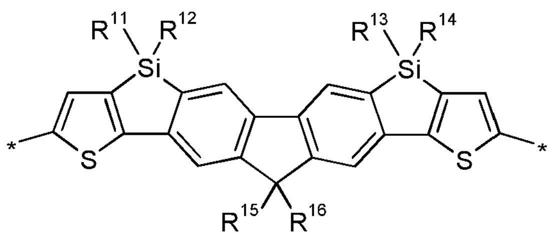

(D82)

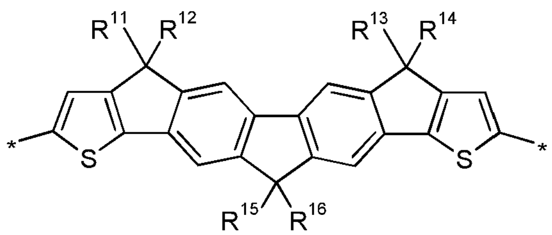

(D83)

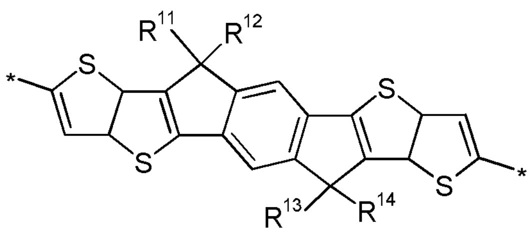

(D84)

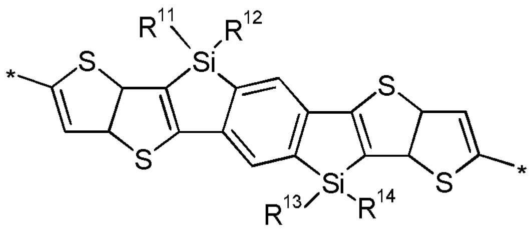

(D85)

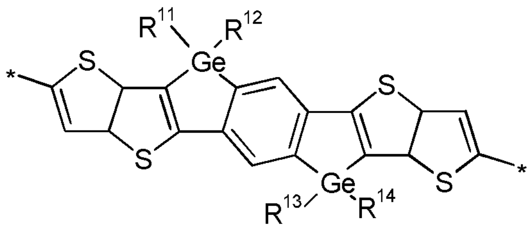

(D86)

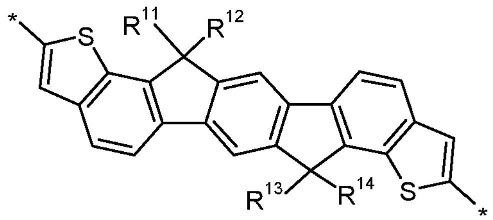

(D87)

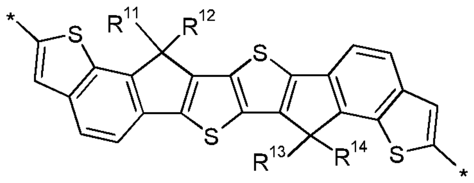

(D88)

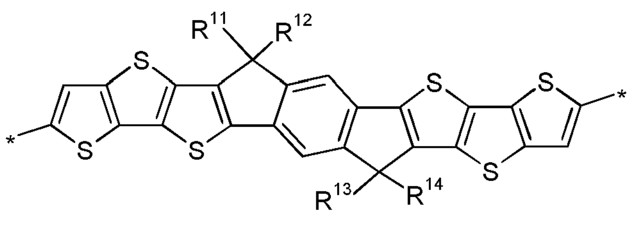

(D89)

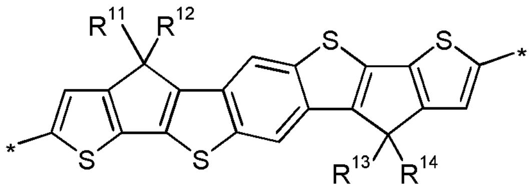

(D90)

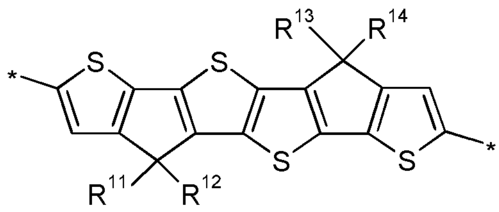

(D91)

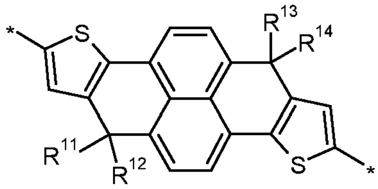

(D92)

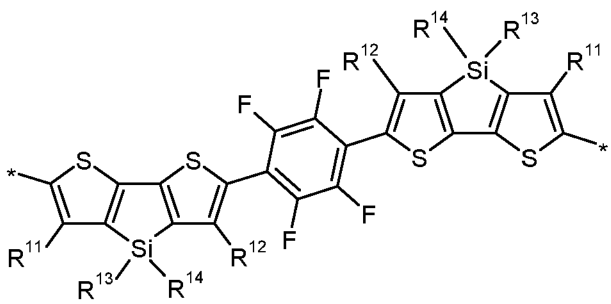

(D93)

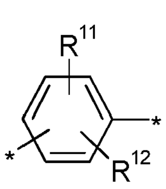

(D94)

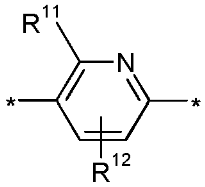

(D95)

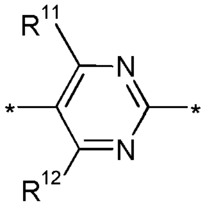

(D96)

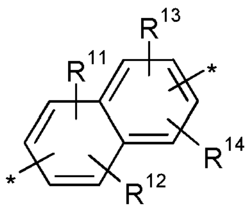

(D97)

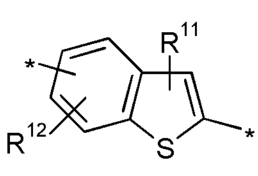

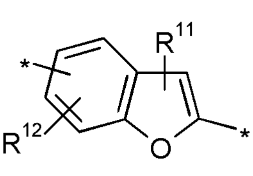

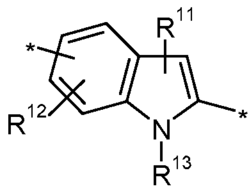

(D98) (D99) (D100)

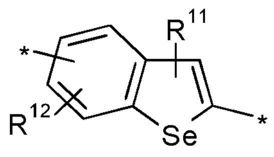 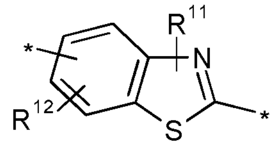 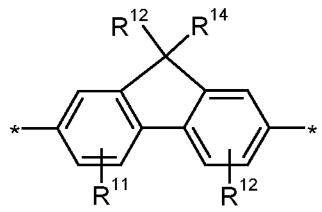

(D101) (D102) (D103)

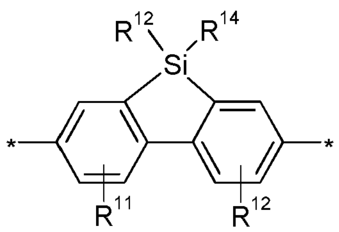 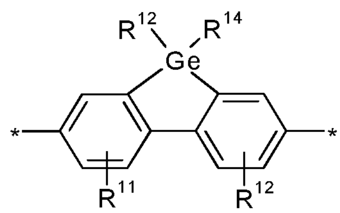 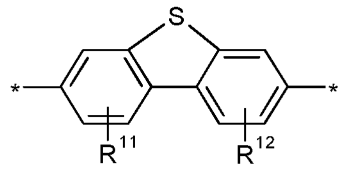

(D104) (D105) (D106)

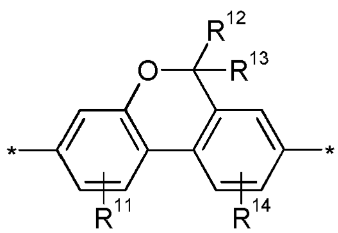 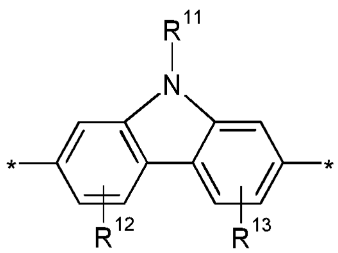 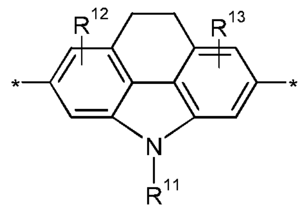

(D107) (D108) (D109)

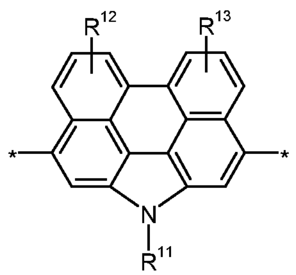 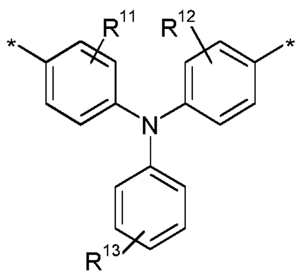 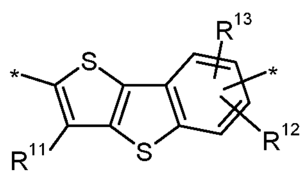

(D110) (D111) (D112)

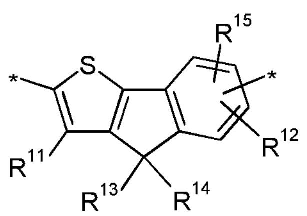 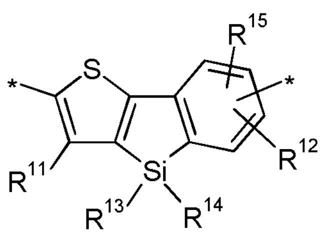 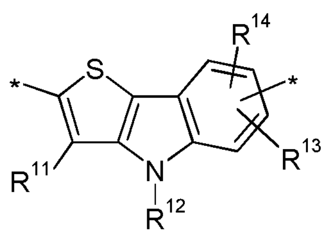

(D113) (D114) (D115)

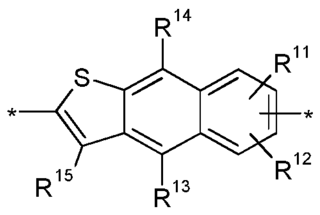

(D116)

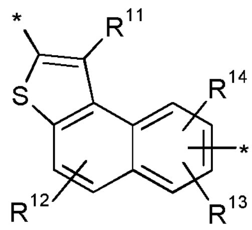

(D117)

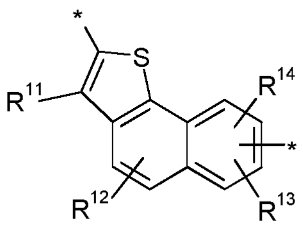

(D118)

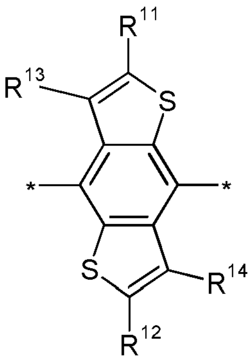

(D119)

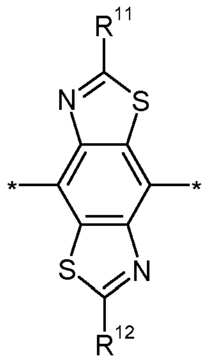

(D120)

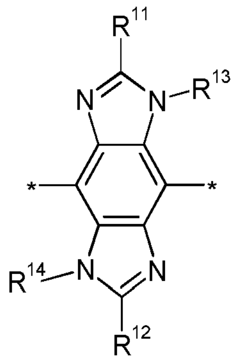

(D121)

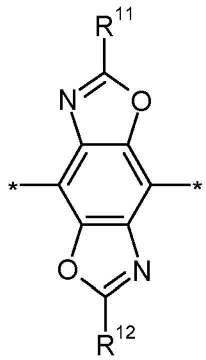

(D122)

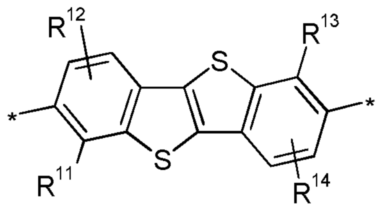

(D123)

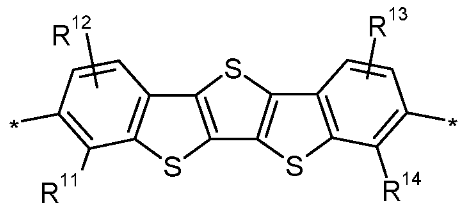

(D124)

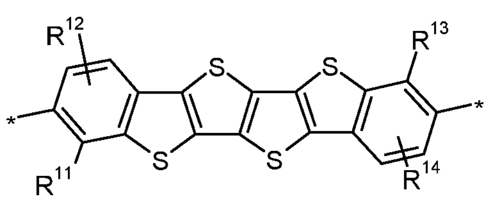

(D125)

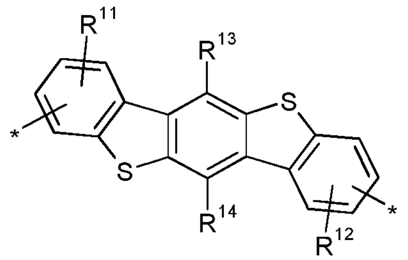

(D126)

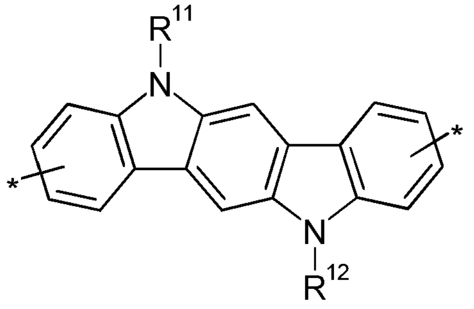

(D127)

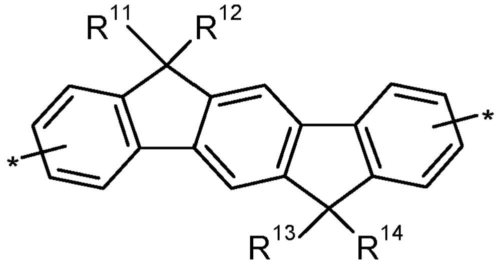

(D128)

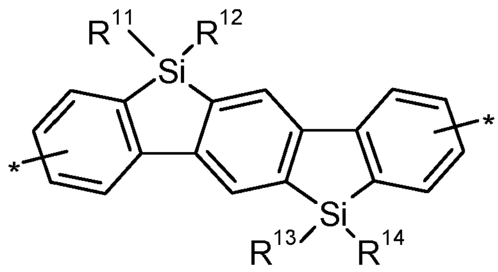

(D129)

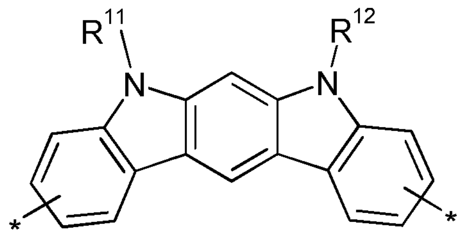

(D130)

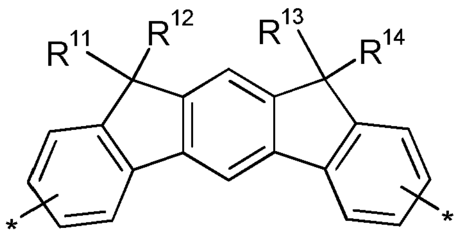

(D131)

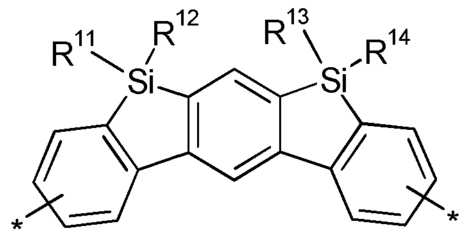

(D132)

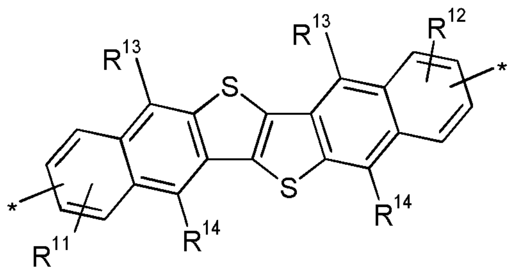

(D133)

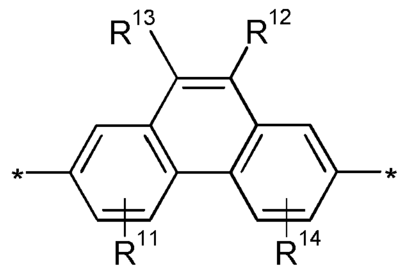

(D134)

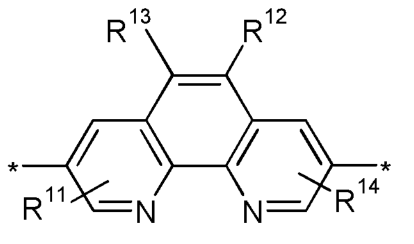

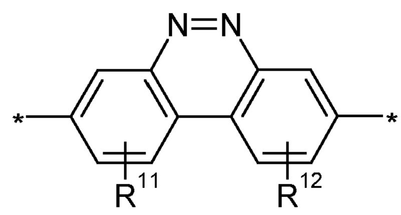

(D135) (D136)

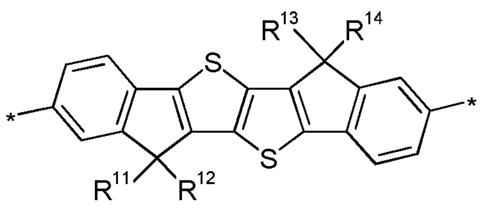

(D137)

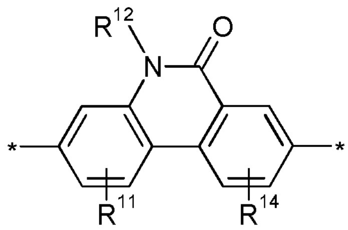

(D138)

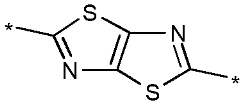

(D139)

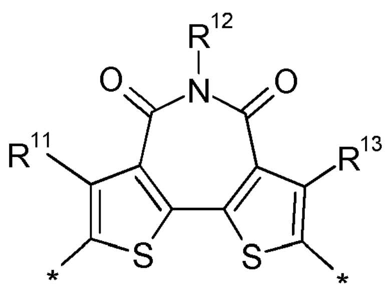

(D140)

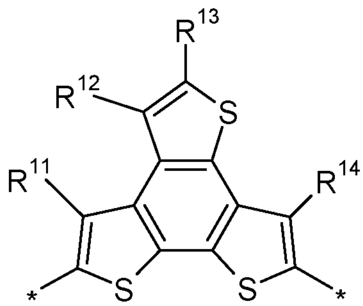

(D141)

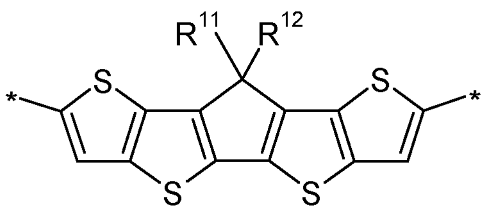

(D142)

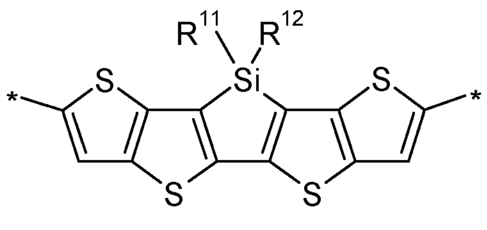

(D143)

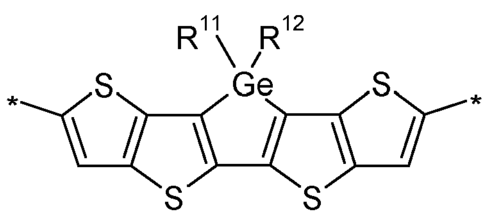

(D144)

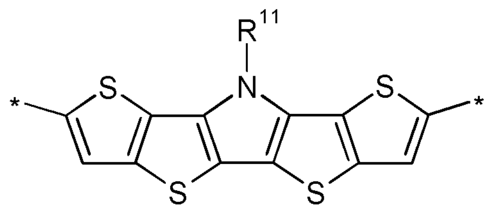

(D145)

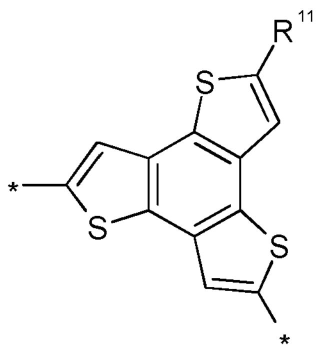

(D146)

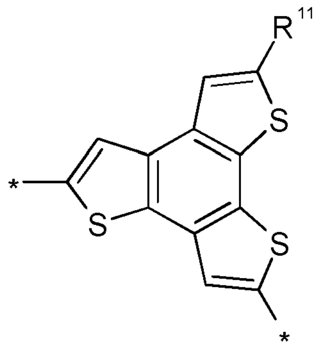

(D147)

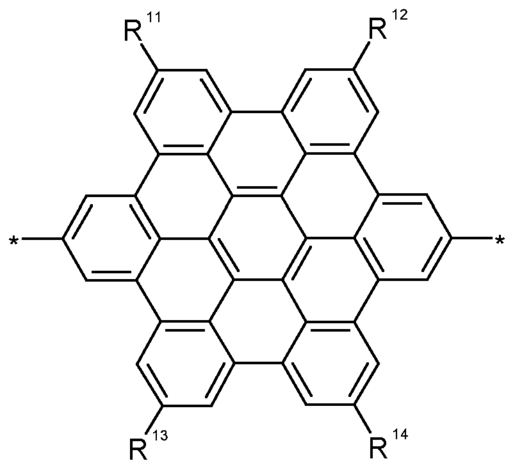

(D148)

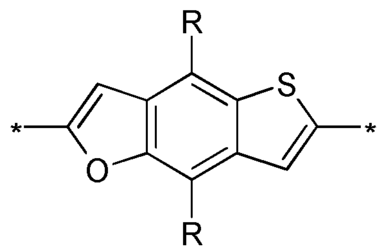

(D149)

wherein $R^{11}$, $R^{12}$, $R^{13}$, $R^{14}$, $R^{15}$, $R^{16}$, $R^{17}$ and $R^{18}$ independently of each other denote H or have one of the meanings of L as defined above and below.

Preferred donor units are selected from formulae D1, D7, D10, D11, D19, D22, D29, D30, D35, D36, D44, D55, D84, D87, D88, D89, D93, D94, D106, D111, D139, D140 or D141 wherein preferably at least one of $R^{11}$, $R^{12}$, $R^{13}$ and $R^{14}$ is different from H.

Further preferred are repeating units, oligomers, and polymers of formulae II1, II2, III, III1-III5, IV, V1, V2, V1a-V1d, VI and their subformulae wherein one or more of $Ar^1$, $Ar^2$, $Ar^3$ and $Ar^4$ denote arylene or heteroarylene, preferably having electron acceptor properties, selected from the group consisting of the following formulae

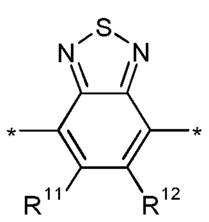

(A1)

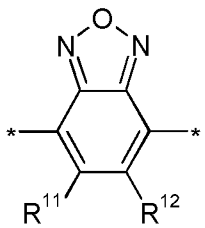

(A2)

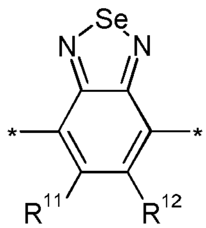

(A3)

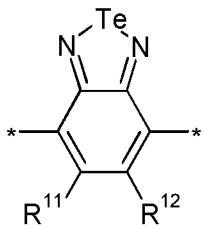

(A4)

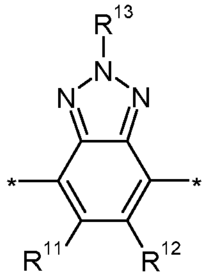

(A5)

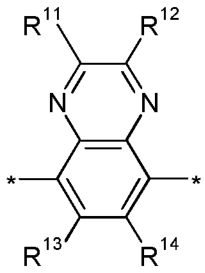

(A6)

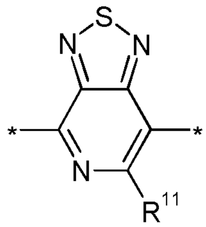

(A7)

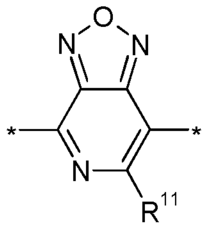

(A8)

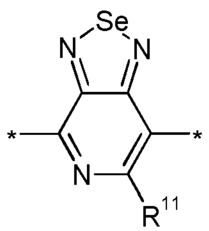

(A9)

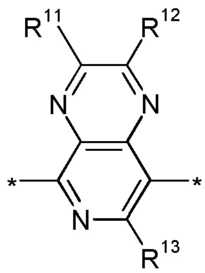

(A10)

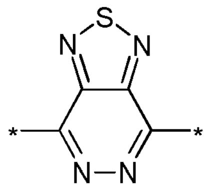

(A11)

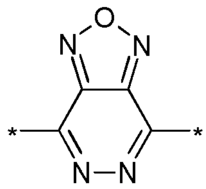

(A12)

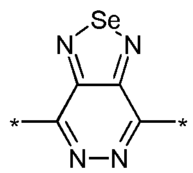

(A13)

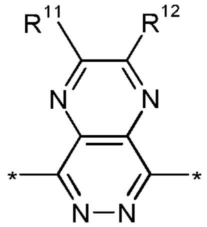

(A14)

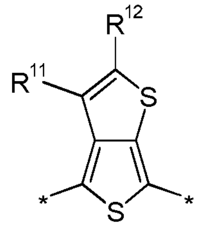

(A15)

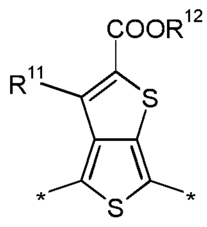

(A16)

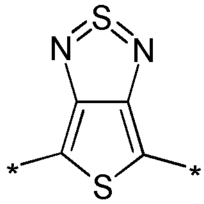

(A17)

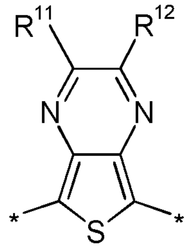

(A18)

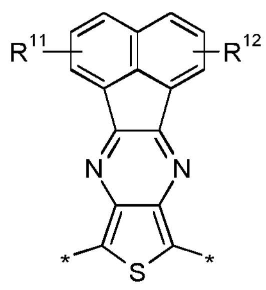

(A19)

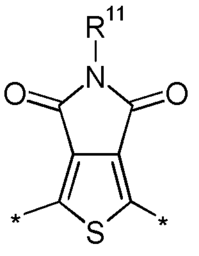

(A20)

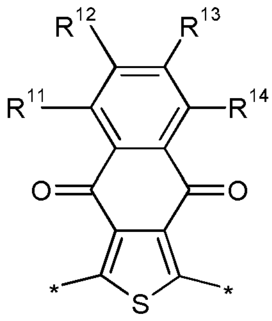

(A21)

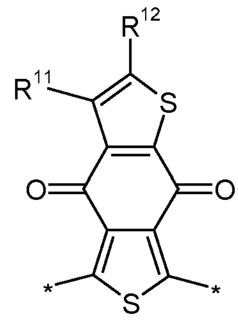

(A22)

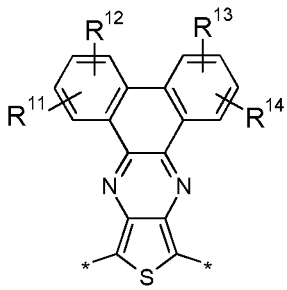

(A23)

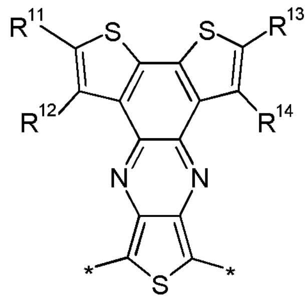

(A24)

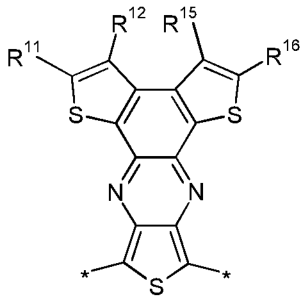

(A25)

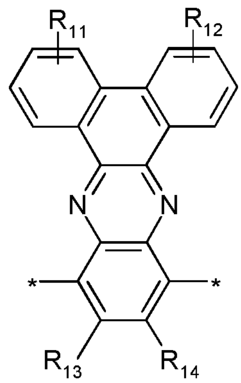

(A26)

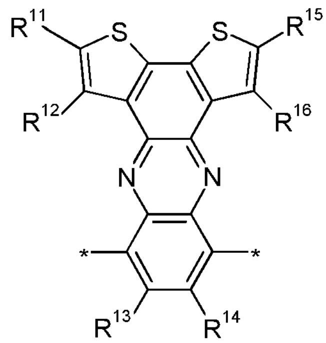

(A27)

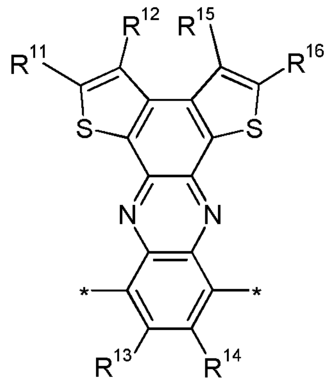

(A28)

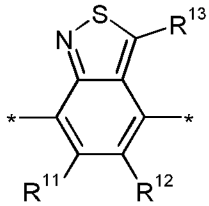

(A29)

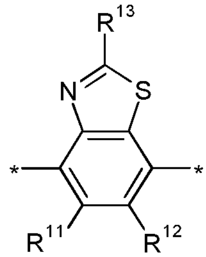

(A30)

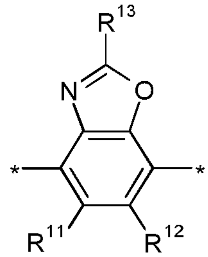

(A31)

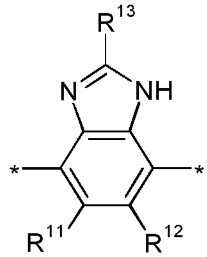

(A32)

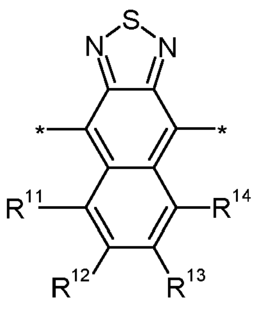

(A33)

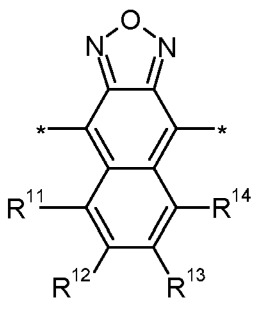

(A34)

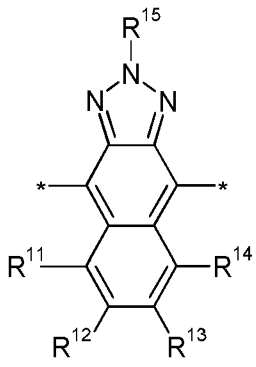

(A35)

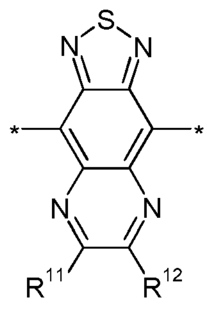

(A36)

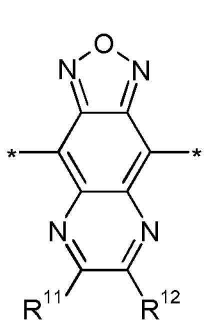

(A37)

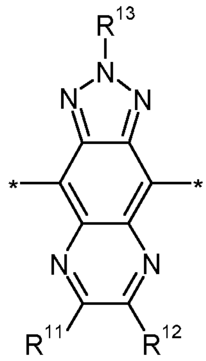

(A38)

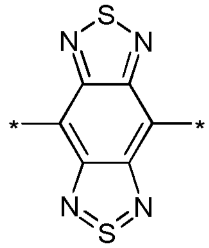

(A39)

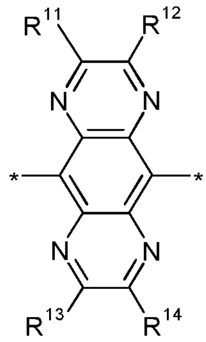

(A40)

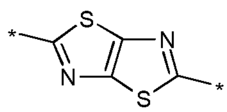

(A41)

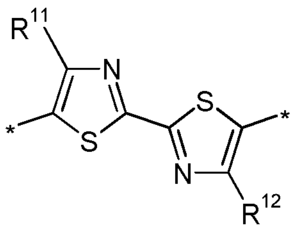

(A42)

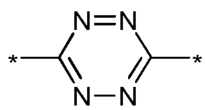

(A43)

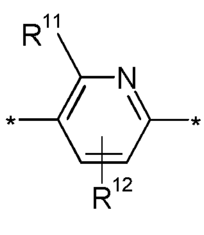

(A44)

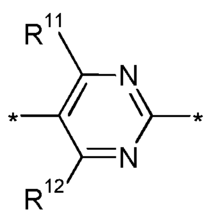

(A45)

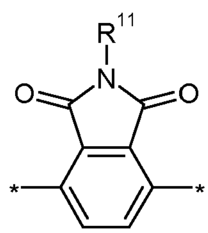

(A46)

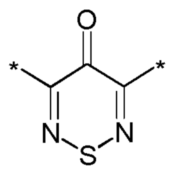

(A47)

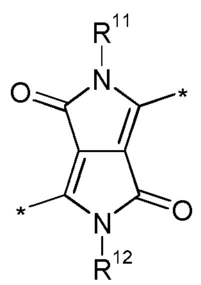

(A48)

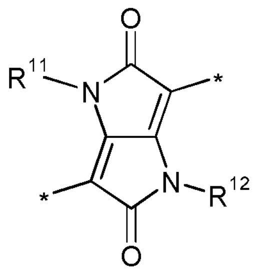

(A49)

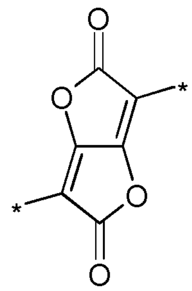

(A50)

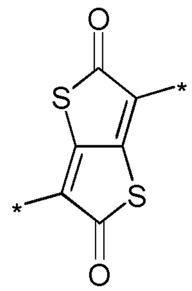

(A51)

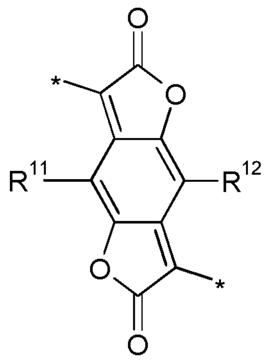

(A52)

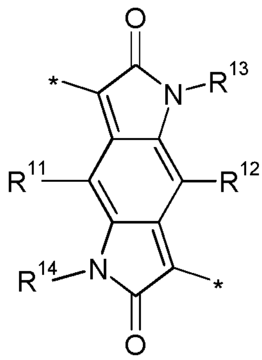

(A53)

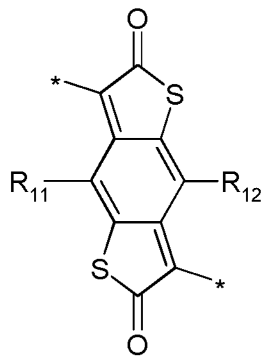

(A54)

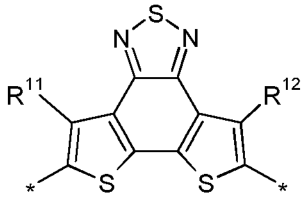

(A55)

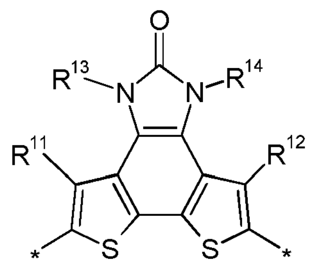

(A56)

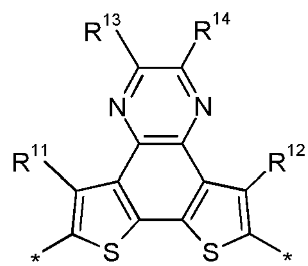

(A57)

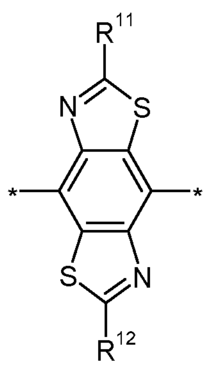

(A58)

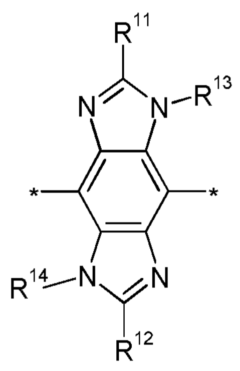

(A59)

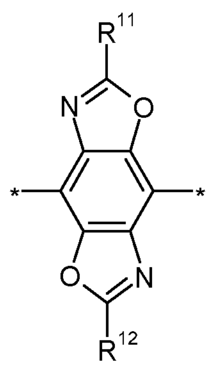

(A60)

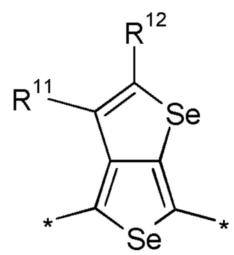

(A61)

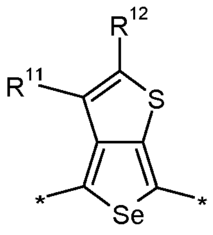

(A62)

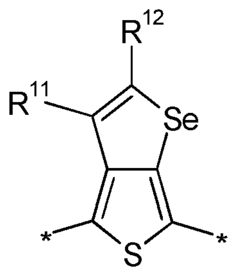

(A63)

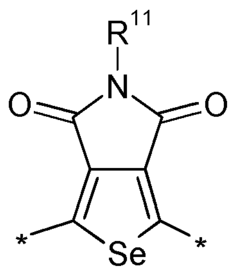

(A64)

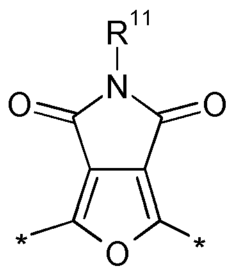

(A65)

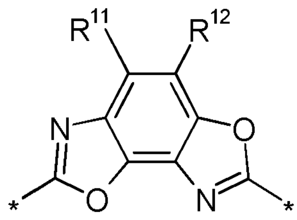

(A66)

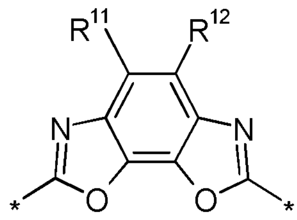

(A67)

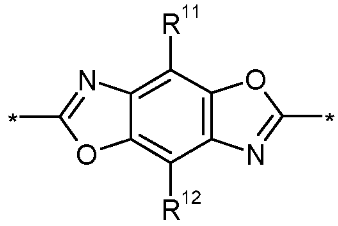

(A68)

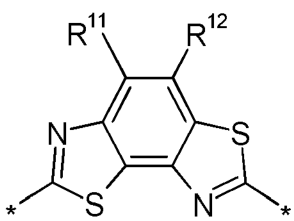

(A69)

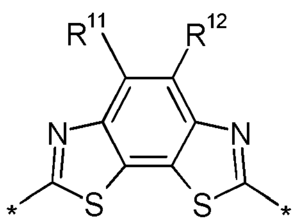

(A70)

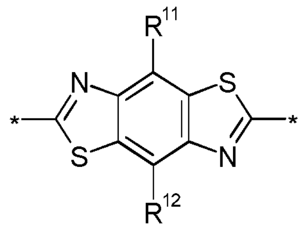

(A71)

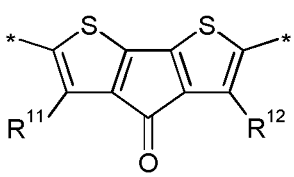

(A72)

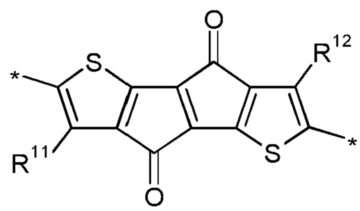

(A73)

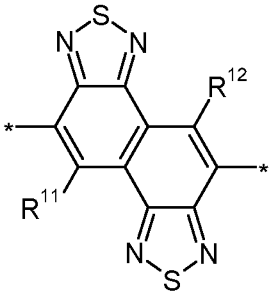

(A74)

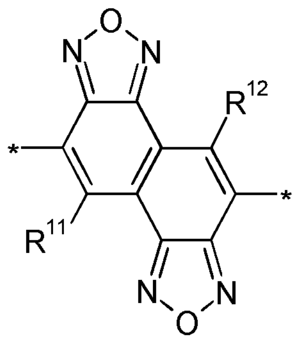

(A75)

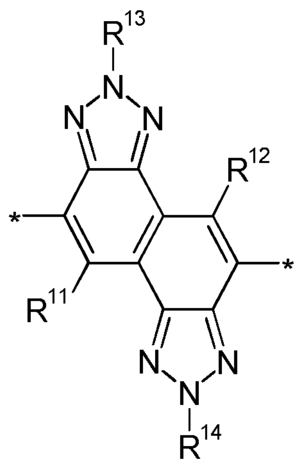

(A76)

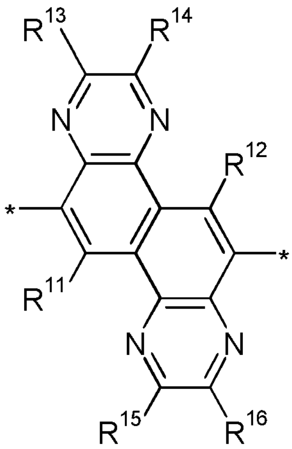

(A77)

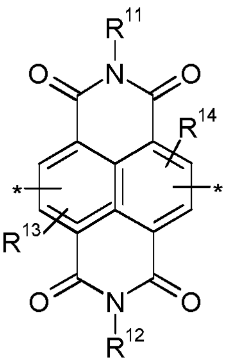

(A78)

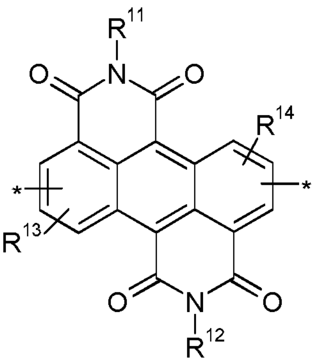

(A79)

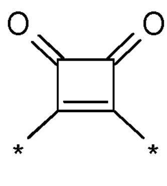

(A80)

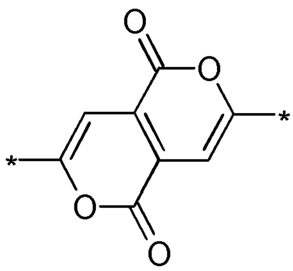

(A81)

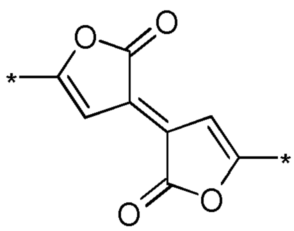

(A82)

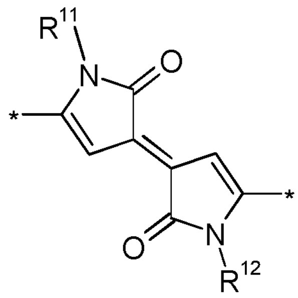

(A83)

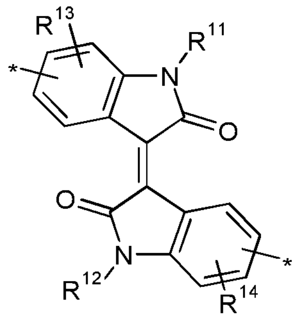

(A84)

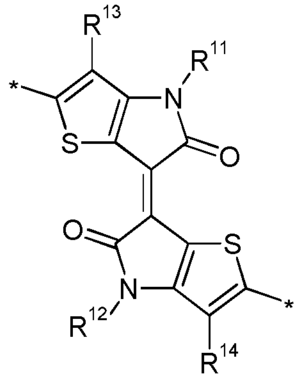

(A85)

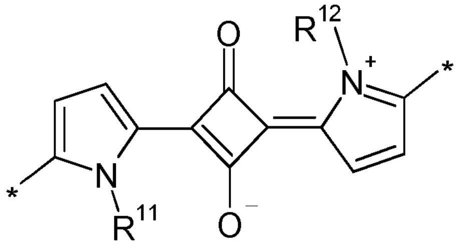

(A86)

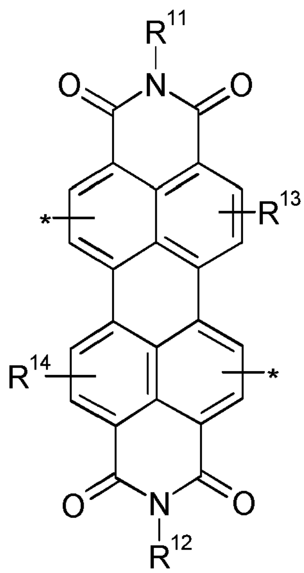

(A87)

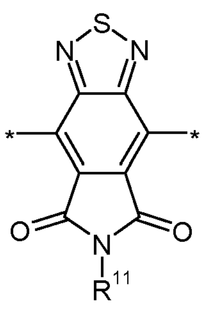

(A88)

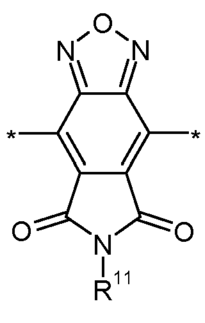

(A89)

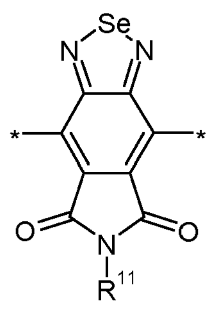

(A90)

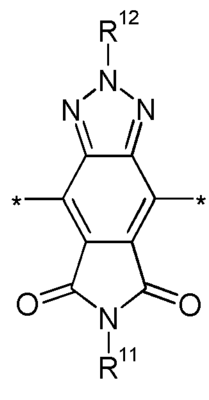

(A91)

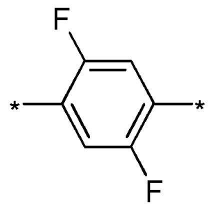
(A92)

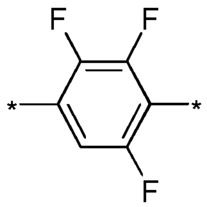
(A93)

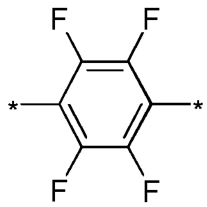
(A94)

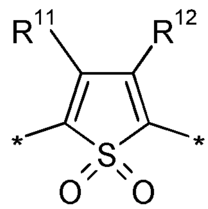
(A95)

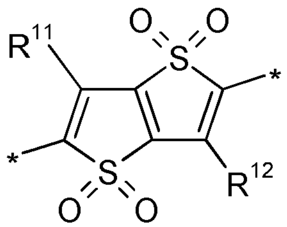
(A96)

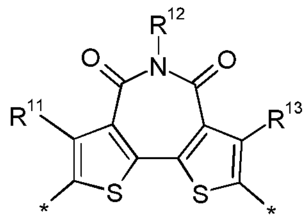
(A97)

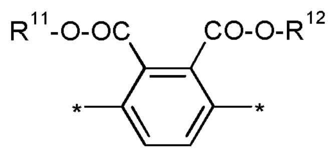
(A98)

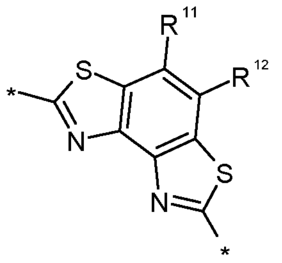
(A99)

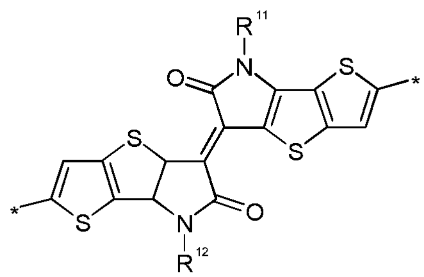
(A100)

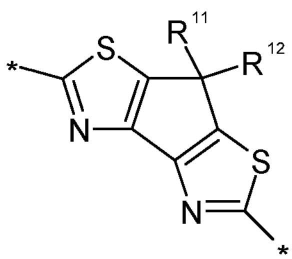
(A101)

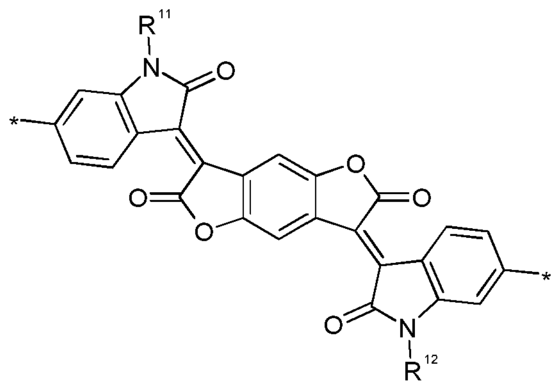
(A102)

wherein $R^{11}$, $R^{12}$, $R^{13}$, $R^{14}$, $R^{15}$ and $R^{16}$ independently of each other denote H or have one of the meanings of L as defined above and below.

Preferred acceptor units are selected from formulae A1, A6, A7, A15, A16, A20, A74, A84, A88, A92 or A98 wherein preferably at least one of $R^{11}$, $R^{12}$, $R^{13}$ and $R^{14}$ is different from H.

Further preferred are repeating units, oligomers, and polymers of formulae II1, II2, III, III1-III5, IV, V1, V2, V1a-V1d, VI and their subformulae wherein one or more of $Ar^1$, $Ar^2$, $Ar^3$ and $Ar^4$ denote arylene or heteroarylene selected from the group consisting of the following formulae

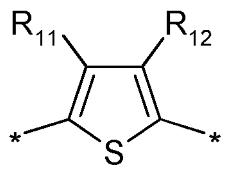

Sp1

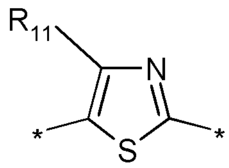

Sp2

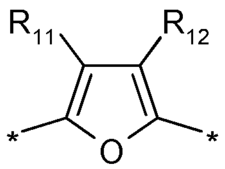

Sp3

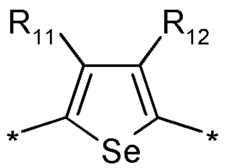

Sp4

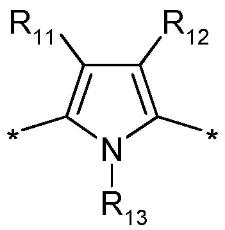

Sp5

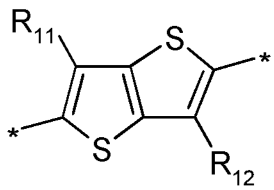

Sp6

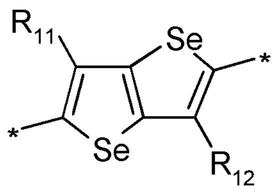

Sp7

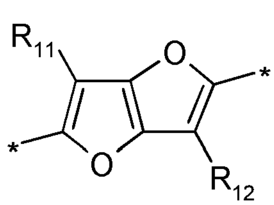

Sp8

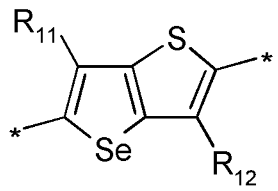

Sp9

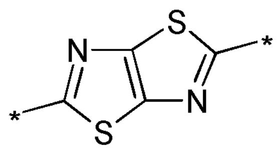

Sp10

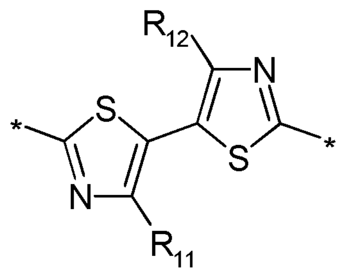

Sp11

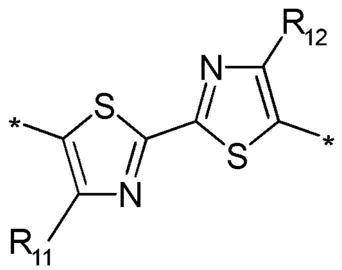

Sp12

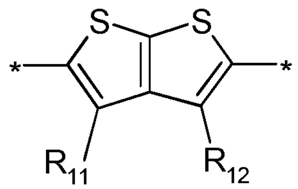

Sp13

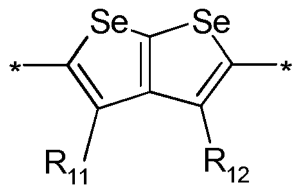

Sp14

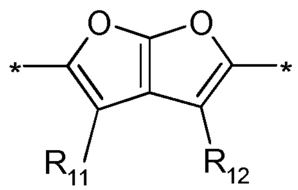

Sp15

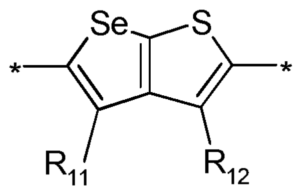

Sp16

wherein $R^{11}$ and $R^{12}$ independently of each other denote H or have one of the meanings of L as defined above and below.

Very preferred are units selected from formulae Sp1, Sp6, Sp10, Sp13, wherein preferably one of $R^{11}$ and $R^{12}$ is H or both $R^{11}$ and $R^{12}$ are H.

Further preferred are repeating units, monomers and polymers of formulae II1, II2, III, III1-III5, IV, V1, V2, V1a-V1d and their subformulae wherein

a) one or more of $Ar^1$, $Ar^2$, $Ar^3$ and $Ar^4$ denote arylene or heteroarylene, preferably having electron donor properties, selected from the group consisting of the formulae D1-D145, very preferably of the formulae D1, D7, D10, D11, D19, D22, D29, D30, D35, D36, D44, D55, D84, D87, D88, D89, D93, D94, D106, D111, D139, D140 and D141, and/or

b) one or more of $Ar^1$, $Ar^2$, $Ar^3$ and $Ar^4$ denote arylene or heteroarylene, preferably having electron accpetor properties, selected from the group consisting of the formulae A1 -A98, very preferably of the formulae A1, A6, A7, A15, A16, A20, A74, A84, A88, A92 and A98,
and

c) one or more of $Ar^1$, $Ar^2$, $Ar^3$ and $Ar^4$ denote arylene or heteroarylene selected from the group consisting of the formulae Sp1-Sp15, very preferably of the formulae Sp1, Sp6, Sp10 and Sp13.

Further preferred are polymers of formula III3 and III5 wherein $Ar^1$ and $Ar^2$ have the same meaning and are selected from formulae D1, D7, D10, D11, D19, D22, D29, D30, D35, D36, D44, D55, D84, D87, D88, D89, D93, D94, D106, D111, D139, D140 and D141, and $R^1$ and $R^2$ denote R or -OR, and $R^3$ and $R^4$ denote C(=O)-R or C(=O)-OR with R being as defined above.

Further preferred are oligomers of formula VI wherein $Ar^{1-10}$ are selected from the following groups

a) the group consisting of the formulae D1-D145, very preferably of the formulae D1, D7, D10, D11, D19, D22, D29, D30, D35, D36, D44, D55, D84, D87, D88, D89, D93, D94, D106, D111, D139, D140 and D141,

b) the group consisting of the formulae A1-A98, very preferably of the formulae A1, A6, A7, A15, A16, A20, A74, A84, A88, A92 or A98,

c) the group consisting of the formulae Sp1-Sp15, very preferably of the formulae Sp1, Sp6, Sp10 and Sp13.

The polymers according to the present invention can be prepared for example by copolymerising one or more monomers of formula V1, V2 or V1a-V1d with each other or with one or monomers of the following formulae in an aryl-aryl coupling reaction

$R^7$-$Ar^1$-$R^8$ MI

$R^7$-$Ar^2$-$R^8$ MII

$R^7$-$Ar^3$-$R^8$ MIII

$R^7$-$Ar^4$-$R^8$ MIV

wherein $Ar^{1-4}$, $R^7$ and $R^8$ have the meanings given in formula II2 and V1 or one of the preferred meanings given above and below.

The polymer according to the present invention can be synthesized according to or in analogy to methods that are known to the skilled person and are described in the literature. Other methods of preparation can be taken from the examples.

For example, the polymer can be suitably prepared by aryl-aryl coupling reactions, such as Yamamoto coupling, C-H activation coupling, Suzuki coupling, Stille coupling, Sonogashira coupling, Heck coupling or Buchwald coupling.

Suzuki coupling, Stille coupling and Yamamoto coupling are especially preferred. The monomers which are polymerised to form the repeat units of the polymers can be prepared according to methods which are known to the person skilled in the art.

Preferably the polymer is prepared from monomers selected from formulae V1, V2, V1a-d and MI-MIV as described above.

Another aspect of the invention is a process for preparing a polymer by coupling one or more identical or different monomers selected from formulae V1, V2, V1a-d with each other and/or with one or more comonomers, preferably selected from formulae MI-MIV, in a polymerisation reaction, preferably in an aryl-aryl coupling reaction.

Preferred aryl-aryl coupling and polymerisation methods used in the processes described above and below are Yamamoto coupling, Kumada coupling, Negishi coupling, Suzuki coupling, Stille coupling, Sonogashira coupling, Heck coupling, C-H activation coupling, Ullmann coupling or Buchwald coupling. Especially preferred are Suzuki coupling, Negishi coupling, Stille coupling and Yamamoto coupling. Suzuki coupling is described for example in WO 00/53656 A1. Negishi coupling is described for example in J. Chem. Soc., Chem. Commun., 1977, 683-684. Yamamoto coupling is described in for example in T. Yamamoto et al., Prog. Polym. Sci., 1993, 17, 1153-1205, or WO 2004/022626 A1.Stille coupling is described for example in Z. Bao et al., J. Am. Chem. Soc., 1995, 117, 12426-12435. C-H activation is described for example for example in M. Leclerc et al, Angew. Chem. Int. Ed. 2012, 51, 2068 - 2071.For example, when using Yamamoto coupling, monomers having two reactive halide groups are preferably used. When using Suzuki coupling, monomers having two reactive boronic acid or boronic acid ester groups or two reactive halide groups are preferably used. When using Stille coupling, monomers having two reactive stannane groups or two reactive halide groups are preferably used. When using Negishi coupling, monomers having two reactive organozinc groups or two reactive halide groups are preferably used. When synthesizing a linear polymer by C-H activation polymerisation, preferably a monomer as described above is used wherein at least one reactive group is an activated hydrogen bond.

Preferred catalysts, especially for Suzuki, Negishi or Stille coupling, are selected from Pd(0) complexes or Pd(II) salts. Preferred Pd(0) complexes are those bearing at least one phosphine ligand such as $Pd(Ph_3P)_4$. Another preferred phosphine ligand is tris(*ortho*-tolyl)phosphine, i.e. $Pd(o\text{-}Tol_3P)_4$. Preferred Pd(II) salts include palladium acetate, i.e. $Pd(Oac)_2$ or trans-di($\mu$-acetato)-bis[o-(di-o-tolylphosphino)benzyl]dipalladium(II). Alternatively the Pd(0) complex can be prepared by mixing a Pd(0) dibenzylideneacetone complex, for example tris(dibenzyl-ideneacetone)dipalladium(0), bis(dibenzylideneacetone)palladium(0), or Pd(II) salts e.g. palladium acetate, with a phosphine ligand, for example triphenylphosphine, tris(*ortho*-tolyl)phosphine, tris(o-methoxyphenyl)phosphine or tri(tert-butyl)phosphine. Suzuki polymerisation is performed in the presence of a base, for example sodium carbonate, potassium carbonate, cesium carbonated, lithium hydroxide, potassium phosphate or an organic base such as tetraethylammonium carbonate or tetraethylammonium hydroxide. Yamamoto polymerisation employs a Ni(0) complex, for example bis(1,5-cyclooctadienyl) nickel(0).

Suzuki, Stille or C-H activation coupling polymerisation may be used to prepare homopolymers as well as statistical, alternating and block random copolymers. Statistical, random block copolymers or block copolymers can be prepared for example from the above monomers, wherein one of the reactive groups is halogen and the other reactive group is a C-H activated bond, boronic acid, boronic acid derivative group or and alkylstannane. The synthesis of statistical, alternating and block copolymers is described in detail for example in WO 03/048225 A2 or WO 2005/014688 A2.

As alternatives to halogen as described above, leaving groups of formula $-O-SO_2Z^1$ can be used wherein $Z^1$ is as defined above. Particular examples of such leaving groups are tosylate, mesylate and triflate.

Preferred polymerisation conditions lead to alternating polymers which are particularly preferred for OTFT application, whereas statistical block co-polymers are prepared preferably for OPV and OPD application. Preferred polycondensation are Suzuki coupling, Stille coupling, Sonogashira coupling, Heck coupling or Buchwald coupling, Negishi coupling or C-H activation coupling where the first set of reactive groups is composed of - Cl, -Br, -I, O-tosylate, O-triflate, O-mesylate and O-nonaflate and the second set of reactive groups is composed of -H, $-SiR_2F$, $-SiRF_2$, $-B(OR)_2$ , -CR=CHR', -C≡CH, -ZnX, -MgX and $-Sn(R_3)$. If a Yamamoto coupling reaction is used to prepare the polymer, the reactive monomer ends are both composed independently of -Cl, -Br, -I, O-tosylate, O-triflate, O-mesylate and O-nonaflate.

Suitable and preferred methods for preparing compounds according to the present invention are illustrated in the reaction schemes below.

Schemes 1-4 show the synthesis of the units of formula I. Therein R and R' have one of the meanings of $R^1$ given in formula I, for example alkyl, $X^{1,2}$ are Br, $SnR_3$ or $B(OR)_2$, $Ar^{1,4}$ are arylene or heteroarylene as defined for example in formula 111.

Synthesis of the 4,8-benzo[1,2-d;4,5-d']bisthiazole core can be achieved as shown by Malika Jeffries-EL in J. Org. Chem., 2010, 75 (2), 495-497, as exemplarily illustrated in Scheme 1.

Scheme 1

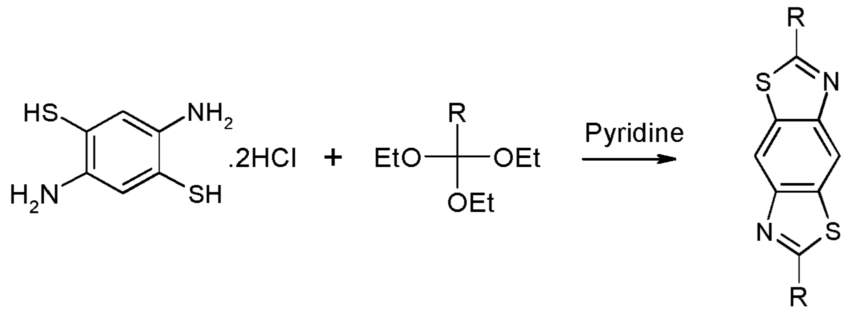


An alternative synthesis was published by Persephonis et al., Org. Lett., 2014, 16 (24), 6358-6361, as shown in Scheme 2.

Scheme 2

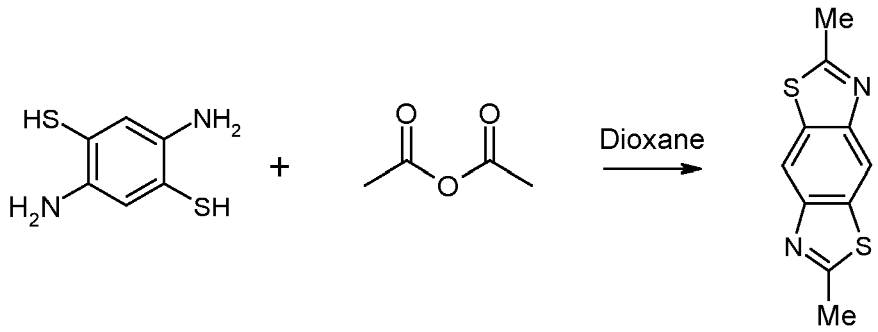


Synthesis of the alky-functionalised core can be achieved as shown in Scheme 3.

Scheme 3

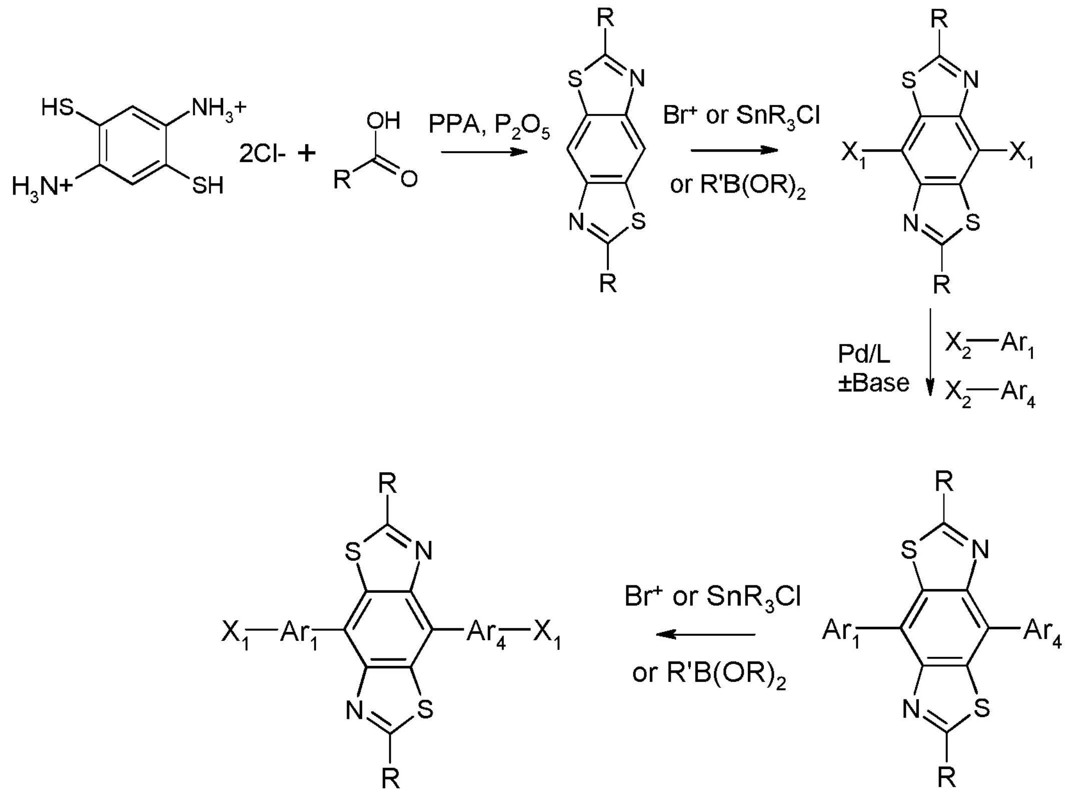


Synthesis of the ester-functionalised core can be achieved as shown in Scheme 4.

Scheme 4

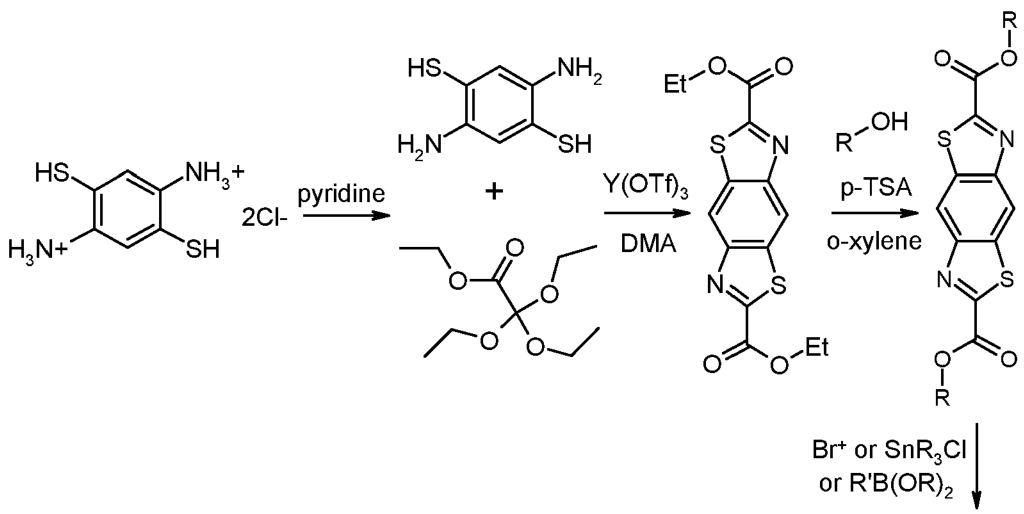

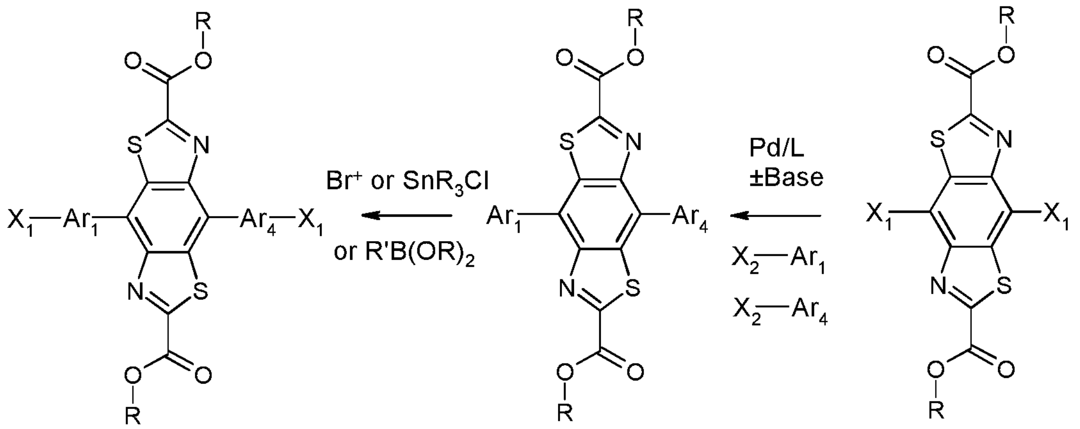


The synthesis of alternating co-polymers of the 4,8-benzo[1,2-d;4,5-d']bisthiazole core is shown in Scheme 5. Therein X1 = Br and X2 = $SnR_3$ or X1 = Br and X2 = $B(OR)_2$ or X1 = $SnR_3$ and X2 = Br or X1 = $B(OR)_2$ and X2 = Br or X1 = Br or Cl, $Ar_x$ is optionally substituted arylene or heteroarylene as defined in formula 111, and a-d are 0 or 1 with $a+b+c+d \geq 0$, and R is for example alkyl.

<u>Scheme 5</u>

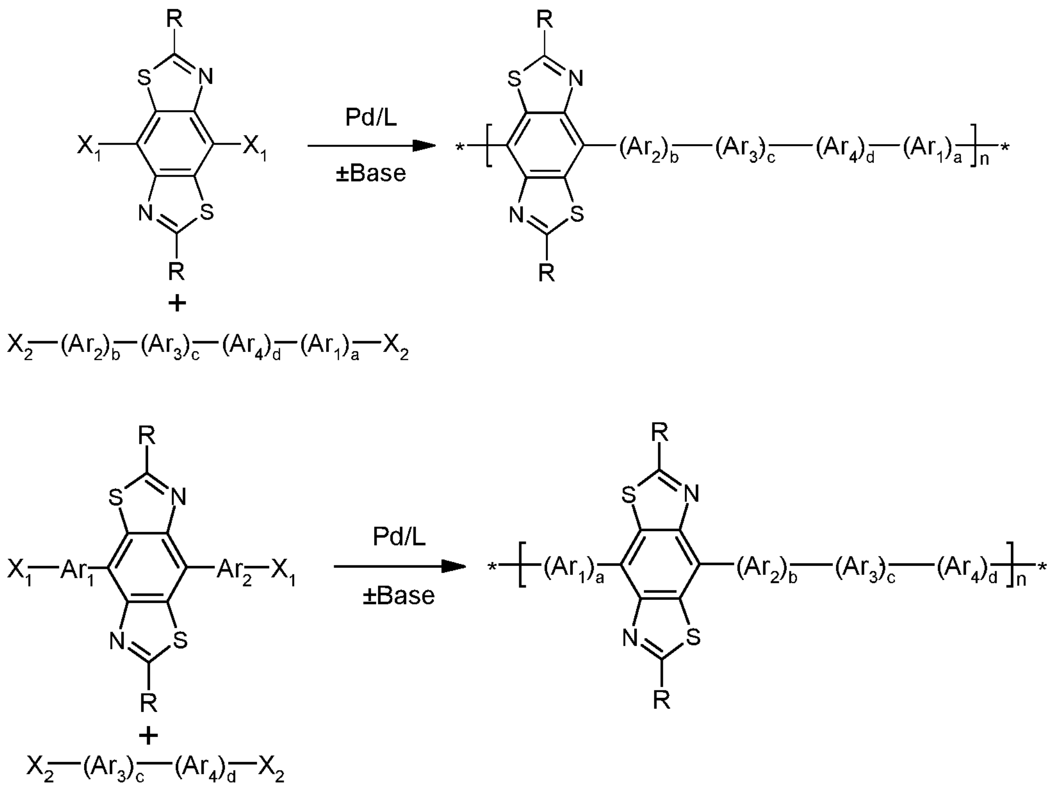

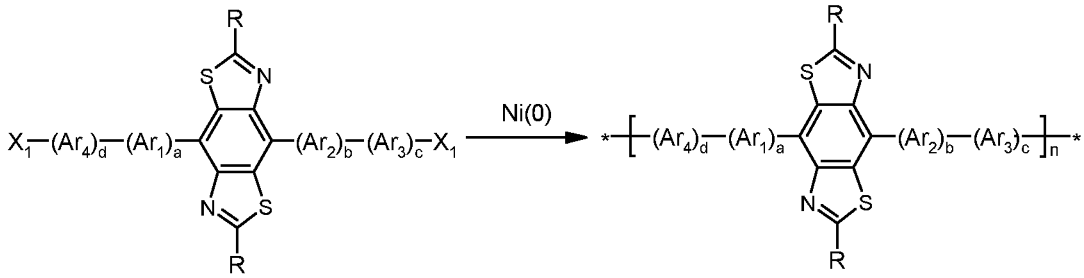


The synthesis of statistical block co-polymers of the 4,8-benzo[1,2-d;4,5-d']bisthiazole core is shown in Scheme 6, wherein the individual radicals are as defined in Scheme 5.

<u>Scheme 6</u>

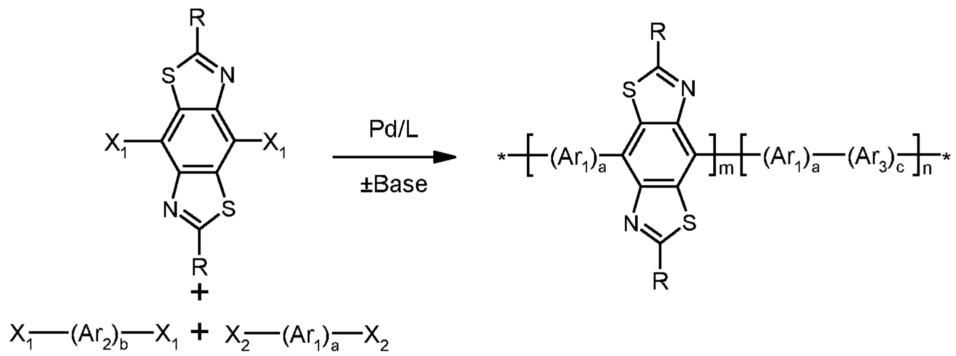


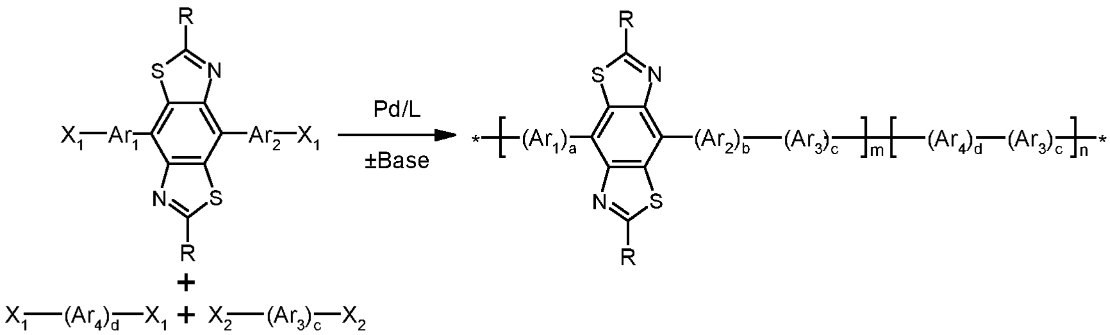


The synthesis of oliogomers and small molecules based on the 4,8-benzo[1,2-d;4,5-d']bisthiazole core is shown in Scheme 7. Alternatively these compounds can be obtained via a convergent synthesis strategy as shown in Scheme 8. Therein X1 = Br and X2 = $SnR_3$ or $B(OR)_2$ or X1 = $SnR_3$ and X2 = Br or X1 = $B(OR)_2$ and X2 = Br, $Ar_{1-8}$ correspond to $Ar^{1-8}$ as defined in formula VI, and $Ar_5$-$Ar_6$-$Ar_7$-$Ar_8$-$R^2_{end}$ is identical to $Ar_4$-$Ar_3$-$Ar_2$-$Ar_1$-$R^1_{end}$, and $R^1_{end}$ and $R^2_{end}$ correspond to $R^{1t}$ and $R^{2t}$ in formula VI.

Scheme 7

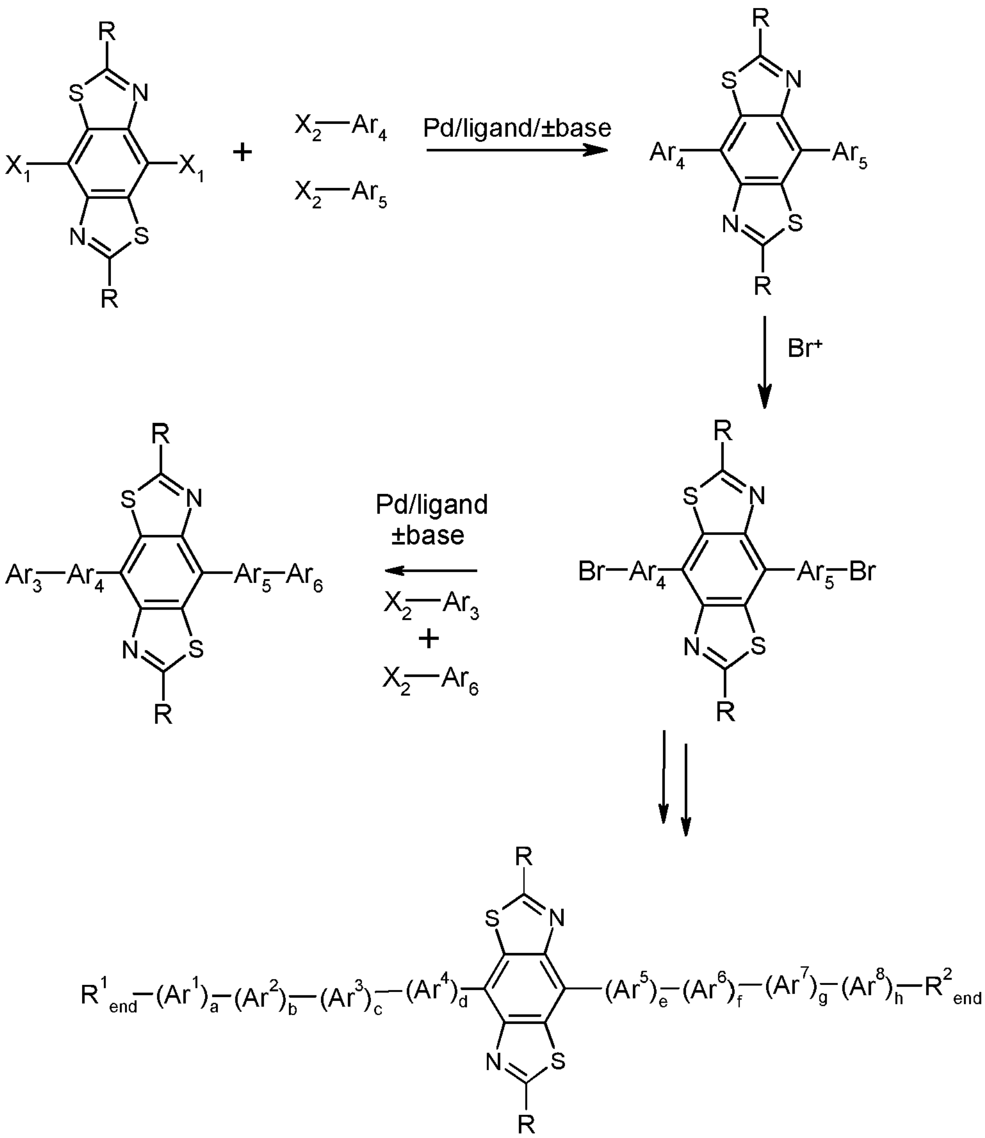


Scheme 8

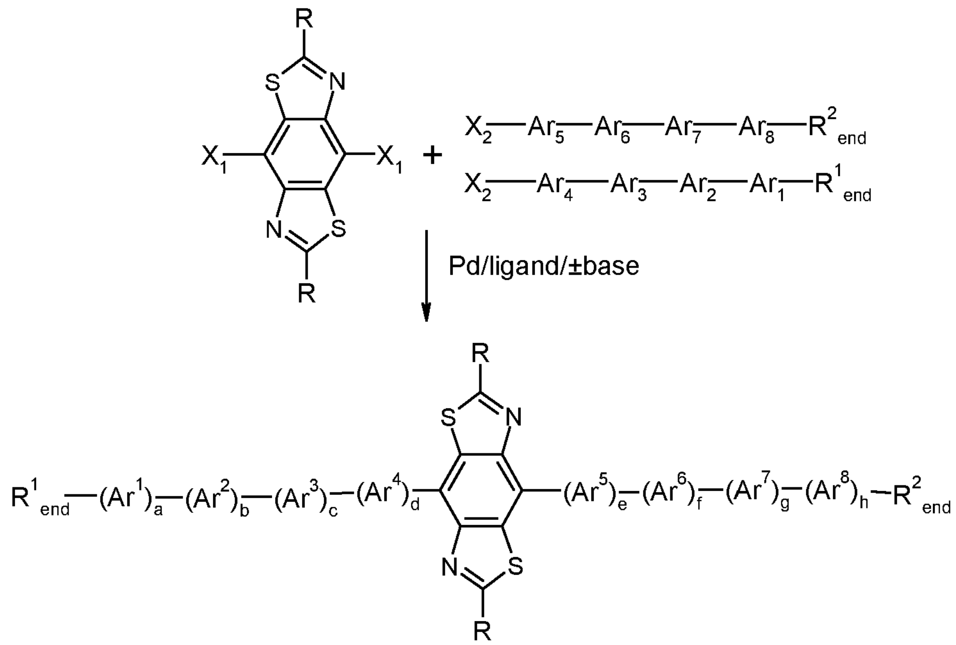


Alternatively the asymmetric small molecules based on 4,8-benzo[1,2-d;4,5-d']bisthiazole obtained via a convergent synthesis strategy as shown in Scheme 9, wherein the individual radicals are as defined in Scheme 7.

Scheme 9

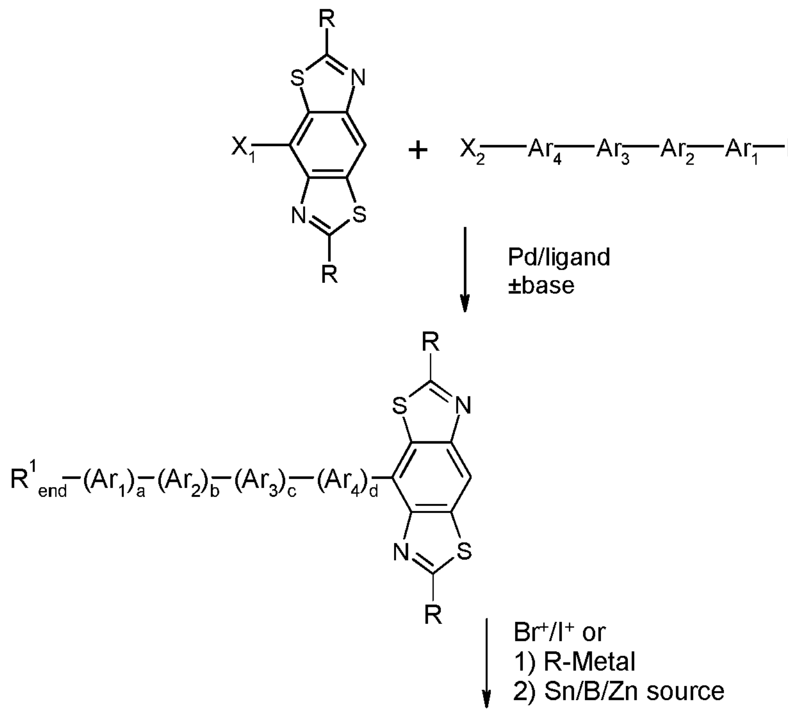

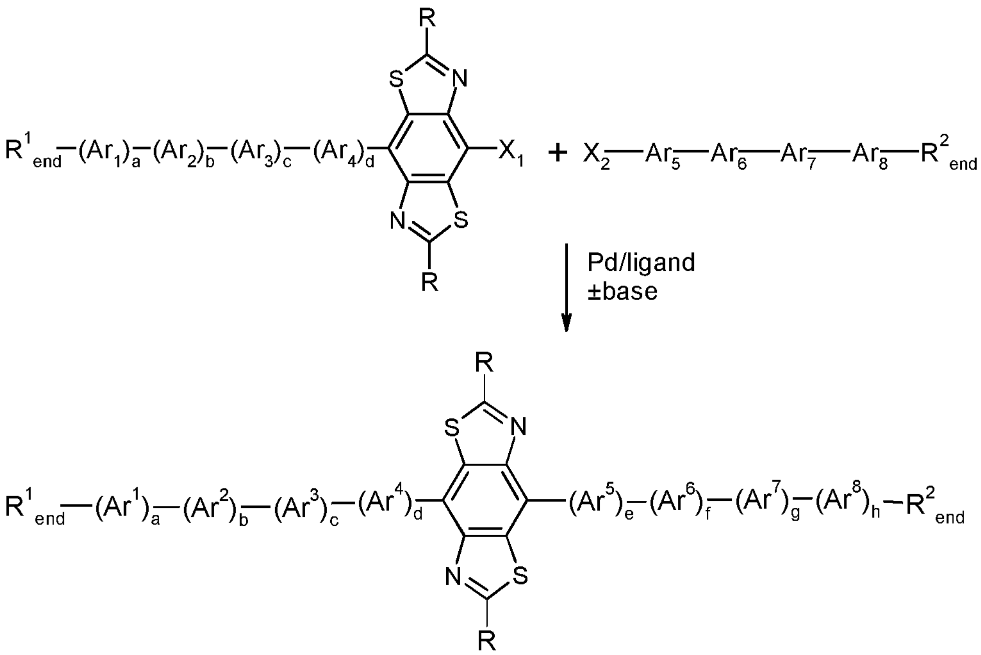


The synthesis of asymmetric compounds containing multiple 4,8-benzo[1,2-d;4,5-d']bisthiazole units via a convergent synthesis strategy is shown in Scheme 10, wherein the individual radicals are as defined in Scheme 7, and $1<n\leq3$.

Scheme 10

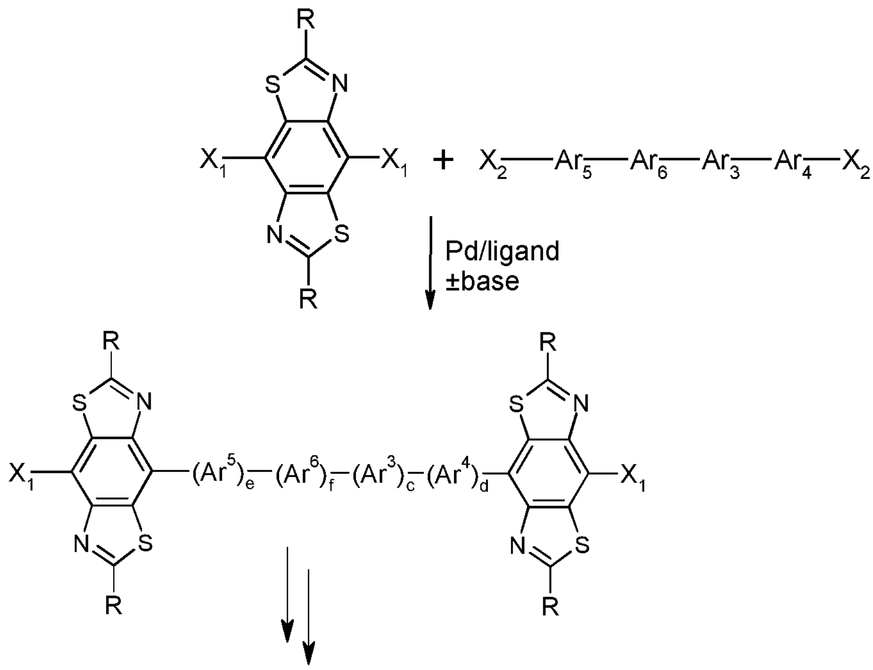

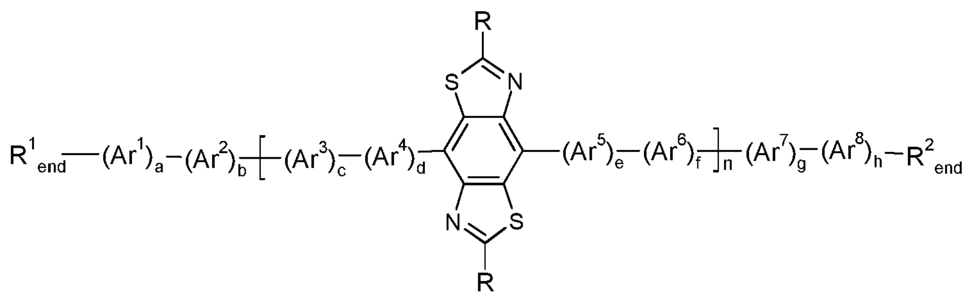

Further substitution can be added to the 4,8-benzo[1,2-d;4,5-d']bisthiazole core at the $R^x_{end}$ substitution after the 4,8-benzo[1,2-d;4,5-d']bisthiazole core organic semiconductors have been prepared as shown in Scheme 11, wherein the individual radicals are as defined in Scheme 10.

Scheme 11

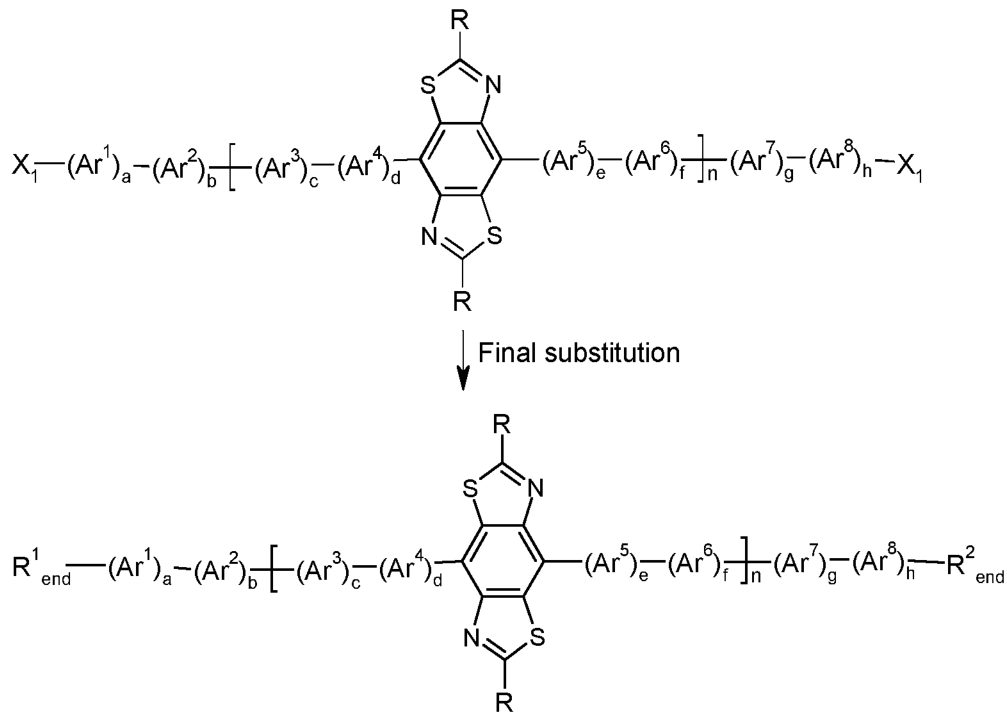


The novel methods of preparing a compound, monomer or polymer as described above and below, and the novel monomers and intermediates used therein, are further aspects of the invention.

The polymer according to the present invention can also be used in mixtures or polymer blends, for example together with monomeric compounds or together with other polymers having charge-transport, semiconducting, electrically conducting, photoconducting and/or light-emitting semiconducting properties, or for example with polymers having hole blocking, electron blocking properties for use as interlayers, charge blocking layers, charge transporting layer in OLED devices, OPV devices or perovskite based solar cells. Thus, another aspect of the invention relates to a polymer blend comprising one or more polymers according to the present invention and one or more further polymers having one or more of the above-mentioned properties. These blends can be prepared by conventional methods that are described in prior art and known to the skilled person. Typically the polymers are mixed with each other or dissolved in suitable solvents and the solutions combined.

Another aspect of the invention relates to a formulation comprising one or more polymers, polymer blends or mixtures as described above and below and one or more organic solvents.

Preferred solvents are aliphatic hydrocarbons, chlorinated hydrocarbons, aromatic hydrocarbons, ketones, ethers and mixtures thereof. Additional solvents which can be used include 1,2,4-trimethylbenzene, 1,2,3,4-tetramethyl benzene, pentylbenzene, mesitylene, cumene, cymene, cyclohexylbenzene, diethylbenzene, tetralin, decalin, 2,6-lutidine, 2-fluoro-m-xylene, 3-fluoro-o-xylene, 2-chlorobenzotrifluoride, N,N-dimethylformamide, 2-chloro-6-fluorotoluene, 2-fluoroanisole, anisole, 2,3-dimethylpyrazine, 4-fluoroanisole, 3-fluoroanisole, 3-trifluoro-methylanisole, 2-methylanisole, phenetol, 4-methylanisole, 3-methylanisole, 4-fluoro-3-methylanisole, 2-fluorobenzonitrile, 4-fluoroveratrol, 2,6-dimethylanisole, 3-fluorobenzo-nitrile, 2,5-dimethylanisole, 2,4-dimethylanisole, benzonitrile, 3,5-dimethyl-anisole, N,N-dimethylaniline, ethyl benzoate, 1-fluoro-3,5-dimethoxy-benzene, 1-methylnaphthalene, N-methylpyrrolidinone, 3-fluorobenzo-trifluoride, benzotrifluoride, dioxane, trifluoromethoxy-benzene, 4-fluorobenzotrifluoride, 3-fluoropyridine, toluene, 2-fluoro-toluene, 2-fluorobenzotrifluoride, 3-fluorotoluene, 4-isopropylbiphenyl, phenyl ether, pyridine, 4-fluorotoluene, 2,5-difluorotoluene, 1-chloro-2,4-difluorobenzene, 2-fluoropyridine, 3-chlorofluoro-benzene, 1-chloro-2,5-difluorobenzene, 4-chlorofluorobenzene, chloro-benzene, o-dichlorobenzene, 2-chlorofluorobenzene, p-xylene, m-xylene, o-xylene or mixture of o-, m-, and p-isomers. Solvents with relatively low polarity are generally preferred. For inkjet printing solvents and solvent mixtures with high boiling temperatures are preferred. For spin coating alkylated benzenes like xylene and toluene are preferred.

Examples of especially preferred solvents include, without limitation, dichloromethane, trichloromethane, tetrachloromethane, chlorobenzene, o-dichlorobenzene, 1,2,4-trichlorobenzene, 1,2-dichloroethane, 1,1,1-trichloroethane, 1,1,2,2-tetrachloroethane, 1,8-diiodooctane, 1-chloronaphthalene, 1,8-octane-dithiol, anisole, 2,5-di-methylanisole, 2,4-dimethylanisole, toluene, o-xylene, m-xylene, p-xylene, mixture of o-, m-, and p-xylene isomers, 1,2,4-trimethylbenzene, mesitylene, cyclohexane, 1-methylnaphthalene, 2-methylnaphthalene, 1,2-dimethylnaphthalene, tetraline, decaline, indane, 1-methyl-4-(1-methylethenyl)-cyclohexene (d-Limonene), 6,6-dimethyl-2-methylenebicyclo[3.1.1]heptanes ($\beta$-pinene), methyl benzoate, ethyl benzoate, nitrobenzene, benzaldehyde, tetrahydrofuran, 1,4-dioxane, 1,3-dioxane, morpholine, acetone, methylethylketone, ethyl acetate, n-butyl acetate, N,N-dimethylformamide, dimethylacetamide, dimethylsulfoxide and/or mixtures thereof.

The concentration of the polymers in the solution is preferably 0.1 to 10% by weight, more preferably 0.5 to 5% by weight. Optionally, the solution also comprises one or more binders to adjust the rheological properties, as described for example in WO 2005/055248 A1.

After the appropriate mixing and ageing, solutions are evaluated as one of the following categories: complete solution, borderline solution or insoluble. The contour line is drawn to outline the solubility parameter-hydrogen bonding limits dividing solubility and insolubility. 'Complete' solvents falling within the solubility area can be chosen from literature values such as published in "Crowley, J.D., Teague, G.S. Jr and Lowe, J.W. Jr., Journal of Paint Technology, 1966, 38 (496), 296 ". Solvent blends may also be used and can be identified as described in "Solvents, W.H.Ellis, Federation of Societies for Coatings Technology, p9-10, 1986".

Such a procedure may lead to a blend of 'non' solvents that will dissolve both the polymers of the present invention, although it is desirable to have at least one true solvent in a blend.

The polymer according to the present invention can also be used in patterned OSC layers in the devices as described above and below. For applications in modern microelectronics it is generally desirable to generate small structures or patterns to reduce cost (more devices/unit area), and power consumption. Patterning of thin layers comprising a polymer according to the present invention can be carried out for example by photolithography, electron beam lithography or laser patterning.

For use as thin layers in electronic or electrooptical devices the polymers, polymer blends or formulations of the present invention may be deposited by any suitable method. Liquid coating of devices is more desirable than vacuum deposition techniques. Solution deposition methods are especially preferred. The formulations of the present invention enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, curtain coating, brush coating, slot dye coating or pad printing.

Ink jet printing is particularly preferred when high resolution layers and devices needs to be prepared. Selected formulations of the present invention may be applied to prefabricated device substrates by ink jet printing or microdispensing. Preferably industrial piezoelectric print heads such as but not limited to those supplied by Aprion, Hitachi-Koki, InkJet Technology, On Target Technology, Picojet, Spectra, Trident, Xaar may be used to apply the organic semiconductor layer to a substrate. Additionally semi-industrial heads such as those manufactured by Brother, Epson, Konica, Seiko Instruments Toshiba TEC or single nozzle microdispensers such as those produced by Microdrop and Microfab may be used.

In order to be applied by ink jet printing or microdispensing, the polymers should be first dissolved in a suitable solvent. Solvents must fulfil the requirements stated above and must not have any detrimental effect on the chosen print head. Additionally, solvents should have boiling points >100°C, preferably >140°C and more preferably >150°C in order to prevent operability problems caused by the solution drying out inside the print head. Apart from the solvents mentioned above, suitable solvents include substituted and non-substituted xylene derivatives, di-$C_{1-2}$-alkyl formamide, substituted and non-substituted anisoles and other phenol-ether derivatives, substituted heterocycles such as substituted pyridines, pyrazines, pyrimidines, pyrrolidinones, substituted and non-substituted *N*,*N*-di-$C_{1-2}$-alkylanilines and other fluorinated

or chlorinated aromatics.

A preferred solvent for depositing a polymer according to the present invention by ink jet printing comprises a benzene derivative which has a benzene ring substituted by one or more substituents wherein the total number of carbon atoms among the one or more substituents is at least three. For example, the benzene derivative may be substituted with a propyl group or three methyl groups, in either case there being at least three carbon atoms in total. Such a solvent enables an ink jet fluid to be formed comprising the solvent with the compound or polymer, which reduces or prevents clogging of the jets and separation of the components during spraying. The solvent(s) may include those selected from the following list of examples: dodecylbenzene, 1-methyl-4-tert-butylbenzene, terpineol, limonene, isodurene, terpinolene, cymene, diethylbenzene. The solvent may be a solvent mixture, that is a combination of two or more solvents, each solvent preferably having a boiling point >100°C, more preferably >140°C. Such solvent(s) also enhance film formation in the layer deposited and reduce defects in the layer.

The ink jet fluid (that is mixture of solvent, binder and semiconducting compound) preferably has a viscosity at 20°C of 1-100 mPas, more preferably 1-50 mPas and most preferably 1-30 mPas.

The polymers, polymer blends, mixtures and formulations according to the present invention can additionally comprise one or more further components or additives selected for example from surface-active compounds, lubricating agents, wetting agents, dispersing agents, hydrophobing agents, adhesive agents, flow improvers, defoaming agents, deaerators, diluents which may be reactive or non-reactive, auxiliaries, colourants, dyes or pigments, sensitizers, stabilizers, nanoparticles or inhibitors.

The polymers, polymer blends and mixtures according to the present invention are useful as charge transport, semiconducting, electrically conducting, photoconducting or light emitting material in optical, electrooptical, electronic, electroluminescent or photoluminescent components or devices. In these devices, a polymer, polymer blend or mixture of the present invention is typically applied as a thin layer or film.

Thus, the present invention also provides the use of the polymer, polymer blend, mixture or layer in an electronic device. The formulation may be used as a high mobility semiconducting material in various devices and apparatus. The formulation may be used, for example, in the form of a semiconducting layer or film. Accordingly, in another aspect, the present invention provides a semiconducting layer for use in an electronic device, the layer comprising a polymer, mixture or polymer blend according to the invention. The layer or film may be less than about 30 microns. For various electronic device applications, the thickness may be less than about 1 micron thick. The layer may be deposited, for example on a part of an electronic device, by any of the aforementioned solution coating or printing techniques.

The invention additionally provides an electronic device comprising a polymer, polymer blend, mixture or organic semiconducting layer according to the present invention. Especially preferred devices are OFETs, TFTs, ICs, logic circuits, capacitors, RFID tags, OLEDs, OLETs, OPEDs, OPVs, OPDs, solar cells, laser diodes, photoconductors, photodetectors, electrophotographic devices, electrophotographic recording devices, organic memory devices, sensor devices, charge injection layers, Schottky diodes, planarising layers, antistatic films, conducting substrates and conducting patterns.

Especially preferred electronic device are OFETs, OLEDs, OPV and OPD devices, in particular bulk heterojunction (BHJ) OPV devices. In an OFET, for example, the active semiconductor channel between the drain and source may comprise the layer of the invention. As another example, in an OLED device, the charge (hole or electron) injection or transport layer may comprise the layer of the invention.

For use in OPV or OPD devices the polymer according to the present invention is preferably used in a formulation that comprises or contains, more preferably consists essentially of, very preferably exclusively of, one or more p-type (electron donor) semiconductor and one or more n-type (electron acceptor) semiconductor. The p-type semiconductor is constituted of a least one polymer according to the present invention. The n-type semiconductor can be an inorganic material such as zinc oxide ($ZnO_x$), zinc tin oxide (ZTO), titanium oxide ($TiO_x$), molybdenum oxide ($MoO_x$), nickel oxide ($NiO_x$), or cadmium selenide (CdSe), or an organic material such as graphene or a fullerene, a conjugated polymer or substituted fullerene, for example a (6,6)-phenyl-butyric acid methyl ester derivatized methano $C_{60}$ fullerene, also known as "PCBM-$C_{60}$" or "$C_{60}$PCBM", as disclosed for example in Science 1995, 270, 1789 and having the structure shown below, or structural analogous compounds with e.g. a $C_{70}$ fullerene group or an organic polymer (see for example Coakley, K. M. and McGehee, M. D. Chem. Mater. 2004, 16, 4533).

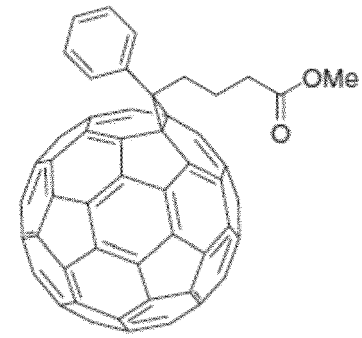

PCBM-$C_{60}$

Preferably the polymer according to the present invention is blended with an n-type semiconductor such as a fullerene or substituted fullerene of formula XII to form the active layer in an OPV or OPD device wherein,

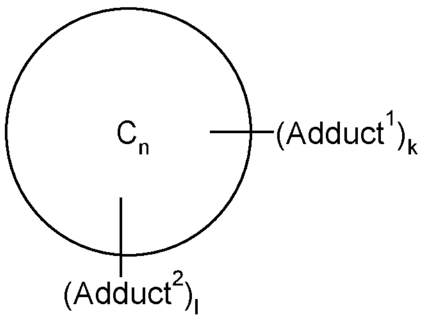

XII

$C_n$ denotes a fullerene composed of n carbon atoms, optionally with one or more atoms trapped inside,

Adduct$^1$ is a primary adduct appended to the fullerene $C_n$ with any connectivity,

Adduct2 is a secondary adduct, or a combination of secondary adducts, appended to the fullerene $C_n$ with any connectivity,

k is an integer $\geq 1$,

and

l is 0, an integer $\geq 1$, or a non-integer > 0.

In the formula XII and its subformulae, k preferably denotes 1, 2, 3 or, 4, very preferably 1 or 2.

The fullerene $C_n$ in formula XII and its subformulae may be composed of any number n of carbon atoms Preferably, in the compounds of formula XII and its subformulae the number of carbon atoms n of which the fullerene $C_n$ is composed is 60, 70, 76, 78, 82, 84, 90, 94 or 96, very preferably 60 or 70.

The fullerene $C_n$ in formula XII and its subformulae is preferably selected from carbon based fullerenes, endohedral fullerenes, or mixtures thereof, very preferably from carbon based fullerenes.

Suitable and preferred carbon based fullerenes include, without limitation, ($C_{60-Ih}$)[5,6]fullerene, ($C_{70-D5h}$)[5,6]fullerene, ($C_{76-D2*}$)[5,6]fullerene, ($C_{84-D2*}$)[5,6]fullerene, ($C_{84-D2d}$)[5,6]fullerene, or a mixture of two or more of the aforementioned carbon based fullerenes.

The endohedral fullerenes are preferably metallofullerenes. Suitable and preferred metallofullerenes include, without limitation, La@$C_{60}$, La@$C_{82}$, Y@$C_{82}$, $S_{C3}N$@$C_{80}$, $Y_3N$@$C_{80}$, $Sc_3C_2$@$C_{80}$ or a mixture of two or more of the aforementioned metallofullerenes.

Preferably the fullerene $C_n$ is substituted at a [6,6] and/or [5,6] bond, preferably substituted on at least one [6,6] bond.

Primary and secondary adduct, named "Adduct" in formula XII and its subformulae, is preferably selected from the following formulae

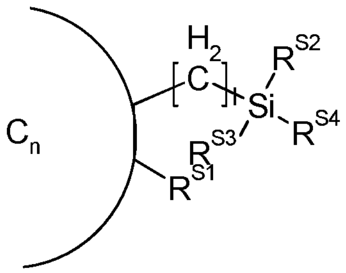

S-1

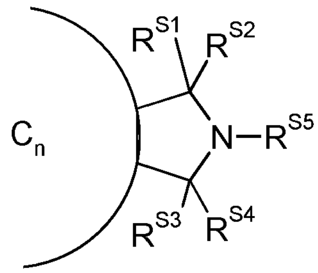

S-2

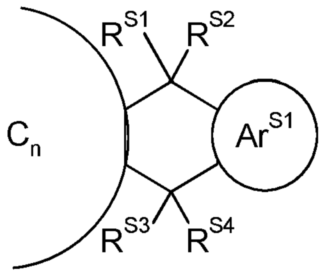

S-3

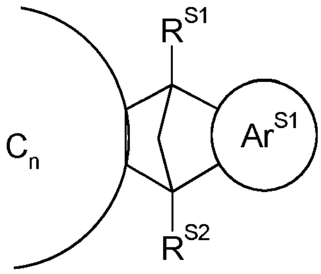

S-4

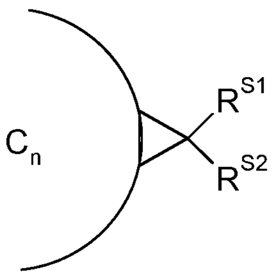

S-5

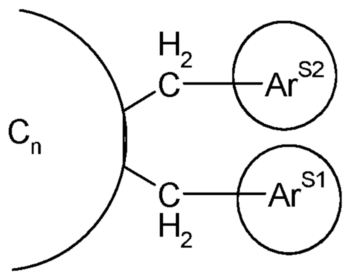

S-6

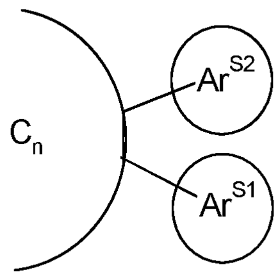

S-7

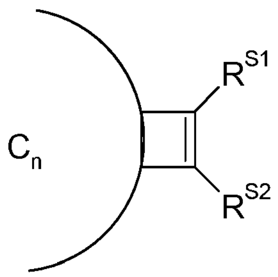

S-8

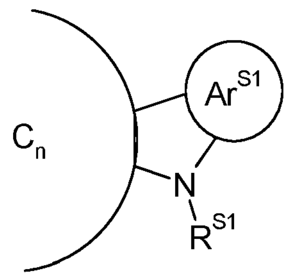

S-9

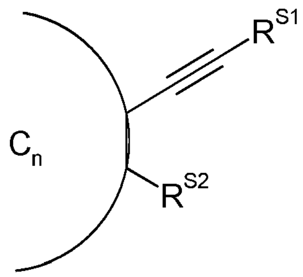

S-10

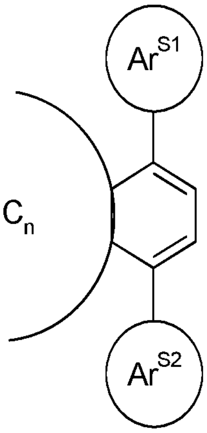

S-11

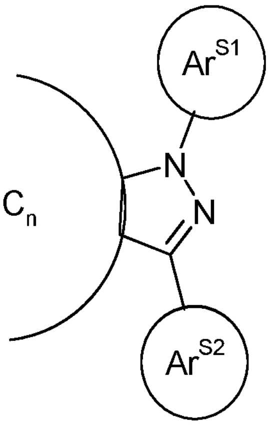

S-12

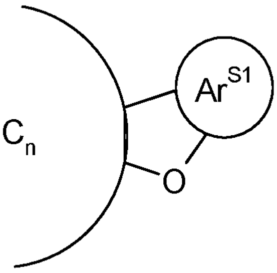

S-13

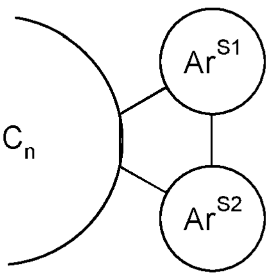

S-14

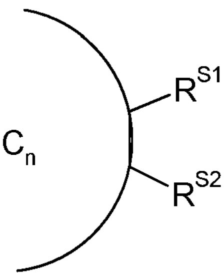

S-15

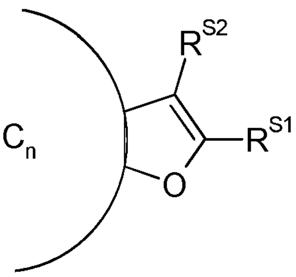

S-16

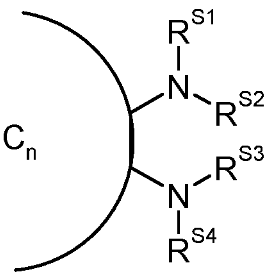

S-17

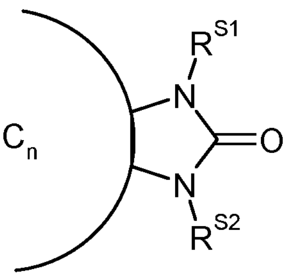

S-18

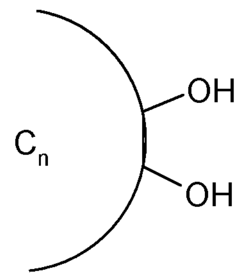

S-19

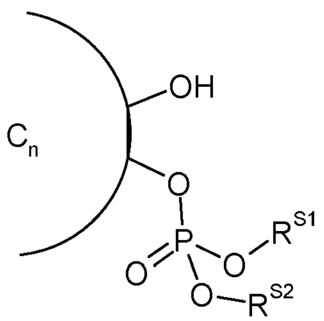

S-20

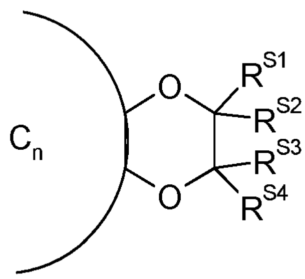

S-21

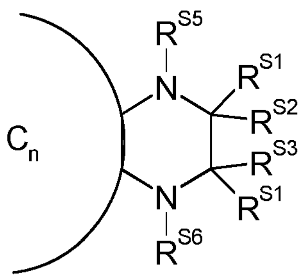

S-22

wherein $C_n$ is as defined in formula XII,

$Ar^{S1}$, $Ar^{S2}$ denote, independently of each other, an arylene or heteroarylene group with 5 to 20, preferably 5 to 15,

ring atoms, which is mono- or polycyclic, and which is optionally substituted by one or more identical or different substituents having one of the meanings of L as defined above and below, and

$R^{S1}$, $R^{S2}$, $R^{S3}$, $R^{S4}$, $R^{S5}$ and $R^{S6}$ independently of each other denote H, CN or have one of the meanings of L as defined above and below.

Preferred compounds of formula XII are selected from the following subformulae:

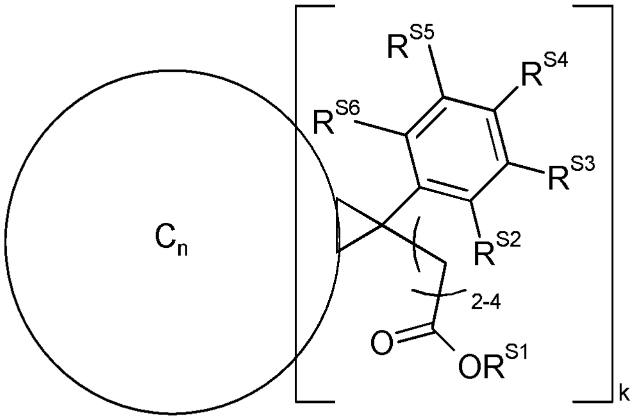

XIIa

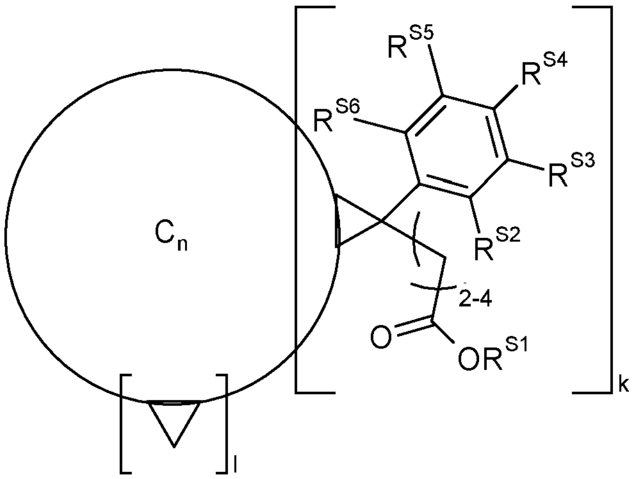

XIIb

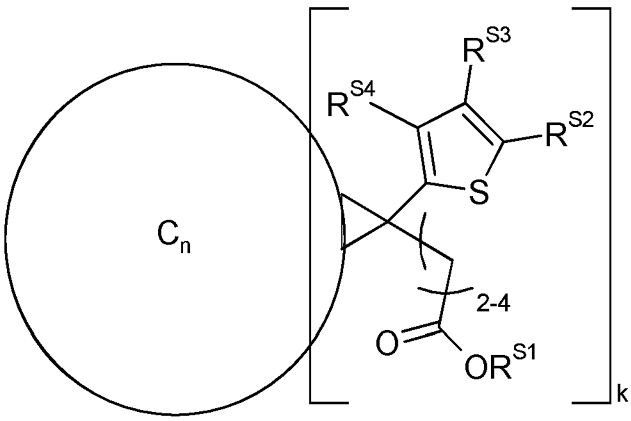

XIIc

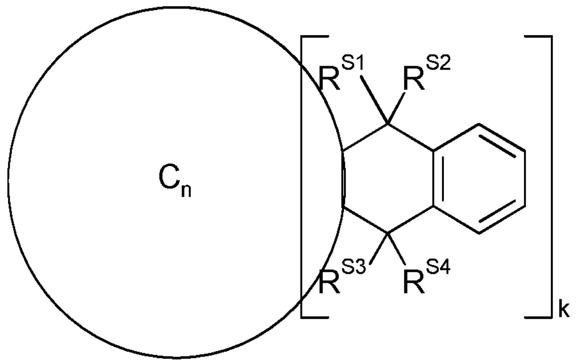

XIId

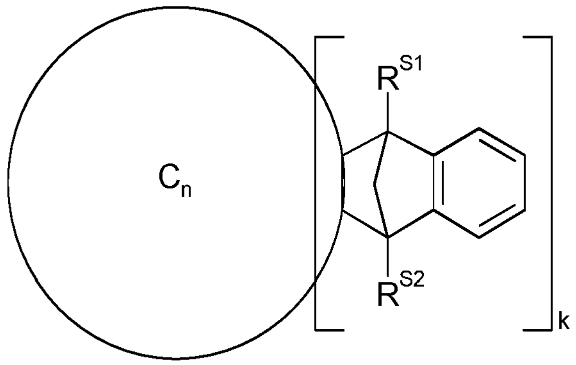

XIIe

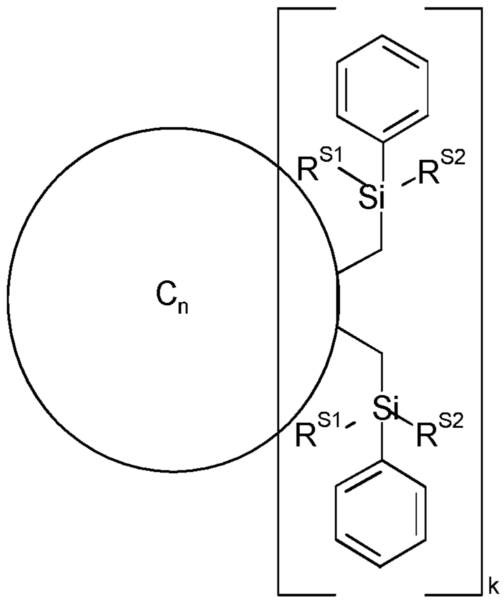

XIIf

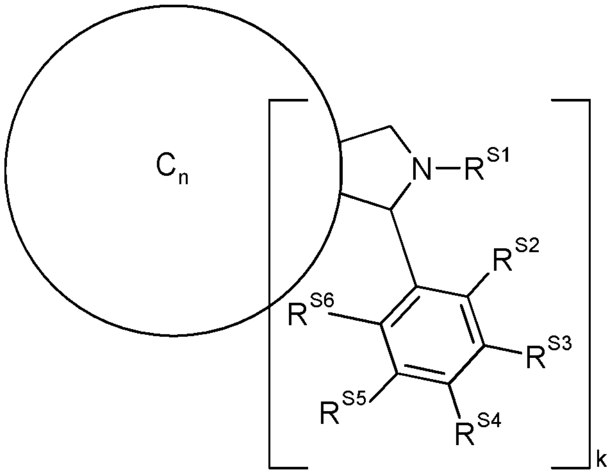

XIIg

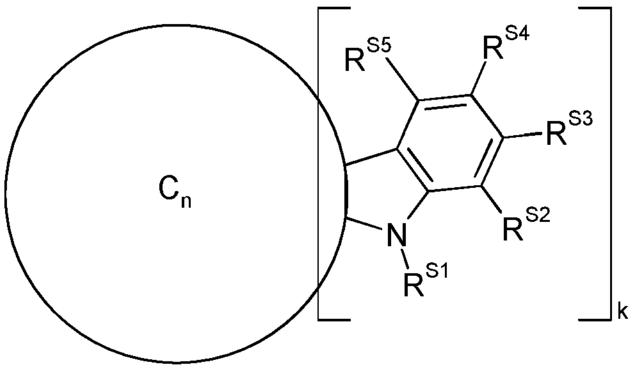

XIIh

wherein $C_n$, k and I are as defined in formula XII, and

$R^{S1}$, $R^{S2}$, $R^{S3}$, $R^{S4}$ $R^{S5}$ and $R^{S6}$ independently of each other denote H or have one of the meanings of L as defined above and below.

Also preferably the polymer according to the present invention is blended with other type of n-type semiconductor such as graphene, a metal oxide, like for example, ZnOx, TiOx, ZTO, MoOx, NiOx, quantum dots, like for example, CdSe or CdS, or a conjugated polymer, like for example a polynaphthalenediimide or polyperylenediimide as described, for example, in WO2013142841 A1 to form the active layer in an OPV or OPD device.

The device preferably further comprises a first transparent or semi-transparent electrode on a transparent or semi-transparent substrate on one side of the active layer, and a second metallic or semi-transparent electrode on the other side of the active layer.

Preferably, the active layer according to the present invention is further blended with additional organic and inorganic compounds to enhance the device properties. For example, metal particles such as Au or Ag nanoparticules or Au or Ag nanoprism for enhancements in light harvesting due to near-field effects (i.e. plasmonic effect) as described, for example in Adv. Mater. 2013, 25 (17), 2385-2396 and Adv. Ener. Mater. 10.1002/aenm.201400206, a molecular dopant such as 2,3,5,6-tetrafluoro-7,7,8,8-tetracyanoquinodimethane for enhancement in photoconductivity as described, for example in Adv. Mater. 2013, 25(48), 7038-7044, or a stabilising agent consisting of a UV absorption agent and/or anti-radical agent and/or antioxidant agent such as 2-hydroxybenzophenone, 2-hydroxyphenylbenzotriazole, oxalic acid anilides, hydroxyphenyl triazines, merocyanines, hindered phenol, N-aryl-thiomorpholine, N-aryl-thiomorpholine-1-oxide, N-aryl-thiomorpholine-1,1-dioxide, N-aryl-thiazolidine, N-aryl-thiazolidine-1-oxide, N-aryl-thiazolidine-1,1-dioxide and 1,4-diazabicyclo[2.2.2]octane as described, for example, in WO2012095796 A1 and in WO2013021971 A1.

The device preferably may further comprise a UV to visible photo-conversion layer such as described, for example, in J. Mater. Chem. 2011, 21, 12331 or a NIR to visible or IR to NIR photo-conversion layer such as described, for example, in J. Appl. Phys. 2013, 113, 124509.

Further preferably the OPV or OPD device comprises, between the active layer and the first or second electrode, one or more additional buffer layers acting as hole transporting layer and/or electron blocking layer, which comprise a material such as metal oxides, like for example, ZTO, $MoO_x$, $NiO_x$, a doped conjugated polymer, like for example PEDOT:PSS and polypyrrole-polystyrene sulfonate (PPy:PSS), a conjugated polymer, like for example polytriarylamine (PTAA), an organic compound, like for example substituted triaryl amine derivatives such as N,N'-diphenyl-N,N'-bis(1-naphthyl)(1,1'-biphenyl)-4,4'diamine (NPB), N,N'-diphenyl-N,N'-(3-methylphenyl)-1,1'-biphenyl-4,4'-diamine (TPD), graphene based materials, like for example, graphene oxide and graphene quantum dots or alternatively as hole blocking layer and/or electron transporting layer, which comprise a material such as metal oxide, like for example, $ZnO_x$, $TiO_x$, AZO (aluminium doped zinc oxide), a salt, like for example LiF, NaF, CsF, a conjugated polymer electrolyte, like for example poly[3-(6-trimethylammoniumhexyl)thiophene], poly(9,9-bis(2-ethylhexyl)-fluorene]-b-poly[3-(6-trimethylammoniumhexyl)thiophene], or poly[(9,9-bis(3'-(N,N-dimethylamino)propyl)-2,7-fluorene)-alt-2,7-(9,9-dioctylfluorene)], a polymer, like for example poly(ethyleneimine) or crosslinked N-containing compound derivatives or an organic compound, like for example tris(8-quinolinolato)-aluminium(III) ($Alq_3$), phenanthroline derivative or $C_{60}$ or $C_{70}$ based fullerenes, like for example, as described in Adv. Energy Mater. 2012, 2, 82-86.

In a blend or mixture of a polymer according to the present invention with a fullerene or modified fullerene, the ratio polymer:fullerene is preferably from 5:1 to 1:5 by weight, more preferably from 2:1 to 1:3 by weight, most preferably 1:1 to 1:2 by weight. A polymeric binder may also be included, from 5 to 95% by weight. Examples of binder include polystyrene (PS), polypropylene (PP) and polymethylmethacrylate (PMMA).

To produce thin layers in BHJ OPV devices the polymers, polymer blends or mixtures of the present invention may be deposited by any suitable method. Liquid coating of devices is more desirable than vacuum deposition techniques. Solution deposition methods are especially preferred. The formulations of the present invention enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, curtain coating, brush coating, slot dye coating or pad printing. For the fabrication of OPV devices and modules area printing method compatible with flexible substrates are preferred, for example slot dye coating, spray coating and the like.

Suitable solutions or formulations containing a blend or mixture of a polymer according to the present invention with a fullerene or modified fullerene like PCBM are preferably prepared. In the preparation of such a formulation, suitable solvents are preferably selected to ensure full dissolution of both component, p-type and n-type and take into account the boundary conditions (for example rheological properties) introduced by the chosen printing method.

Organic solvent are generally used for this purpose. Typical solvents can be aromatic solvents, halogenated solvents or chlorinated solvents, including chlorinated aromatic solvents. Examples include, but are not limited to dichloromethane, trichloromethane, tetrachloromethane, chlorobenzene, o-dichlorobenzene, 1,2,4-trichlorobenzene, 1,2-dichloroethane, 1,1,1-trichloroethane, 1,1,2,2-tetrachloroethane, 1,8-diiodooctane, 1-chloronaphthalene, 1,8-octanedithiol, anisole, 2,5-dimethylanisole, 2,4-dimethylanisole, toluene, o-xylene, m-xylene, p-xylene, mixture of xylene o-, m-, and p-isomers, 1,2,4-trimethylbenzene, mesitylene, cyclohexane, 1-methylnaphthalene, 2-methylnaphthalene, 1,2-dimethylnaphthalene, tetraline, decaline, indane, 1-methyl-4-(1-methylethenyl)-cyclohexene (d-Limonene), 6,6-dimethyl-2-methylenebicyclo[3.1.1]heptanes ($\beta$ -pinene), methyl benzoate, ethyl benzoate, nitrobenzene, benzaldehyde, tetrahydrofuran, 1,4-dioxane, 1,3-dioxane, morpholine, acetone, methylethylketone, ethyl acetate, n-butyl acetate, N,N-dimethylformamide, dimethylacetamide, dimethylsulfoxide and/or mixtures thereof.

The OPV device can for example be of any type known from the literature (see e.g. Waldauf et al., Appl. Phys. Lett., 2006, 89, 233517).

A first preferred OPV device according to the invention comprises the following layers (in the sequence from bottom to top):

- optionally a substrate,
- a high work function electrode, preferably comprising a metal oxide, like for example ITO and FTO, serving as anode,
- an optional conducting polymer layer or hole transport layer, preferably comprising an organic polymer or polymer blend, for example PEDOT:PSS (poly(3,4-ethylenedioxythiophene): poly(styrene-sulfonate), substituted triaryl amine derivatives, for example,TBD (N,N'-dyphenyl-N-N'-bis(3-methylphenyl)-1,1'biphenyl-4,4'-diamine) or NBD (N,N'-dyphenyl-N-N'-bis(1-napthylphenyl)-1,1'biphenyl-4,4'-diamine),
- a layer, also referred to as "active layer", comprising of at least one p-type and at least one n-type organic semiconductor, which can exist for example as a p-type/n-type bilayer or as distinct p-type and n-type layers, or as blend or p-type and n-type semiconductor, forming a BHJ,
- optionally a layer having electron transport properties, for example comprising LiF, $TiO_x$, $ZnO_x$, PFN, a poly(ethyleneimine) or crosslinked nitrogen containing compound derivatives or a phenanthroline derivatives
- a low work function electrode, preferably comprising a metal like for example aluminum, serving as cathode,

wherein at least one of the electrodes, preferably the anode, is transparent to visible and/or NIR light, and
wherein at least one p-type semiconductor is a polymer according to the present invention.

A second preferred OPV device according to the invention is an inverted OPV device and comprises the following layers (in the sequence from bottom to top):

- optionally a substrate,
- a high work function metal or metal oxide electrode, comprising for example ITO and FTO, serving as cathode,
- a layer having hole blocking properties, preferably comprising a metal oxide like $TiO_x$ or $ZnO_x$, or comprising an organic compound such as polymer like poly(ethyleneimine) or crosslinked nitrogen containing compound derivatives or phenanthroline derivatives,
- an active layer comprising at least one p-type and at least one n-type organic semiconductor, situated between the electrodes, which can exist for example as a p-type/n-type bilayer or as distinct p-type and n-type layers, or as blend or p-type and n-type semiconductor, forming a BHJ,
- an optional conducting polymer layer or hole transport layer, preferably comprising an organic polymer or polymer blend, for example of PEDOT:PSS or substituted triaryl amine derivatives, for example, TBD or NBD,
- an electrode comprising a high work function metal like for example silver, serving as anode,

wherein at least one of the electrodes, preferably the cathode, is transparent to visible and/or NIR light, and wherein at least one p-type semiconductor is a polymer according to the present invention.

In the OPV devices of the present invention the p-type and n-type semiconductor materials are preferably selected from the materials, like the polymer/fullerene systems or polymer/polymer systems, as described above

When the active layer is deposited on the substrate, it forms a BHJ that phase separates at nanoscale level. For discussion on nanoscale phase separation see Dennler et al, Proceedings of the IEEE, 2005, 93 (8), 1429 or Hoppe et al, Adv. Func. Mater, 2004, 14(10), 1005. An optional annealing step may be then necessary to optimize blend morpohology and consequently OPV device performance.

Another method to optimize device performance is to prepare formulations for the fabrication of OPV(BHJ) devices that may include high boiling point additives to promote phase separation in the right way. 1,8-Octanedithiol, 1,8-diiodooctane, nitrobenzene, 1-chloronaphthalene, N,N-dimethylformamide, dimethylacetamide, dimethylsulfoxide and other additives have been used to obtain high-efficiency solar cells. Examples are disclosed in J. Peet, et al, Nat. Mater., 2007, 6, 497 or Fréchet et al. J. Am. Chem. Soc., 2010, 132, 7595-7597.

The polymers, polymer blends, mixtures and layers of the present invention are also suitable for use in an OFET as the semiconducting channel. Accordingly, the invention also provides an OFET comprising a gate electrode, an insulating (or gate insulator) layer, a source electrode, a drain electrode and an organic semiconducting channel connecting the source and drain electrodes, wherein the organic semiconducting channel comprises a polymer, polymer blend, mixture or organic semiconducting layer according to the present invention. Other features of the OFET are well known to those skilled in the art.

OFETs where an OSC material is arranged as a thin film between a gate dielectric and a drain and a source electrode, are generally known, and are described for example in US 5,892,244, US 5,998,804, US 6,723,394 and in the references cited in the background section. Due to the advantages, like low cost production using the solubility properties of the compounds according to the invention and thus the processibility of large surfaces, preferred applications of these FETs are such as integrated circuitry, TFT displays and security applications.

The gate, source and drain electrodes and the insulating and semiconducting layer in the OFET device may be arranged in any sequence, provided that the source and drain electrode are separated from the gate electrode by the insulating layer, the gate electrode and the semiconductor layer both contact the insulating layer, and the source electrode and the drain electrode both contact the semiconducting layer.

An OFET device according to the present invention preferably comprises:

- a source electrode,
- a drain electrode,
- a gate electrode,
- a semiconducting layer,
- one or more gate insulator layers,
- optionally a substrate.

wherein the semiconductor layer preferably comprises a polymer, polymer blend or mixture according to the present invention.

The OFET device can be a top gate device or a bottom gate device. Suitable structures and manufacturing methods of an OFET device are known to the skilled in the art and are described in the literature, for example in US 2007/0102696 A1.

The gate insulator layer preferably comprises a fluoropolymer, like e.g. the commercially available Cytop 809M® or Cytop 107M® (from Asahi Glass). Preferably the gate insulator layer is deposited, e.g. by spin-coating, doctor blading, wire bar coating, spray or dip coating or other known methods, from a formulation comprising an insulator material and one or more solvents with one or more fluoro atoms (fluorosolvents), preferably a perfluorosolvent. A suitable perfluorosolvent is e.g. FC75® (available from Acros, catalogue number 12380). Other suitable fluoropolymers and fluorosolvents are known in prior art, like for example the perfluoropolymers Teflon AF® 1600 or 2400 (from DuPont) or Fluoropel® (from Cytonix) or the perfluorosolvent FC 43® (Acros, No. 12377). Especially preferred are organic dielectric materials having a low permittivity (or dielectric constant) from 1.0 to 5.0, very preferably from 1.8 to 4.0 ("low k materials"), as disclosed for example in US 2007/0102696 A1 or US 7,095,044.

In security applications, OFETs and other devices with semiconducting materials according to the present invention, like transistors or diodes, can be used for RFID tags or security markings to authenticate and prevent counterfeiting of documents of value like banknotes, credit cards or ID cards, national ID documents, licenses or any product with monetry value, like stamps, tickets, shares, cheques etc.

Alternatively, the polymers, polymer blends and mixtures according to the invention can be used in OLEDs, e.g. as the active display material in a flat panel display applications, or as backlight of a flat panel display like e.g. a liquid crystal display. Common OLEDs are realized using multilayer structures. An emission layer is generally sandwiched between one or more electron-transport and/or hole-transport layers. By applying an electric voltage electrons and holes as charge carriers move towards the emissive layer where their recombination leads to the excitation and hence luminescence of the lumophor units contained in the emission layer.

The polymers, polymer blends and mixtures according to the invention can be employed in one or more of a buffer layer, electron or hole transport layer, electron or hole blocking layer and emissive layer, corresponding to their electrical and/or optical properties. Furthermore their use within the emissive layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see, e.g., Müller et al, Synth. Metals, 2000, 111-112, 31-34, Alcala, J. Appl. Phys., 2000, 88, 7124-7128 and the literature cited therein.

According to another use, the polymers, polymer blends and mixtures according to this invention, especially those showing photoluminescent properties, may be employed as materials of light sources, e.g. in display devices, as described in EP 0 889 350 A1 or by C. Weder et al., Science, 1998, 279, 835-837.

A further aspect of the invention relates to both the oxidised and reduced form of a polymer according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g., $I_2$, $Cl_2$, Br2, ICl, $ICl_3$, IBr and IF), Lewis acids (e.g., $PF_5$, $AsF_5$, SbFs, $BF_3$, $BCl_3$, $SbCl_5$, $BBr_3$ and $SO_3$), protonic acids, organic acids, or amino acids (e.g., HF, HCl, $HNO_3$, $H_2SO_4$, $HClO_4$, $FSO_3H$ and $ClSO_3H$), transition metal compounds (e.g., $FeCl_3$, FeOCl, $Fe(ClO_4)_3$, $Fe(4\text{-}CH_3C_6H_4SO_3)_3$, $TiCl_4$, $ZrCl_4$, $HfCl_4$, NbFs, $NbCl_5$, $TaCl_5$, $MoF_5$, $MoCl_5$, $WF_5$, $WCl_6$, $UF_6$ and $LnCl_3$ (wherein Ln is a lanthanoid), anions (e.g., $Cl^-$, $Br^-$, $I^-$, $I_3^-$, $HSO_4^-$, $SO_4^{2-}$, $NO_3^-$, $ClO_4^-$, $BF_4^-$, $PF_6^-$, $AsF_6^-$, $SbF_6^-$, $FeCl_4^-$, $Fe(CN)_6^{3-}$, and anions of various sulfonic acids, such as aryl-$SO_3^-$). When holes are used as carriers, examples of dopants are cations (e.g., $H^+$, $Li^+$, $Na^+$, $K^+$, $Rb^+$ and $Cs^+$), alkali metals (e.g., Li, Na, K, Rb, and Cs), alkaline-earth metals (e.g., Ca, Sr, and Ba), $O_2$, $XeOF_4$, $(NO_2^+)$ $(SbF_6^-)$, $(NO_2^+)$ $(SbCl_6^-)$, $(NO_2^+)$ $(BF_4^-)$, $AgClO_4$, $H_2IrCl_6$, $La(NO_3)_3 \cdot 6H_2O$, $FSO_2OOSO_2F$, Eu, acetylcholine, $R_4N^+$, (R is an alkyl group), $R_4P^+$ (R is an alkyl group), $R_6As^+$ (R is an alkyl group), and $R_3S^+$ (R is an alkyl group).

The conducting form of a polymer of the present invention can be used as an organic "metal" in applications including, but not limited to, charge injection layers and ITO planarising layers in OLED applications, films for flat panel displays and touch screens, antistatic films, printed conductive substrates, patterns or tracts in electronic applications such as printed circuit boards and condensers.

The polymers, polymer blends and mixtures according to the present invention may also be suitable for use in organic plasmon-emitting diodes (OPEDs), as described for example in Koller et al., Nat. Photonics, 2008, 2, 684.

According to another use, the polymers according to the present invention can be used alone or together with other materials in or as alignment layers in LCD or OLED devices, as described for example in US 2003/0021913. The use of charge transport polymers according to the present invention can increase the electrical conductivity of the alignment layer. When used in an LCD, this increased electrical conductivity can reduce adverse residual dc effects in the switchable LCD cell and suppress image sticking or, for example in ferroelectric LCDs, reduce the residual charge produced by the switching of the spontaneous polarisation charge of the ferroelectric LCs. When used in an OLED

device comprising a light emitting material provided onto the alignment layer, this increased electrical conductivity can enhance the electroluminescence of the light emitting material. The polymers according to the present invention having mesogenic or liquid crystalline properties can form oriented anisotropic films as described above, which are especially useful as alignment layers to induce or enhance alignment in a liquid crystal medium provided onto said anisotropic film. The polymers according to the present invention may also be combined with photoisomerisable compounds and/or chromophores for use in or as photoalignment layers, as described in US 2003/0021913 A1.

According to another use the polymers, polymer blends and mixtures according to the present invention, especially their water-soluble derivatives (for example with polar or ionic side groups) or ionically doped forms, can be employed as chemical sensors or materials for detecting and discriminating DNA sequences. Such uses are described for example in L. Chen, D. W. McBranch, H. Wang, R. Helgeson, F. Wudl and D. G. Whitten, Proc. Natl. Acad. Sci. U.S.A., 1999, 96, 12287; D. Wang, X. Gong, P. S. Heeger, F. Rininsland, G. C. Bazan and A. J. Heeger, Proc. Natl. Acad. Sci. U.S.A., 2002, 99, 49; N. DiCesare, M. R. Pinot, K. S. Schanze and J. R. Lakowicz, Langmuir, 2002, 18, 7785; D. T. McQuade, A. E. Pullen, T. M. Swager, Chem. Rev., 2000, 100, 2537.

Unless the context clearly indicates otherwise, as used herein plural forms of the terms herein are to be construed as including the singular form and vice versa.

Throughout the description and claims of this specification, the words "comprise" and "contain" and variations of the words, for example "comprising" and "comprises", mean "including but not limited to", and are not intended to (and do not) exclude other components.

It will be appreciated that variations to the foregoing embodiments of the invention can be made while still falling within the scope of the invention. Each feature disclosed in this specification, unless stated otherwise, may be replaced by alternative features serving the same, equivalent or similar purpose. Thus, unless stated otherwise, each feature disclosed is one example only of a generic series of equivalent or similar features.

All of the features disclosed in this specification may be combined in any combination, except combinations where at least some of such features and/or steps are mutually exclusive. In particular, the preferred features of the invention are applicable to all aspects of the invention and may be used in any combination. Likewise, features described in non-essential combinations may be used separately (not in combination).

Above and below, unless stated otherwise percentages are percent by weight and temperatures are given in degrees Celsius. Values of the dielectric constant ε ("permittivity") refer to values taken at 20°C and 1,000 Hz.

The invention will now be described in more detail by reference to the following examples, which are illustrative only and do not limit the scope of the invention.

A) Monomer and Polymer Examples

Example M1

Monomer 1 was prepared as follows:

Benzo[1,2-d:4,5-d']bisthiazole-2,6-dicarboxylic acid diethyl ester (1.1)

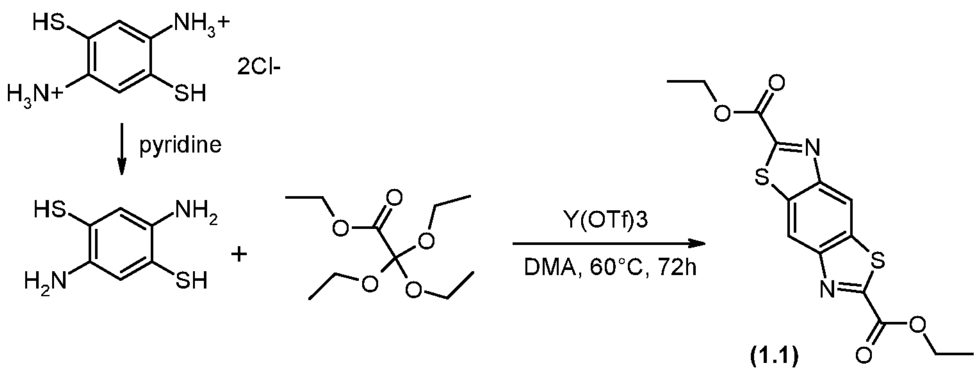


(1.1)

2,5-Diamino-benzene-1,4-dithiol hydrochloride (5.00 g; 20.39 mmol; 1.00 eq.) is suspended in anhydrous N,N-dimethylformamide (37 $cm^3$) at 50 °C. Anhydrous pyridine (3.39 g; 3.49 $cm^3$; 42.82 mmol; 2.10 eq.) is slowly added and the mixture stirred at 50 °C for 1 hour under a flow of nitrogen. The clear yellow solution obtained is transferred to a flask containing triethoxy-acetic acid ethyl ester (16.62 g; 75.45 mmol; 3.70 eq.) and yttrium(III) triflate (0.57 g; 1.02 mmol;

0.05 eq.). Slow precipitation is observed. The mixture is stirred at 60 °C for 72 hours under nitrogen. Subsequently, the mixture is cooled to room temperature. The precipitate is isolated by filtration and washed copiously with methanol, yielding the product as a yellow powder (4.07 g, 59.3%).
$^1$H NMR (300 MHz, Chloroform-d) δ: 8.84 (s, 2H), 4.60 (q, 4h), 1.52 (t, 6H).

Benzo[1,2-d;4,5-d']bisthiazole-2,6-dicarboxylic acid didodecyl ester (1.2)

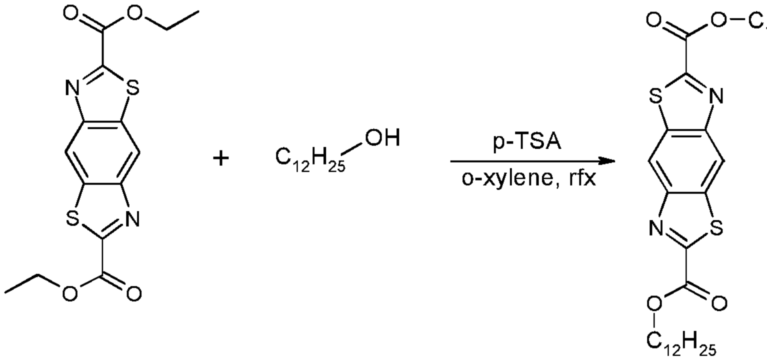


(1.2)

Benzo[1,2-d;4,5-d']bisthiazole-2,6-dicarboxylic acid diethyl ester (4.07 g; 12.10 mmol; 1.00 eq.), toluene-4-sulfonic acid (0.42 g; 2.42 mmol; 0.20 eq.) and dodecan-1-ol (4.73 g; 25.41 mmol; 2.10 eq.) are dissolved in o-xylene (200 cm$^3$). Dean-Stark apparatus is attached to the flask and mixture is heated at 140 °C for 16 hours. The solvent is removed in vacuo, and another portion of dodecan-1-ol (4.73 g; 25.41 mmol; 2.10 eq.), toluene-4-sulfonic acid (0.42 g; 2.42 mmol; 0.20 eq.) and o-xylene (200 cm$^3$) are added and the mixture refluxed under Dean-Stark trap for 16 hours. Subsequently, the solution is cooled to ca. 60 °C and passed through a short silica plug. The solvent is removed in vacuo and the residue is recrystallized from boiling petroleum ether (100 cm$^3$, b.p. 80-100 °C), yielding the product as a light yellow powder (4.89 g; 65.5 %).
$^1$H NMR (400 MHz, Chloroform-d) δ: 8.84 (s, 2H), 4.52 (t, 4H, J = 6.84 Hz), 1.88 (m, 4H), 1.53-1.20 (m, 36H), 0.86 (t, 4H, J = 7.0Hz).

4,8-Dibromo-benzo[1,2-d;4,5-d']bisthiazole-2,6-dicarboxylic acid didodecyl ester (1)

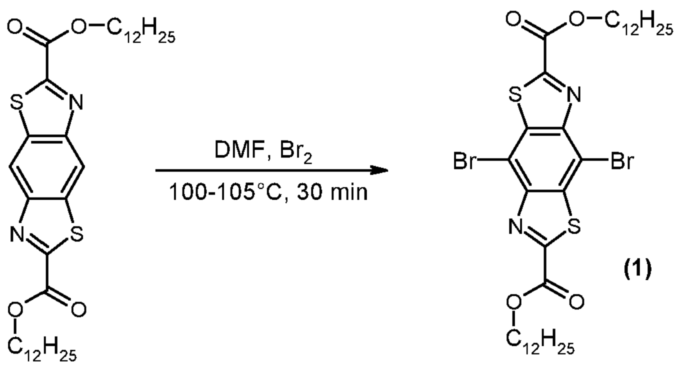


(1)

Benzo[1,2-d;4,5-d']bisthiazole-2,6-dicarboxylic acid didodecyl ester (1.60 g; 2.59 mmol; 1.00 eq.) is dissolved in anhydrous N,N-dimethylformamide (24.0 cm$^3$), stirred at 60 °C for 30 minutes. Subsequently, bromine (16.99 g; 5.45 cm$^3$; 4 eq.) is added by syringe over a period of 5 minutes. The solution is then heated at 100-105 °C for 30 minutes, under nitrogen. The exhaust fumes are passed through a bubbler filled with aqueous sodium sulphite. Subsequently, the reaction is quenched by pouring into 200 cm$^3$ saturated aq. $Na_2SO_3$ and filtered. The solid is washed with water and methanol and purified by silica gel column chromatography (eluent: dichloromethane), to yield the product as yellow crystals (837 mg, 41.7%). $^1$H NMR (300 MHz, Chloroform-d) δ 4.51 (t, J = 6.83 Hz. 4H), 1.94 - 1.80 (m, 4H), 1.52-1.19 (m, 36H), 0.87 (t, J = 7.05 Hz, 6H).

Example M2

Monomer **2** was prepared as follows:

Benzo[1,2-d;4,5-d']bisthiazole-2,6-dicarboxylic acid bis-(3,7-dimethyl-octyl) ester (2.1)

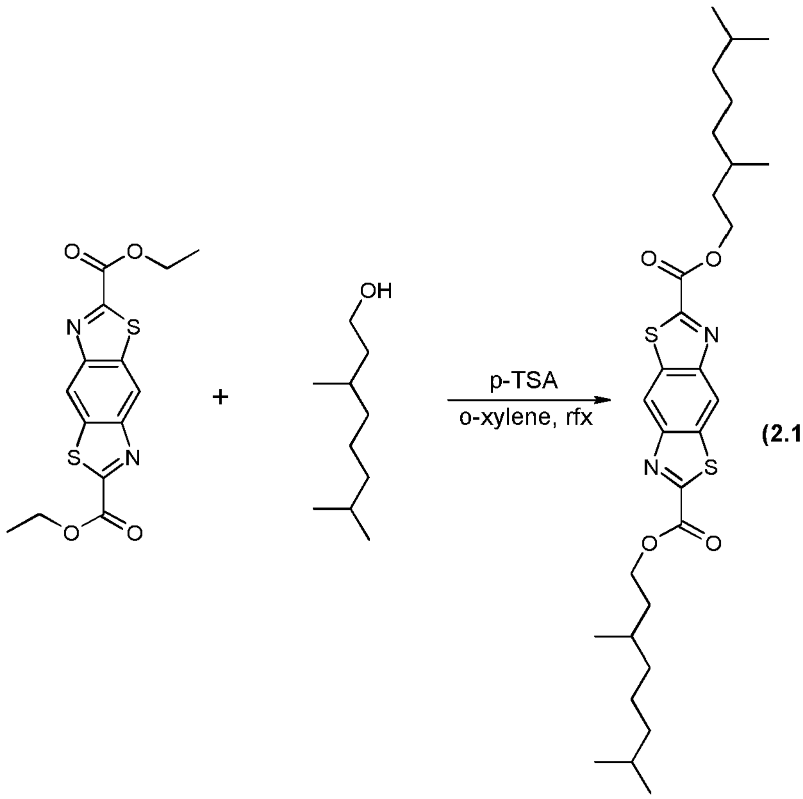


**(2.1)**

Benzo[1,2-d;4,5-d']bisthiazole-2,6-dicarboxylic acid diethyl ester (0.99 g; 2.94 mmol; 1.00 eq.), toluene-4-sulfonic acid (0.10 g; 0.589 mmol; 0.20 eq.) and 3,7-dimethyl-octan-1-ol (0.98 g; 6.18 mmol; 2.10 eq.) are dissolved in o-xylene (50 $cm^3$). Dean-Stark apparatus is attached to the flask and the mixture is heated at 140 °C for 16 hours. The solvents are removed in vacuo and the residue is redissolved in dichloromethane (50 $cm^3$). The product is precipitated by addition of methanol (50 $cm^3$), water (200 $cm^3$) and additional methanol (until disappearance of the 3,7-dimethyl-octan-1-ol phase), collected by filtration and purified by silica gel column chromatography (eluent: petroleum ether b.p. 40-60 °C / dichloromethane). Yield: 1.06 g (64.5%).

4,8-Dibromo-benzo[1,2-d;4,5-d']bisthiazole-2,6-dicarboxylic acid bis-(3,7-dimethyl-octyl) ester (2)

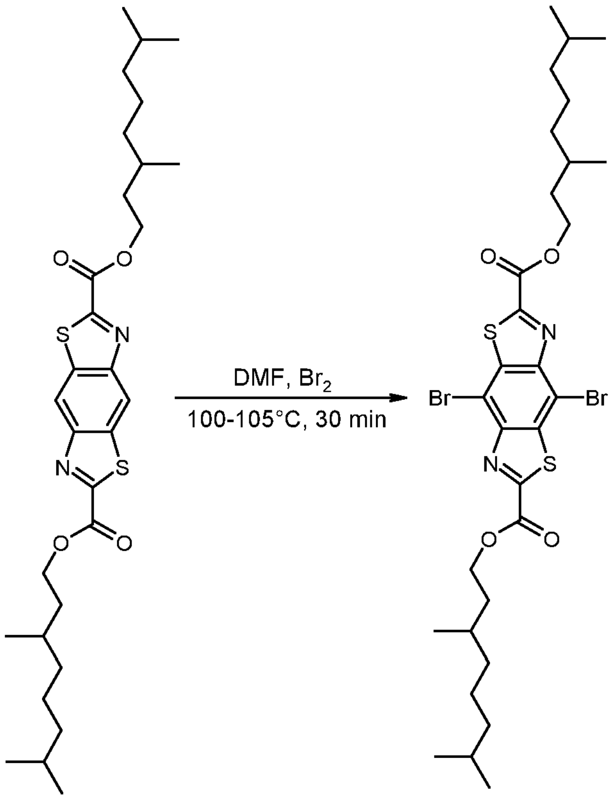


**(2)**

Benzo[1,2-d;4,5-d']bisthiazole-2,6-dicarboxylic acid bis-(3,7-dimethyl-octyl) ester (1.06 g; 1.89 mmol; 1.00 eq.) is dissolved in anhydrous N,N-dimethylformamide (16 $cm^3$) at 60 °C. Bromine (12.38 g; 3.97 $cm^3$; 77.49 mmol; 41.00 eq.) is added and the solution is heated at 110 °C for 30 minutes under nitrogen. Subsequently, the reaction is quenched by pouring into 200 $cm^3$ saturated aq. $Na_2SO_3$ and filtered. The solid is washed with water and methanol and purified by silica gel column chromatography (eluent: petroleum ether / dichloromethane), to yield the product as a light yellow powder (571 mg; 0.79 mmol; 42.0%).

1H NMR (500 MHz, Chloroform-d) $\delta$ 4.60 - 4.51 (m, 4H), 1.95-1.87 (m, 2H), 1.73 - 1.62 (m, 4H), 1.59 - 1.48 (m, 2H), 1.41 - 1.13 (m, 12H), 0.99 (d, J = 6.4 Hz, 6H), 0.90 - 0.82 (m, 12H).

Example M3

Compound **3.1** was prepared as follows:

2,6-Didodecyl-benzo[1,2-d;4,5-d']bisthiazole (3.1)

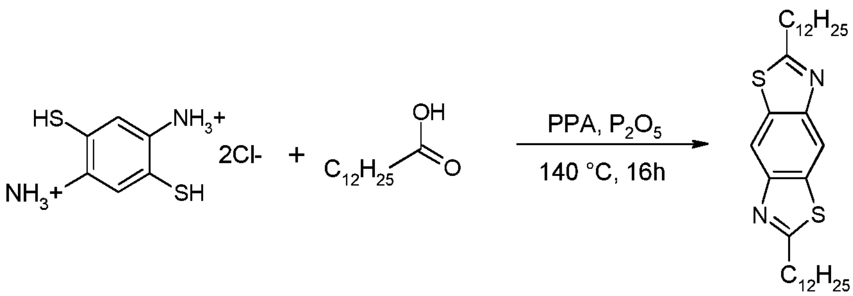


**(3.1)**

To 3-necked flask equipped with a mechanical agitator is added polyphosphoric acid (40 g) and 2,5-diamino-benzene-1,4-dithiol hydrochloride (5.00 g, 20.39 mmol, 1.00 eq). The mixture is stirred and heated at 70 °C and 13 mbar for 5 minutes and at 100 °C and 10 mbar for a further 30 minutes. The mixture is then allowed to cool to 60 °C, tridecanoic

acid (8.96 g, 41.80 mmol, 2.05 eq.) and phosphorous pentoxide (26.0 g, 91.58 mmol, 4.49 eq.) are added. The mixture is stirred at 100 °C for 30 minutes followed by stirring at 140 °C for 16 hours. Subsequently, the reaction mixture was cooled to 80 °C and water was added (30 $cm^3$). The mixture was stirred for 1 hour, allowed to cool to 40 °C and dichloromethane (100 $cm^3$) is added. The phases are separated, the organic phase is washed with aq. sodium hydroxide solution, dried over magnesium sulphate and concentrated in vacuo. The product is obtained as a yellow solid (5.00 g, 46.4%).
$^1$H NMR (300 MHz, Chloroform-d) $\delta$ 8.38 (s, 1H), 3.13 (t, 4H, J = 7.7 Hz), 1.96-1.82 (m, 4H), 1.50-1.20 (m, 18H), 0.88 (t, J = 6.7 Hz, 4H).

Example M4

Monomer **4** was prepared as follows:

2,6-Bis-(1-hexyl-nonyl)-benzo[1,2-d;4,5-d']bisthiazole (4.1)

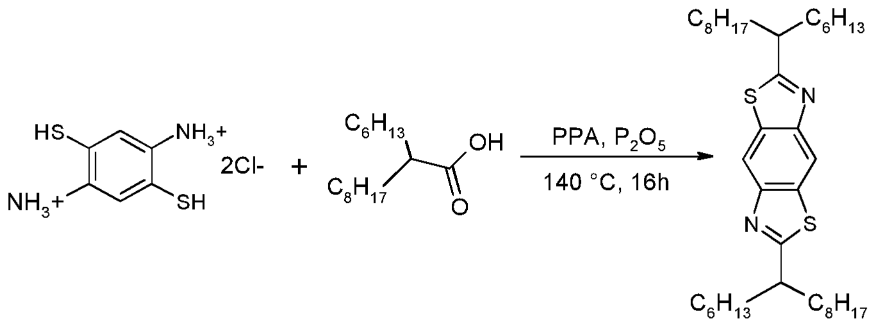


(4.1)

To 3-necked flask equipped with a mechanical agitator is added polyphosphoric acid (20.0 g) and 2,5-diamino-benzene-1,4-dithiol hydrochloride (2.50 g; 10.20 mmol; 1.00 eq.)). The mixture is stirred and heated at 100 °C and 10 mbar for 2 hours. The mixture is then allowed to cool to 60 °C, 2-hexyl-decanoic acid (5.36 g; 20.90 mmol; 2.05 eq.) and phosphorous pentoxide (13.03 g; 45.88 mmol; 4.50 eq.) are added. The mixture is carefully degassed and stirred at 140 °C for 18 hours, under nitrogen. Subsequently, the reaction mixture is cooled to 80 °C and water was added (50 $cm^3$). The mixture is extracted with dichloromethane, washed with saturated aq. $NaHCO_3$, washed with brine, dried over $MgSO_4$ and filtered. The solvent is removed in vacuo and the residue purified by column chromatography on silica (eluent: petroleum ether / dichloromethane), yielding the product as a yellow oil (4.38 g; 70.1%).
1H NMR (300 MHz, Chloroform-d) $\delta$: 8.41 (s, 2H), 3.15 (tt, J=7.2Hz, 2H), 1.79 (m, 8H, CH-CH2), 1.2-1.4 (m, 40H), 0.8-0.9 (m, 12H).

4,8-Dibromo-2,6-bis-(1-hexyl-nonyl)-benzo[1,2-d;4,5-d']bisthiazole (4)

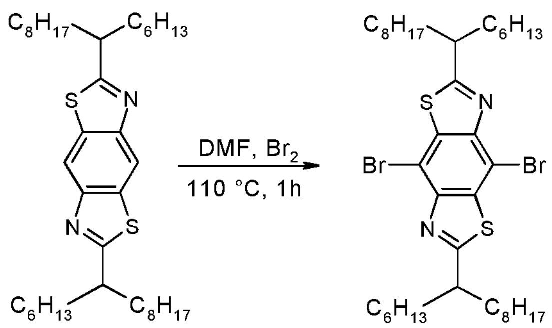


**(4)**

2,6-Bis-(1-hexyl-nonyl)-benzo[1,2-d;4,5-d']bisthiazole (4.38 g; 7.14 mmol; 1.00 eq.) is dissolved in anhydrous N,N-dimethylformamide (75 $cm^3$). Bromine (46.8 g; 15.0 $cm^3$; 292.9 mmol; 41.0 eq.) is added and the solution is heated

at 110 °C for 30 minutes under nitrogen. Subsequently, the reaction is quenched by pouring into 200 $cm^3$ saturated aq. $Na_2SO_3$. The reaction mixture is extracted with dichloromethane (2× 100 $cm^3$), combined organic phases washed with water, dried over anhydrous magnesium sulphate and filtered. The solvent is removed in vacuo and the residue purified by silica gel column chromatography (eluent: petroleum ether b.p. 40-60 °C / dichloromethane), yielding the product as a yellow oil (2.26 g, 41.0%).
1H NMR (400 MHz, Chloroform-d) δ 3.23 (m, 2H), 1.77 (m, 8H), 1.2-1.4 (m, 40H), 0.8-0.9 (m, 12H).

Example M5

Monomer **5** was prepared as follows.

2,6-Bis-(3-ethyl-heptyl)-benzo[1,2-d;4,5-d']bisthiazole (**5.1**)

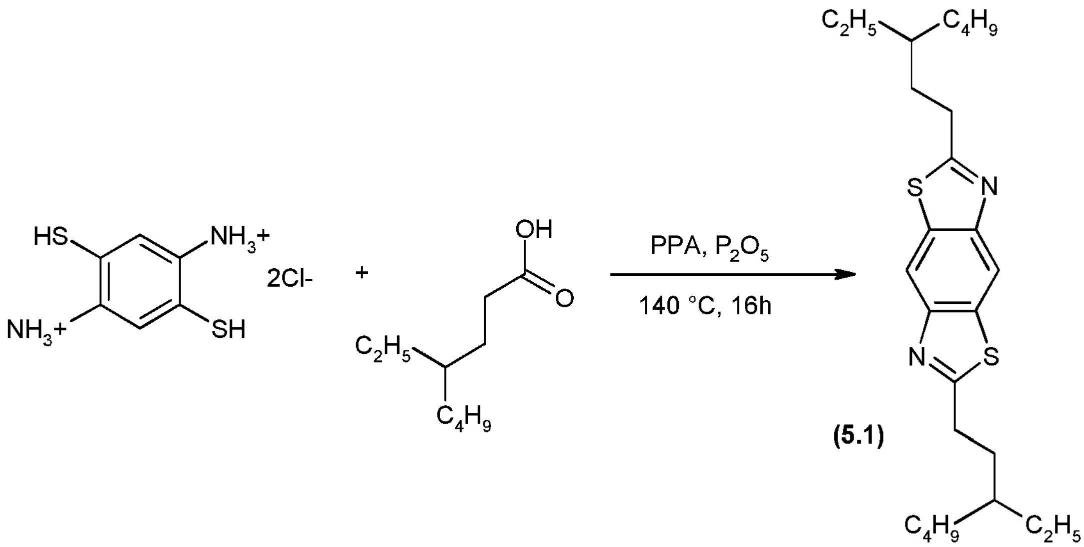


**(5.1)**

To 3-necked flask equipped with a mechanical agitator is added polyphosphoric acid (48 g) and 2,5-diamino-benzene-1,4-dithiol hydrochloride (6.00 g, 24.47 mmol, 1.00 eq). The mixture is stirred and heated at 70 °C and 13 mbar for 5 minutes and at 100 °C and 10 mbar for a further 30 minutes. The mixture is then allowed to cool to 60 °C, 4-ethyl-octanoic acid (8.64 g, 50.16 mmol, 2.05 eq.) and phosphorous pentoxide (31.26 g, 110.12 mmol, 4.49 eq.) are added. The mixture is stirred at 100 °C for 30 minutes followed by stirring at 140 °C for 16 hours. Subsequently, the reaction mixture was cooled to 80 °C and water was added (30 $cm^3$). The mixture was stirred for 1 hour, allowed to cool to 40 °C and dichloromethane (100 $cm^3$) is added. The phases are separated, the organic phase is washed with aq. sodium hydroxide solution, dried over magnesium sulphate, passed through a silica plug and concentrated in vacuo. The product is obtained as a pale brown solid (5.25 g, 48.3%).
$^1$H NMR (400 MHz, Chloroform-d) δ 8.38 (s, 2H), 3.16 - 3.07 (m, 4H), 1.91 - 1.81 (m, 4H), 1.47 - 1.36 (m, 8H), 1.36 - 1.22 (m, 10H), 0.96 - 0.84 (m, 12H).

4,8-Dibromo-2,6-bis-(3-ethyl-heptyl)-benzo[1,2-d;4,5-d']bisthiazole (5.2)

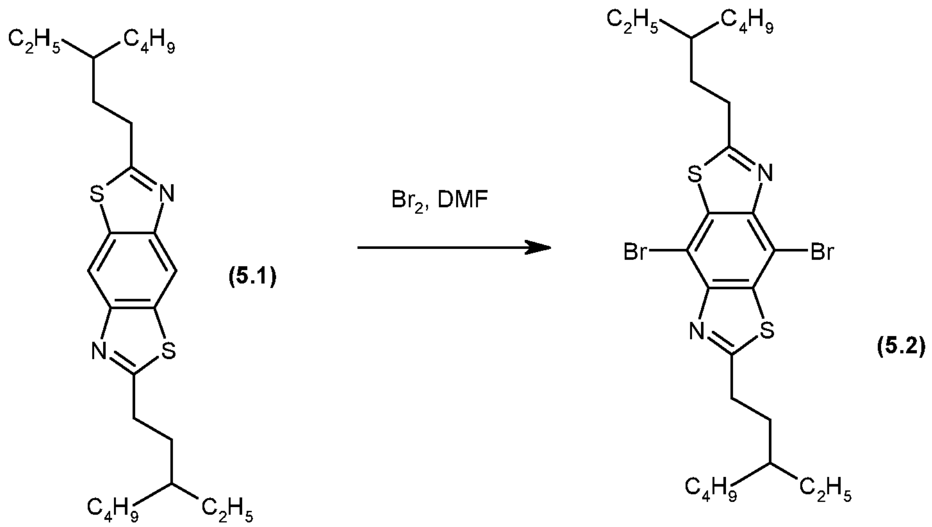


(5.1) (5.2)

2,6-Bis-(3-ethyl-heptyl)-benzo[1,2-d;4,5-d']bisthiazole (5.25 g; 11.80 mmol; 1.00 eq.) is dissolved in anhydrous N,N-dimethylformamide (125 $cm^3$). Bromine (77.35 g; 24.8 $cm^3$; 483.99 mmol; 41.0 eq.) is added and the solution is heated at 110 °C for 10 minutes under nitrogen. Subsequently, the reaction is quenched by pouring into 200 $cm^3$ saturated aq. $Na_2SO_3$. The product precipitates and is collected by extraction with DCM. The crude product dissolved in petrol:DCM (roughly 1:4) and passed through a silica plug, the solvents removed, the residue dissolved in warm petrol and purified by column chromatography on silica (eluent: petroleum ether b.p. 40-60 °C / dichloromethane), yielding the product as a yellow oil (2.30 g, 32.0%).
$^1$H NMR (400 MHz, Chloroform-d) $\delta$ 3.16 - 3.01 (m, 4H), 1.77 (dt, J = 11.6, 6.3 Hz, 2H), 1.54-1.38 (m, 8H), 1.38 - 1.20 (m, 12H), 0.94 - 0.76 (m, 12H).

4,8-Bis-(4-dodecyl-thiophen-2-yl)-2,6-bis-(3-ethyl-heptyl)-benzo[1,2-d:4,5-d']bisthiazole (5.3)

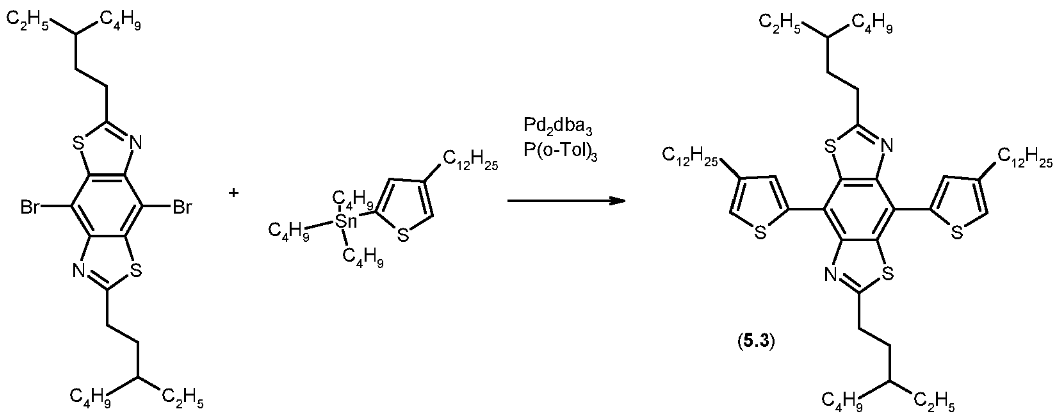


(5.3)

4,8-Dibromo-2,6-bis-(3-ethyl-heptyl)-benzo[1,2-d;4,5-d']bisthiazole (1.10 g; 1.83 mmol; 1.00 eq.), tributyl-(4-dodecyl-thiophen-2-yl)-stannane (2.27 g; 4.20 mmol; 2.30 eq.), Pd2(dba)3 (0.13 g; 0.15 mmol; 0.08 eq.) and tri-o-tolyl-phosphane (0.09 g; 0.29 mmol; 0.16 eq.) are placed into round bottom flask and degassed for 15 minutes. Degassed toluene (13 $cm^3$) is added and the reaction mixture is further degassed for 30 mins. The reaction mixture is heated at 120 °C overnight. After cooling to room temperature, the reaction mixture is precipitated into methanol (150 $cm^3$). A fine red precipitate is formed, and a dark, oily liquid phase appears on the bottom of the flask. The fine precipitate is filtered off (glass fibre membrane, suction), redissolved in cyclohexane and purified on column (Biotage, 100g cartridge, petroleum ether/dichloromethane followed by Biotage, 100g cartridge, petroleum ether isocratic) to yield the product as an off-white solid (27 % yield).
$^1$H NMR (400 MHz, Chloroform-d) $\delta$ 7.00 (d, *J* = 1.4 Hz, 2H, thiophene Carom-H), 6.79 (d, *J* = 1.4 Hz, 2H, thiophene Carom-H), 2.59 (t, *J* = 7.7 Hz, 4H), 1.64 (m, 4H), 1.28 (s, 74H), 0.95 - 0.86 (m, 6H).

4,8-Bis-(5-bromo-4-dodecyl-thiophen-2-yl)-2,6-bis-(3-ethyl-heptyl)-benzon[1,2-d:4.5-d']bisthiazole (5)

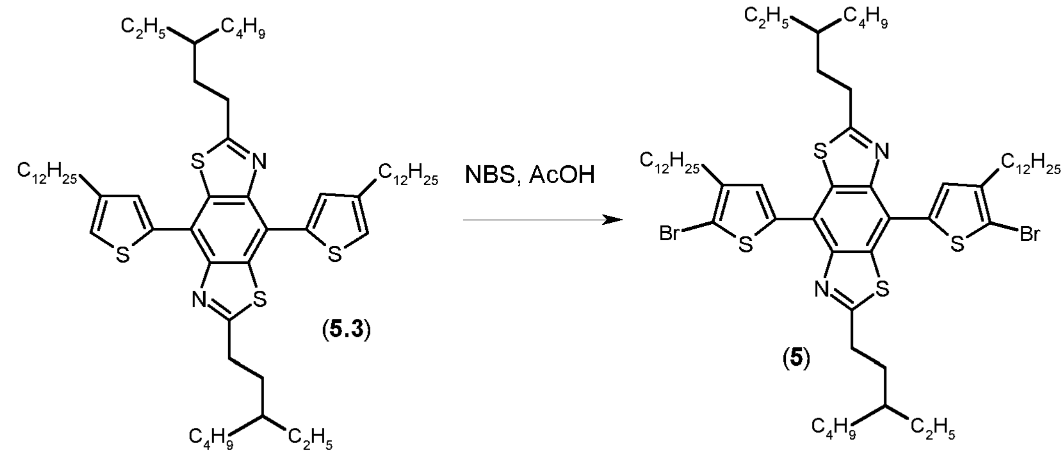


(**5.3**) (**5**)

To a round bottom flask is added 4,8-bis-(4-dodecyl-thiophen-2-yl)-2,6-bis-(3-ethyl-heptyl)-benzo[1,2-d;4,5-d']bisthiazole (352.00 mg; 0.37 mmol; 1.00 eq.), chloroform (8.71 $cm^3$), acetic acid (2.90 $cm^3$). The mixture is stirred at room temperature. 1-Bromo-pyrrolidine-2,5-dione (139.13 mg; 0.78 mmol; 2.10 eq.) is added in one portion and the mixture stirred until dissolution and then left at room temperature for 6 days. Water (50 $cm^3$) is added and the product is extracted with petroleum ether (200 $cm^3$). The crude material is purified on column (Biotage, 100 g cartridge, petroleum ether) to yield product as a solid.
$^1$H NMR (400 MHz, Chloroform-d) $\delta$ 6.77 (s, 2H, Carom-H), 2.51 (d, J = 7.7 Hz, 4H), 1.62 - 1.50 (m, 8H), 1.35-1.20 (m, 68H), 0.88 (t, J = 6.7 Hz, 8H).

Example M6

Compound **6** was prepared as follows

4,8-Bis-(4-dodecyl-thiophen-2-yl)-benzo[1,2-d;4,5-d']bisthiazole-2,6-dicarboxylic acid didodecyl ester (**6**)

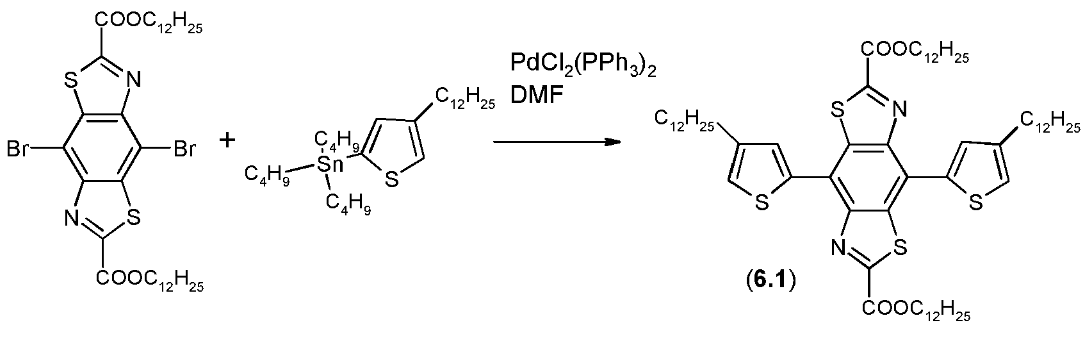


(**6.1**)

4,8-Dibromo-benzo[1,2-d;4,5-d']bisthiazole-2,6-dicarboxylic acid didodecyl ester (1.00 g; 1.29 mmol; 1.00 eq.), tributyl-(4-dodecyl-thiophen-2-yl)-stannane (2.10 g; 3.87 mmol; 3.00 eq.), PdCl2(PPh3)2 (36.34 mg; 0.05 mmol; 0.04 eq.) are charged into a round-bottomed flask and degassed. *N,N*-Dimethylformamide (24 $cm^3$) is added and the mixture is degassed for further 10 minutes and heated to 120°C for 2h under nitrogen atmosphere with vigorous stirring. Subsequently, the mixture is allowed to cool to 25°C and 400 $cm^3$ of methanol is added. The precipitate is filtered off, dried in air and purified by column chromatography using petroleum ether (b.p. 40-60°C) and dichloromethane as eluents. The yield is 262 mg (18%).
$^1$H NMR (400 MHz, Chloroform-d) $\delta$ 7.85 (d, J = 1.4 Hz, 2H), 7.21 (d, J = 1.4 Hz, 2H), 4.50 (t, J = 6.7 Hz, 4H), 2.74 (m 4H), 1.86 (m, 4H), 1.71 (m, 4H), 1.55-1.25 (m, 72H), , 0.86 (m, 12H).

Example M7

Monomer **7** was prepared as follows

4,8-Bis-[4-(2-octyl-dodecyl)-thiophen-2-yl]-benzo[1,2-d;4,5-d']bisthiazole-2,6-dicarboxylic acid didodecyl ester (**7**.1)

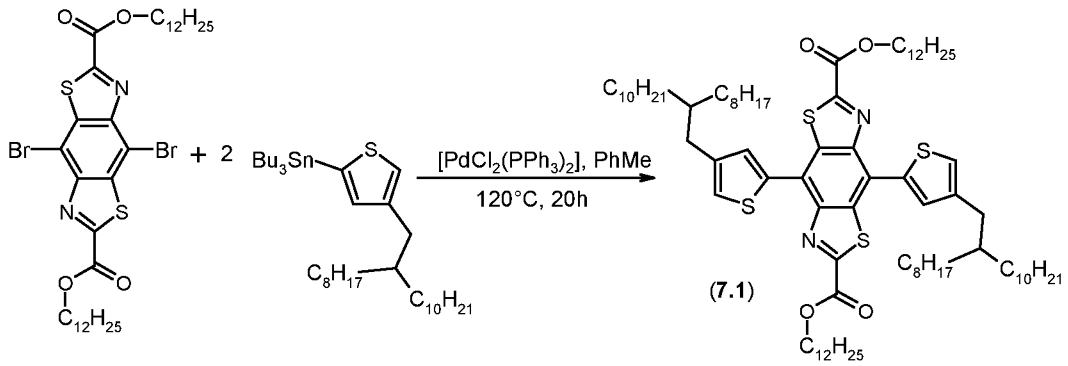


(**7.1**)

4,8-Dibromo-benzo[1,2-d;4,5-d']bisthiazole-2,6-dicarboxylic acid didodecyl ester (571 mg; 0.74 mmol; 1.00 eq.), tributyl-[4-(2-octyl-dodecyl)-thiophen-2-yl]-stannane (1108 mg; 1.70 mmol; 2.30 eq.), [$Pd_2(dba)_3$] (54 mg; 0.06 mmol; 0.08 eq.), tri-o-tolyl-phosphane (36 mg; 0.12 mmol; 0.16 eq.) are placed into a round bottomed flask and degassed. Toluene (6.0 $cm^3$) is added and the reaction mixture is carefully degassed for 30 minutes. The mixture is then heated to 120°C for 20h under nitrogen atmosphere and vigorous stirring. Subsequently, the mixture is allowed to cool to 25°C, diluted by addition of dichloromethane (20 $cm^3$) and precipitated by addition of methanol (400 $cm^3$). The precipitate was filtered off and purified by column chromatography using petroleum ether (bp. 40-60°C) and dichloromethane as eluents. The product is obtained as a dark red oil which solidifies on standing (688 mg, 69.5%).
$^1$H NMR and $^1$H-$^1$H COSY NMR (400 MHz, Chloroform-d) $\delta$ 7.81 (d, $J$ = 1.4 Hz, 2H, thiophene Carom-H), 7.19 (d, $J$ = 1.4 Hz, 2H, thiophene Carom-H), 4.49 (t, $J$ = 6.8 Hz, 4H, COOC$H_2$), 2.68 (d, $J$ = 6.8 Hz, 4H, $C_{arom}$-C$H_2$), 1.93 - 1.83 (m, 4H, $COOCH_2$-C$H_2$), 1.71 (bs, 2H, Carom-CH2-CH), 1.55 - 1.45 (m, 4H, $COOCH_2$-$CH_2$-C$H_2$), 1.42 - 1.14 (m, 76H), 0.91 - 0.82 (m, 18H, -$CH_3$).

4,8-Bis-[5-bromo-4-(2-octyl-dodecyl)-thiophen-2-yl]-benzo[1,2-d;4,5-d']bisthiazole-2,6-dicarboxylic acid didodecyl ester (**7**)

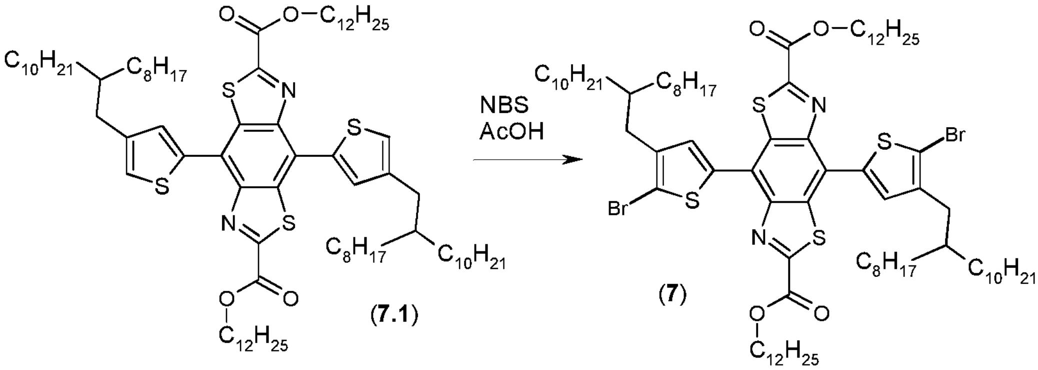


(**7.1**) (**7**)

To a round-bottom flask is added 4,8-bis-[4-(2-octyl-dodecyl)-thiophen-2-yl]-benzo[1,2-d;4,5-d']bisthiazole-2,6-dicarboxylic acid didodecyl ester (688.00 mg; 0.51 mmol; 1.00 eq.), chloroform (12 $cm^3$), acetic acid (4 $cm^3$). The mixture is stirred at r.t. and degassed. 1-Bromo-pyrrolidine-2,5-dione (187.02 mg; 1.05 mmol; 2.05 eq.) is added in one portion and the mixture stirred for 2 hours(TLC control). The product is precipitated by addition of methanol (200 $cm^3$) and filtered off. The crude material is then dissolved in cyclohexane and purified by column chromatography using petroleum ether (b.p. 40-60°C) and dichloromethane as eluents and recrystallised from iso-propanol. The yield is 641 mg (83.4%).

$^1$H NMR (400 MHz, Chloroform-d) δ 7.65 (s, 2H, thiophene Carom-H), 4.54 (t, *J* = 6.7 Hz, 4H, COOC$H_2$), 2.65 (d, *J* = 7.1 Hz, 4H, $C_{arom}$-C$H_2$), 1.91 (m, 4H, COOCH2-C$H_2$), 1.78 (bs, 2H, Carom-CH2-CH), 1.62 - 1.50 (m, 4H, $COOCH_2$-$CH_2$-C$H_2$), 1.49 - 1.17 (m, 76H), 0.94 - 0.81 (m, 18H).

Example P1

Polymer **P1** was prepared as follows.

Poly-[2,6-(4,8-didodecyl-benzo[1,2-b;4,5-b']dithiophene)-co-4,7-(5,6-bis-octyloxy-benzo[1,2,5]thiadiazole)-co-4,8-(benzo[1,2-d;4,5-d']bisthiazole-2,6-dicarboxylic acid didodecyl ester)] (P1)

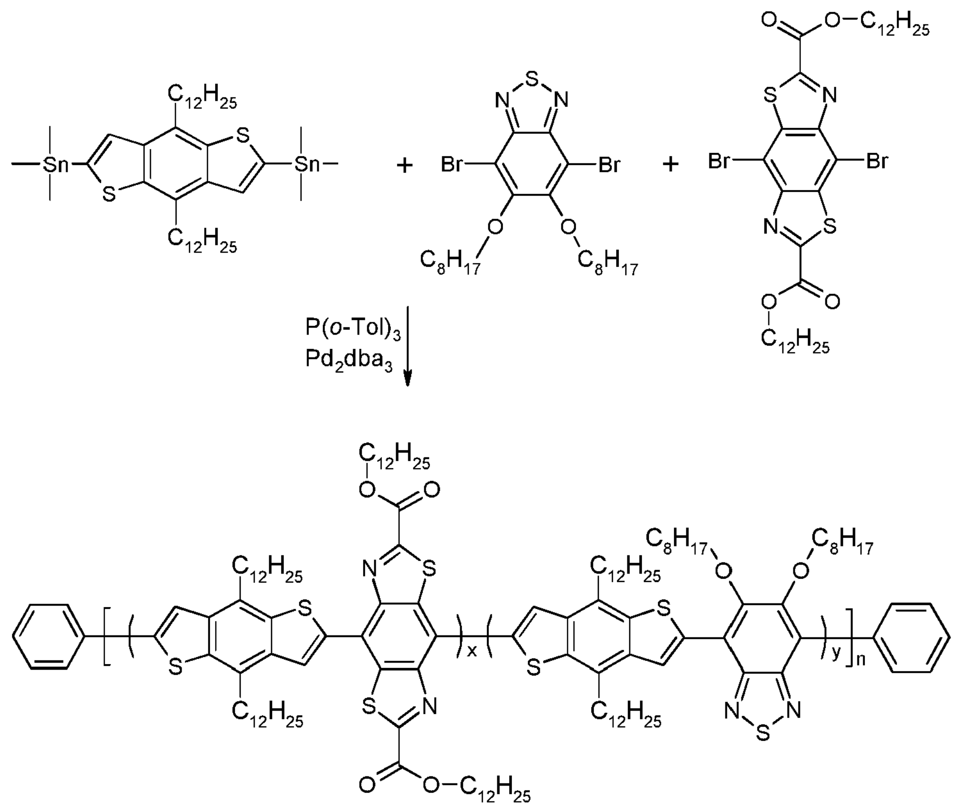


To a round bottomed flask is added 4,8-didodecyl-2,6-bis-trimethylstannanyl-benzo[1,2-b;4,5-b']dithiophene (426.27 mg; 0.50 mmol; 1.00 eq.), 4,7-dibromo-5,6-bis-octyloxy-benzo[1,2,5]thiadiazole (137.60 mg; 0.25 mmol; 0.50 eq.), 4,8-dibromo-benzo[1,2-d;4,5-d']bisthiazole-2,6-dicarboxylic acid didodecyl ester (193.68 mg; 0.25 mmol; 0.50 eq.), tris(dibenzylideneacetone)dipalladium(0) (18.3 mg; 20 μmol; 0.04 eq.) and tri-o-tolyl-phosphine (24.4 mg; 80 μmol; 0.16 eq.). To the vessel is added degassed chlorobenzene (6.25 $cm^3$) and the reaction mixture is degassed further for 15 minutes. The reaction mixture is heated to 110 °C and stirred at this temperature for 1 hour. Tributyl-phenyl-stannane (734 mg; 2 mmol; 4.00 eq.) is then added followed by an additional 1 hour heating at 130 °C. Next, bromo-benzene (471 mg; 3.00 mmol; 6 eq.) is added followed by an additional 1 hour heating at 130 °C. After completion of the reaction, the reaction mixture is allowed to cool to 60 °C and precipitated into stirred methanol (100 $cm^3$). The polymer is collected by filtration and washed with methanol (2 x 10 $cm^3$) to give a solid. The polymer is sequentially extracted by Soxhlet extraction with acetone, petroleum ether (40-60 °C), cyclohexane, dichloromethane, chloroform and chlorobenzene. The chloroform fraction is precipitated by addition to stirred methanol (100 $cm^3$) and collected by filtration to give a black solid (51 mg).

GPC (50 °C, chlorobenzene): Mn=88.8 kg/mol, Mw=410.7 kg/mol, PDI=4.62

The chlorobenzene fraction is precipitated by addition to stirred methanol (100 $cm^3$) and collected by filtration to give a black solid (385 mg).

GPC (50 °C, chlorobenzene): Mn=103.3 kg/mol, Mw=569.4 kg/mol, PDI=5.50

Example P2

Polymer **P2** was prepared as follows.

Poly-[2,6-(4,8-didodecyl-benzo[1,2-b;4,5-b']dithiophene)-co-4,8-(6-(3,7-dimethyl-octyl)-[1,2,5]thiadiazolo[3,4-e]isoindole-5,7-dione)-co-4,8-(benzo[1,2-d;4,5-d']bisthiazole-2,6-dicarboxylic acid didodecyl ester)] (P2)

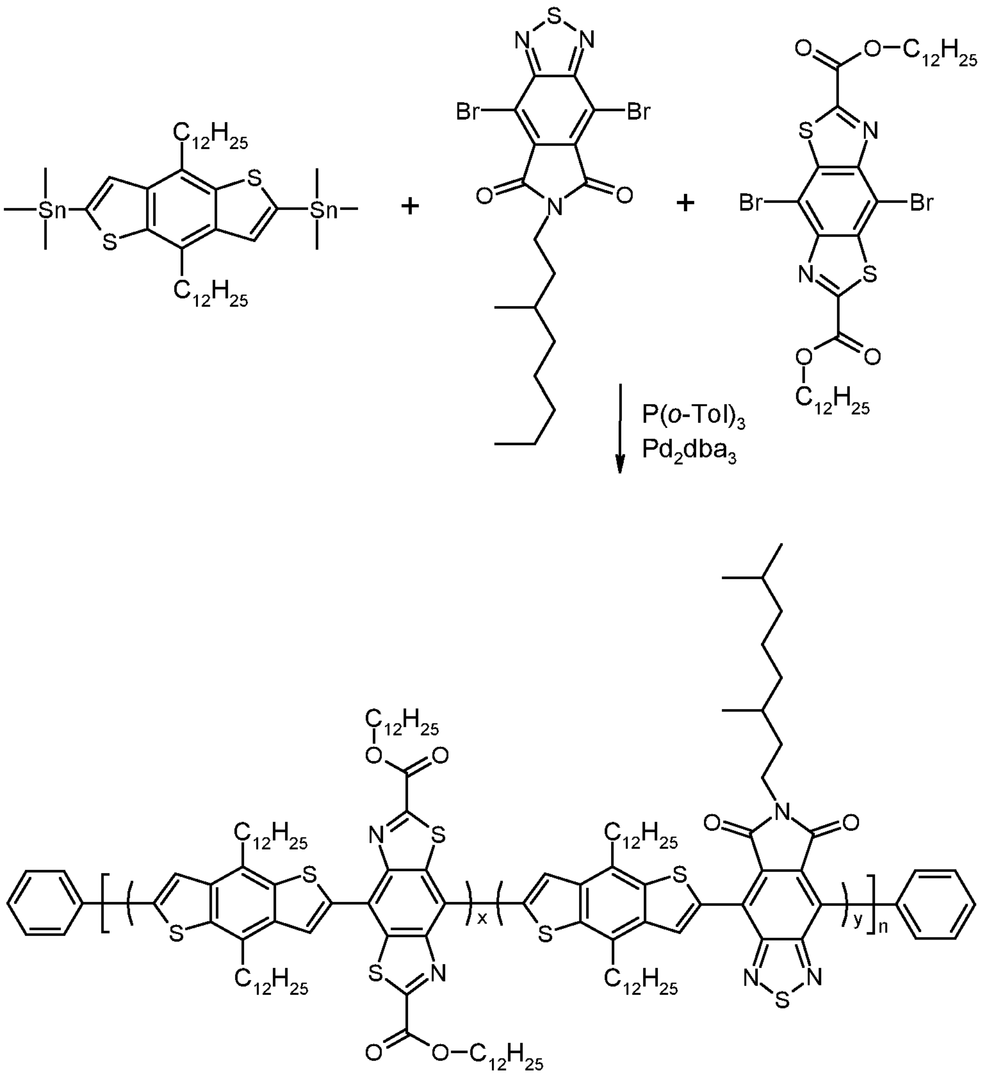


To a round bottomed flask is added 4,8-didodecyl-2,6-bis-trimethylstannanyl-benzo[1,2-b;4,5-b']dithiophene (298.39 mg; 0.35 mmol; 1.00 eq.), 4,8-dibromo-benzo[1,2-d;4,5-d']bisthiazole-2,6-dicarboxylic acid didodecyl ester (203.36 mg; 0.26 mmol; 0.75 eq.), 4,8-dibromo-6-(3,7-dimethyl-octyl)-[1,2,5]thiadiazolo[3,4-e]isoindole-5,7-dione (44.03 mg; 0.09 mmol; 0.25 eq.), tri-o-tolyl-phosphine (17 mg; 56 $\mu$mol; 0.16 eq.) and tris(dibenzylideneacetone)dipalladium(0) (12.8 mg; 14 $\mu$mol; 0.04 eq.). To the vessel is added degassed chlorobenzene (4.4 $cm^3$) and the reaction mixture is degassed further for 15 minutes. The reaction mixture is heated to 110 °C and stirred at this temperature for 1 hour. Tributyl-phenyl-stannane (514 mg; 1.40 mmol; 4.00 eq.) is then added followed by an additional 1 hour heating at 130 °C. Next, bromobenzene (329.72 mg; 2.10 mmol; 6 eq.) is added followed by an additional 1 hour heating at 130 °C. After completion of the reaction, the reaction mixture is allowed to cool to 60 °C and precipitated into stirred methanol (100 $cm^3$). The polymer is collected by filtration and washed with methanol (2 x 10 $cm^3$) to give a solid. The polymer is sequentially extracted by Soxhlet extraction with acetone, petroleum ether (40-60 °C), cyclohexane, chloroform and chlorobenzene. The cyclohexane fraction is precipitated by addition to stirred methanol (100 $cm^3$) and collected by filtration to give a black solid (306 mg).

GPC (50 °C, chlorobenzene): Mn=11.3 kg/mol, Mw=23.3 kg/mol, PDI=2.061

Example P3

Polymer **P3** was prepared as follows.

Poly-[2,6-(4,8-didodecyl-benzo[1,2-b;4,5-b']dithiophene)-co-2,6-(benzo[1,2-b:4,5-b']dithiophene-4,8-dicarboxylic acid didodecyl ester)-co-4,8-(benzo[1,2-d;4,5-d']bisthiazole-2,6-dicarboxylic acid didodecyl ester)] (P3)

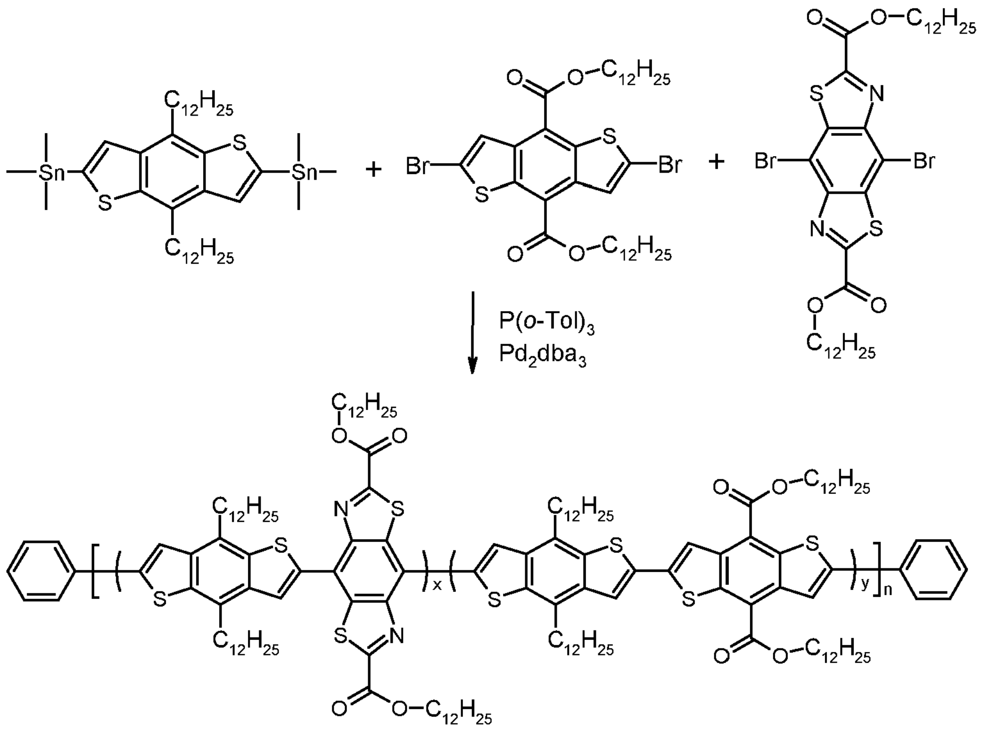


To a round bottomed flask is added 4,8-didodecyl-2,6-bis-trimethylstannanyl-benzo[1,2-b;4,5-b']dithiophene (255.76 mg; 0.30 mmol; 1.00 eq.), 4,8-dibromo-benzo[1,2-d;4,5-d']bisthiazole-2,6-dicarboxylic acid didodecyl ester (209.17 mg; 0.27 mmol; 0.90 eq.), 2,6-dibromo-benzo[1,2-b;4,5-b']dithiophene-4,8-dicarboxylic acid didodecyl ester (23.18 mg; 0.03 mmol; 0.10 eq.), tri-o-tolyl-phosphine (14.61 mg; 48.00 μmol; 0.16 eq.) and tris(dibenzylideneacetone)dipalladium(0) (10.99 mg; 12.00 μmol; 0.04 eq.) To the vessel is added degassed chlorobenzene (4.15 $cm^3$) and the reaction mixture is degassed further for 15 minutes. The reaction mixture is heated to 110 °C and stirred at this temperature for 1 hour. Tributyl-phenyl-stannane (441 mg; 1.20 mmol; 4.00 eq.) is then added followed by an additional 1 hour heating at 130 °C. Next, bromobenzene (282 mg; 1.20 mmol; 6 eq.) is added followed by an additional 1 hour heating at 130 °C. After completion of the reaction, the reaction mixture is allowed to cool to 60 °C and precipitated into stirred methanol (100 $cm^3$). The polymer is collected by filtration and washed with methanol (2 x 10 $cm^3$) to give a solid. The polymer is sequentially extracted by Soxhlet extraction with acetone, petroleum ether (40-60 °C), cyclohexane, chloroform and chlorobenzene. The cyclohexane fraction is precipitated by addition to stirred methanol (100 $cm^3$) and collected by filtration to give a black solid (304 mg).

GPC (50 °C, chlorobenzene): Mn=12.3 kg/mol, Mw=25.2 kg/mol, PDI=2.04

Example P4

Polymer **P4** was prepared as follows.

Poly-[2,6-(4,8-didodecyl-benzo[1,2-b;4,5-b']dithiophene)-co- 2,5-bis-thieno[3,2-b]thiophene-co-4,8-(benzo[1,2-d;4,5-d']bisthiazole-2,6-dicarboxylic acid didodecyl ester)] (P4)

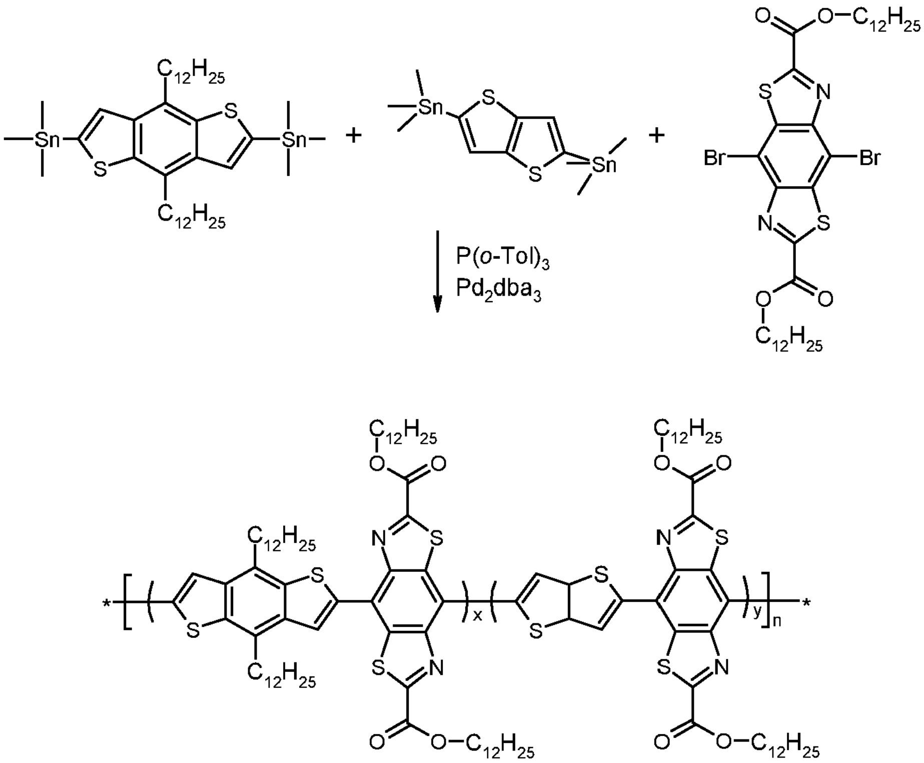


To a round bottomed flask is added 4,8-didodecyl-2,6-bis-trimethylstannanyl-benzo[1,2-b;4,5-b']dithiophene (131.39 mg; 0.15 mmol; 0.50 eq.) 2,5-bis-trimethylstannanyl-thieno[3,2-b]thiophene (71.80 mg; 0.15 mmol; 0.50 eq.), 4,8-dibromo-benzo[1,2-d;4,5-d']bisthiazole-2,6-dicarboxylic acid didodecyl ester (238.80 mg; 0.31 mmol; 1.00 eq.), tri-o-tolyl-phosphine (15 mg; 49 μmol; 0.16 eq.) and tris(dibenzylideneacetone)dipalladium(0) (11 mg; 12 μmol; 0.04 eq.). To the vessel is added degassed chlorobenzene (2.20 $cm^3$) and the reaction mixture is degassed further for 10 minutes. The reaction mixture is heated to 110 °C and stirred at this temperature for 15 minutes. After completion of the reaction, the reaction mixture is allowed to cool to 60 °C and washed with methanol. The polymer is collected by filtration and washed with methanol (2 x 10 $cm^3$) to give a solid. The polymer is sequentially extracted by Soxhlet extraction with acetone, petroleum ether (40-60 °C), cyclohexane, chloroform and chlorobenzene, but is found to be insoluble in these solvents.

Example P5

Polymer **P5** was prepared as follows.

Poly-[2,6-(4,8-didodecyl-benzo[1,2-b;4,5-b']dithiophene)-co-4,8-(benzo[1,2-d;4,5-d']bisthiazole-2,6-dicarboxylic acid didodecyl ester)-co-4,8-(2,6-bis-(1-hexyl-nonyl)-benzo[1,2-d;4,5-d']bisthiazole)] (P5)

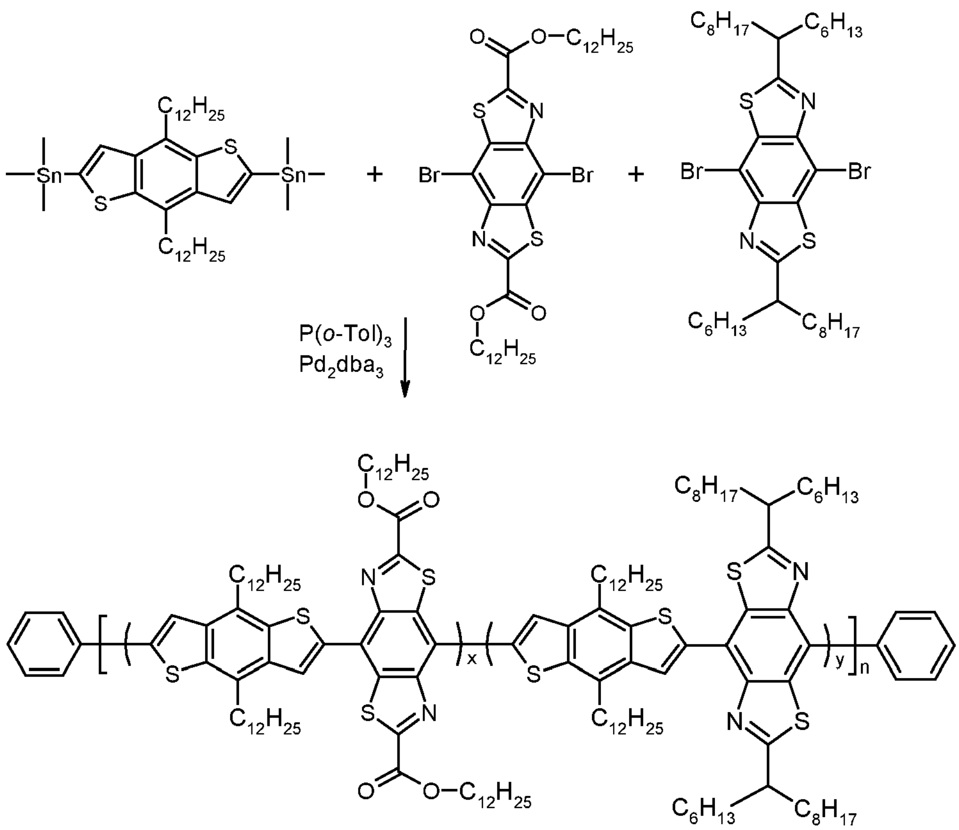


To a round bottomed flask is added 4,8-didodecyl-2,6-bis-trimethylstannanyl-benzo[1,2-b;4,5-b']dithiophene (285.44 mg; 0.33 mmol; 1.00 eq.), 4,8-dibromo-2,6-bis-(1-hexyl-nonyl)-benzo[1,2-d;4,5-d']bisthiazole (129.05 mg; 0.17 mmol; 0.50 eq.), 4,8-dibromo-benzo[1,2-d;4,5-d']bisthiazole-2,6-dicarboxylic acid didodecyl ester (129.69 mg; 0.17 mmol; 0.50 eq.), tri-o-tolyl-phosphine (16 mg; 54 μmol; 0.16 eq.) and tris(dibenzylideneacetone)dipalladium(0) (12.26 mg; 13.39 μmol; 0.04 eq.). To the vessel is added degassed chlorobenzene (4.2 $cm^3$) and the reaction mixture is degassed further for 10 minutes. The reaction mixture is heated to 110 °C and stirred at this temperature for 30 minutes. After completion of the reaction, the reaction mixture is heated to 110 °C with additional 15 $cm^3$ of chlorobenzene, cooled to 60 °C and precipitated by addition to methanol (150 $cm^3$). The polymer is collected by filtration and washed with methanol (2 x 10 $cm^3$) to give a solid. The polymer is sequentially extracted by Soxhlet extraction with acetone, petroleum ether (40-60 °C), cyclohexane, chloroform and chlorobenzene. The chloroform fraction is precipitated by addition to methanol (250 $cm^3$) and collected by filtration to give a black solid (20 mg, 5.2%).

GPC (50 °C, chlorobenzene) $M_n$ = 24.0 kg $mol^{-1}$; $M_w$ = 94.5 kg $mol^{-1}$; PDI = 3.93.

Example P6

Polymer **P6** was prepared as follows.

Poly-[4,8-(benzo[1,2-d;4,5-d']bisthiazole-2,6-dicarboxylic acid didodecyl ester)-co-4,8-(2,6-bis-(1-hexyl-nonyl)-benzo[1,2-d;4,5-d']bisthiazole)-co-2,5-bis-thiophene] (P6)

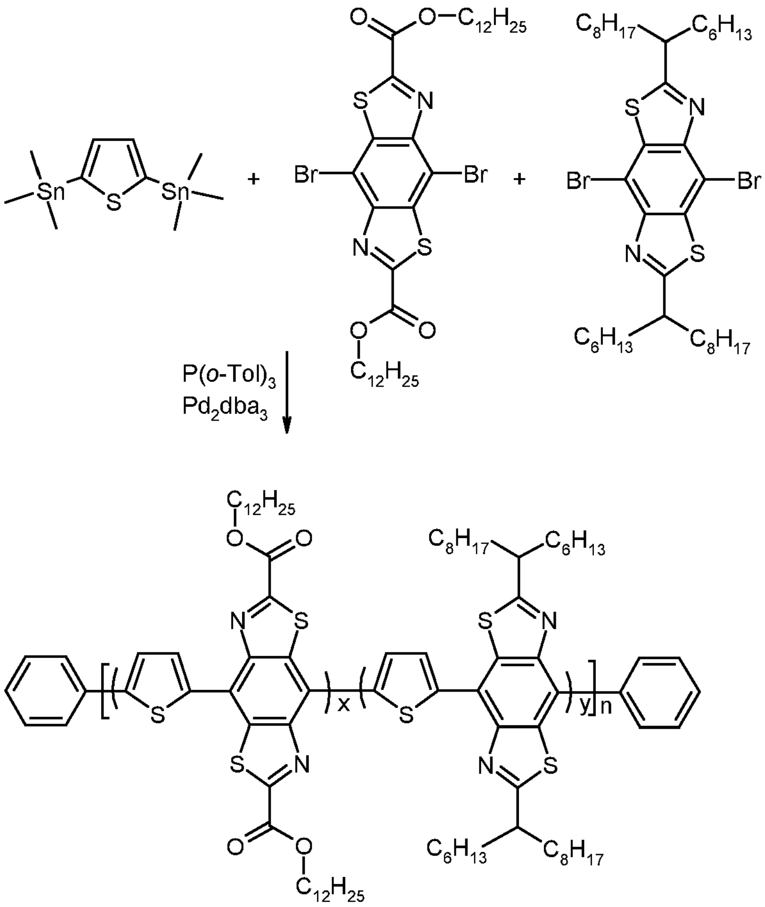


To a round bottomed flask is added 2,5-bis-trimethylstannanyl-thiophene (140.00 mg; 0.34 mmol; 1.00 eq.), 4,8-dibromo-benzo[1,2-d;4,5-d']bisthiazole-2,6-dicarboxylic acid didodecyl ester (132.35 mg; 0.17 mmol; 0.50 eq.), 4,8-dibromo-2,6-bis-(1-hexyl-nonyl)-benzo[1,2-d;4,5-d']bisthiazole (131.69 mg; 0.17 mmol; 0.50 eq.), tris(dibenzylideneacetone)dipalladium(0) (12 mg; 14 μmol; 0.04 eq.) and tri-o-tolyl-phosphine (17 mg; 55 μmol; 0.16 eq.). To the vessel is added degassed chlorobenzene (4.3 $cm^3$) and the reaction mixture is degassed further for 15 minutes. The reaction mixture is heated to 110 °C and stirred at this temperature for 1 hour. Tributyl-phenyl-stannane (501.79 mg; 1.37 mmol; 4.00 eq.) is then added followed by an additional 1 hour heating at 110 °C. Next, bromobenzene (321.87 mg; 2.05 mmol; 6.00 eq.) is added followed by an additional 1 hour heating at 110 °C. After completion of the reaction, the reaction mixture is allowed to cool to 60 °C and precipitated into methanol (100 $cm^3$). The polymer is sequentially extracted by Soxhlet extraction with acetone, petroleum ether (40-60 °C), cyclohexane, chloroform and chlorobenzene. The chloroform fraction is precipitated by addition to methanol (250 $cm^3$) and collected by filtration to give a dark blue solid (197.00 mg; 0.14 mmol; 82.9 %).

GPC (50 °C, chlorobenzene) $M_n$ = 32.6 kg $mol^{-1}$; $M_w$ = 135.2 kg $mol^{-1}$; PDI = 4.14.

Example P7

Polymer **P7** was prepared as follows.

Poly-[4,8-(2,6-bis-(1-hexyl-nonyl)-benzo[1,2-d;4,5-d']bisthiazole)-co-2,5-bis-thiophene-co-5-thiophen-2-yl-(4,8-bis-6-(2-octyl-dodecyl)-[1,2,5]thiadiazolo[3,4-e]isoindole-5,7-dione] (P7)

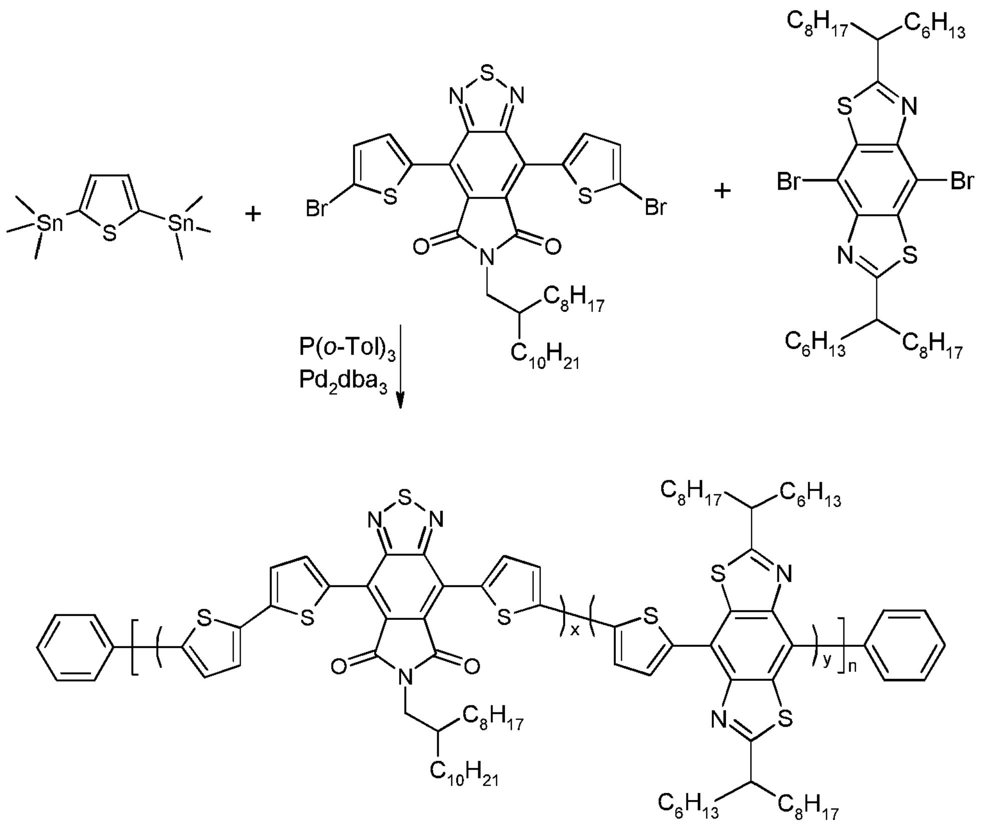


To a round bottomed flask is added 2,5-bis-trimethylstannanyl-thiophene (149.54 mg; 0.36 mmol; 2.00 eq.), 4,8-bis-(5-bromo-thiophen-2-yl)-6-(2-octyl-dodecyl)-[1,2,5]thiadiazolo[3,4-e]isoindole-5,7-dione (147.40 mg; 0.18 mmol; 1.00 eq.), 4,8-dibromo-2,6-bis-(1-hexyl-nonyl)-benzo[1,2-d;4,5-d']bisthiazole (140.66 mg; 0.18 mmol; 1.00 eq.), tris(dibenzylideneacetone)dipalladium(0) (6.68 mg; 7.30 μmol; 0.04 eq.) and tri-o-tolyl-phosphine (8.89 mg; 29.20 μmol; 0.16 eq.).To the vessel is added degassed chlorobenzene (5.00 $cm^3$) and the reaction mixture is degassed further for 15 minutes. The reaction mixture is heated to 110 °C and stirred at this temperature for 30 minutes. Tributyl-phenyl-stannane (0.3 $cm^3$) is then added followed by an additional 1 hour heating at 110 °C. Next, bromobenzene (0.2 $cm^3$) is added followed by an additional 1 hour heating at 110 °C. After completion of the reaction, the reaction mixture is allowed to cool to 60 °C and precipitated into methanol (100 $cm^3$). The material is sequentially extracted by Soxhlet extraction with acetone and petroleum ether (40-60 °C), cyclohexane, chloroform and chlorobenzene. The cyclohexane fraction is precipitated by addition to methanol (250 $cm^3$) and collected by filtration to give a dark solid (98 mg).

GPC (50 °C, chlorobenzene) $M_n$ = 21.4 kg $mol^{-1}$; $M_w$ = 41.1 kg $mol^{-1}$; PDI = 1.92.

The chloroform fraction is precipitated by addition to methanol (250 $cm^3$) and collected by filtration to give a dark solid (116 mg).

GPC (50 °C, chlorobenzene) $M_n$ = 28.6 kg $mol^{-1}$; $M_w$ = 64.7 kg $mol^{-1}$; PDI = 2.26.

Example P8

Polymer **P8** was prepared as follows

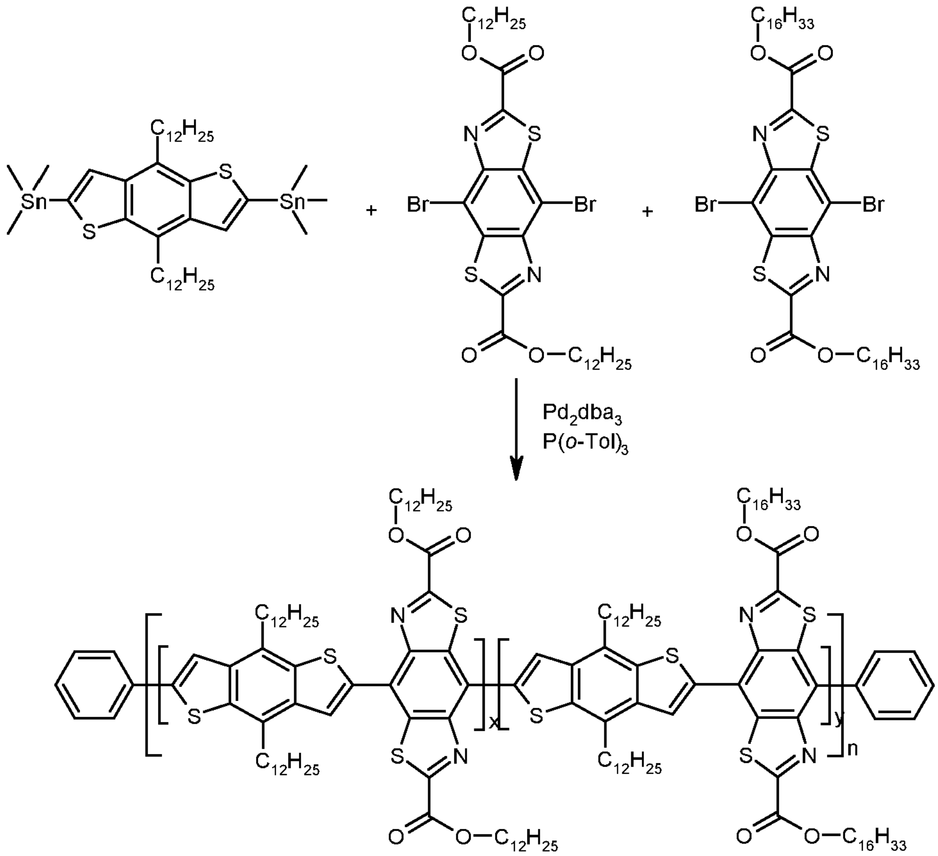


To a round bottomed flask is added 4,8-didodecyl-2,6-bis-trimethylstannanyl-benzo[1,2-b;4,5-b']dithiophene (426.27 mg; 0.50 mmol; 1.00 eq.), 4,8-dibromo-benzo[1,2-d;4,5-d']bisthiazole-2,6-dicarboxylic acid dihexadecyl ester (221.73 mg; 0.25 mmol; 0.50 eq.), 4,8-dibromo-benzo[1,2-d;4,5-d']bisthiazole-2,6-dicarboxylic acid didodecyl ester (193.68 mg; 0.25 mmol; 0.50 eq.), Pd2(dba)3 (18.31 mg; 20.00 μmol; 0.04 eq.) and tri-o-tolyl-phosphine (24.35 mg; 80.00 μmol; 0.16 eq.). To the vessel is added degassed chlorobenzene ( 6.25 $cm^3$) and the reaction mixture is degassed further for 15 minutes. The reaction mixture is heated to 110 °C and stirred at this temperature for 1 hours. Tributyl-phenyl-stannane (734.31 mg; 2.00 mmol; 4.00 eq.) is then added followed by an additional 1 hour heating at 130 °C. Next, bromo-benzene (471.02 mg; 3.00 mmol; 6.00 eq.) is added followed by an additional 1 hour heating at 130 °C. After completion of the reaction, the reaction mixture is allowed to cool to 60 °C and precipitated into stirred methanol (100 $cm^3$). The polymer is collected by filtration and washed with methanol (2 x 10 $cm^3$) to give a solid. The polymer is sequentially extracted by Soxhlet extraction with acetone, petroleum ether (40-60 °C), cyclohexane and chloroform. The cyclohexane fraction is precipitated by addition to methanol (100 $cm^3$) and collected by filtration to give a black solid (556.00 mg; 93.0 %).

GPC (50 °C, chlorobenzene): Mn= 43.6 kg/mol, Mw = 190.4 kg/mol, PDI=4.362

Example P9

Polymer **P9** was prepared as follows

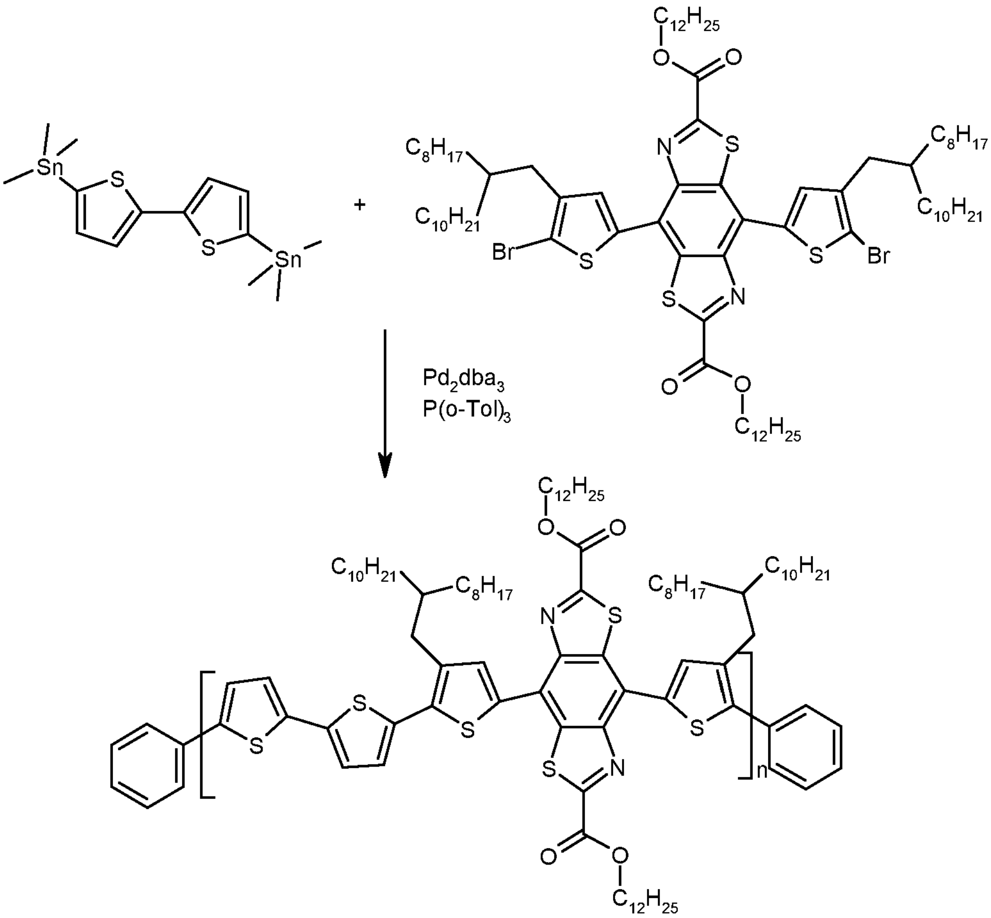


To a round bottomed flask is added 5,5'-bis-trimethylstannanyl-[2,2']bithiophenyl (140.00 mg; 0.28 mmol; 1.00 eq.), 4,8-bis-[5-bromo-4-(2-octyl-dodecyl)-thiophen-2-yl]-benzo[1,2-d;4,5-d']bisthiazole-2,6-dicarboxylic acid didodecyl ester (426.94 mg; 0.28 mmol; 1.00 eq.), tri-o-tolyl-phosphine (13.86 mg; 45.54 μmol; 0.16 eq.) and Pd2(dba)3 (10.43 mg; 11.38 μmol; 0.04 eq.). To the vessel is added degassed chlorobenzene (3.56 $cm^2$) and the reaction mixture is degassed further for 10 minutes. The reaction mixture is heated to 110 °C and stirred at this temperature for 60 minutes.

Tributyl-phenyl-stannane (0.3 $cm^3$) is then added followed by an additional 1 hour heating at 110 °C. Next, bromo-benzene (0.5 $cm^3$) is added followed by an additional overnight heating at 110 °C.

After completion of the reaction, the reaction mixture is transferred to a flask containing 150 $cm^3$ methanol. The polymer is collected by filtration and washed with methanol (2 x 10 $cm^3$) to give a solid. The polymer is sequentially extracted by Soxhlet extraction with acetone, petroleum ether (40-60 °C), cyclohexane and chloroform. The cyclohexane fraction is precipitated by addition to methanol (250 $cm^3$) and collected by filtration to give a black solid: (69.80 mg; 16.9 %).

GPC (50 °C, chlorobenzene) $M_n$ = 48.8 kg $mol^{-1}$; $M_w$ = 239.2 kg $mol^{-1}$; PDI = 4.9

<u>Example P10</u>

Polymer **P10** was prepared as follows

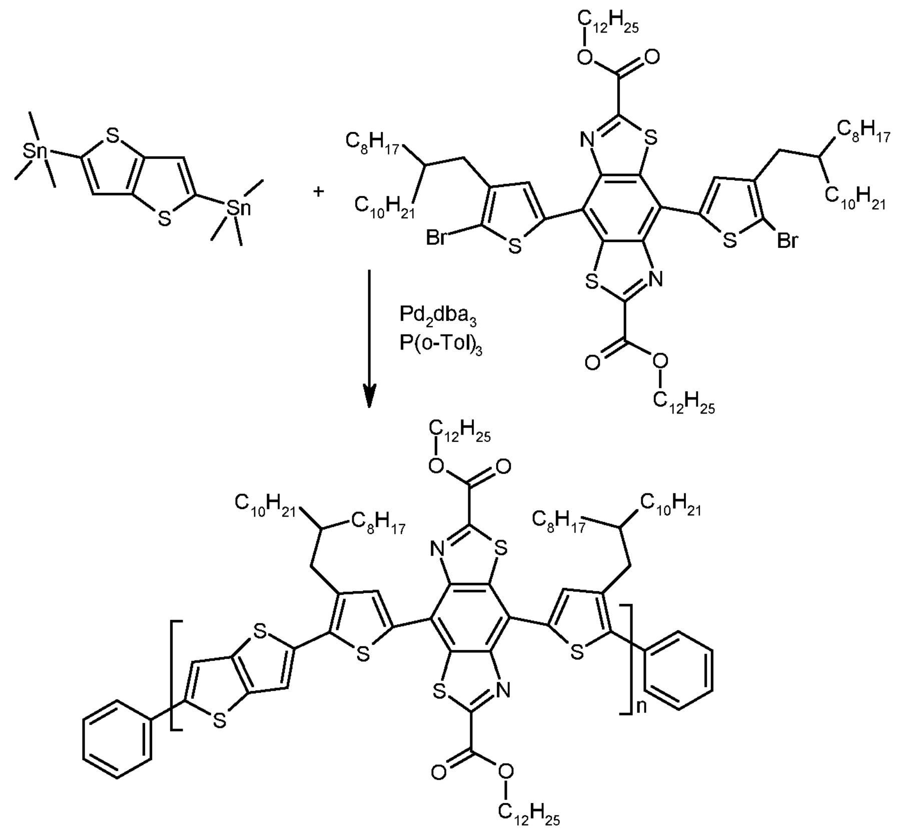


To a round bottomed flask is added 5,5'-bis-trimethylstannanyl-[2,2']bithiophenyl (140.00 mg; 0.28 mmol; 1.00 eq.), 4,8-bis-[5-bromo-4-(2-octyl-dodecyl)-thiophen-2-yl]-benzo[1,2-d;4,5-d']bisthiazole-2,6-dicarboxylic acid didodecyl ester (207.50 mg; 0.14 mmol; 1.00 eq.), tri-o-tolyl-phosphine (6.74 mg; 22.13 μmol; 0.16 eq.) and Pd2(dba)3 (5.07 mg; 5.53 μmol; 0.04 eq.). To the vessel is added degassed chlorobenzene (1.71 $cm^3$) and the reaction mixture is degassed further for 10 minutes. The reaction mixture is heated to 110 °C and stirred at this temperature for 60 minutes. Tributyl-phenyl-stannane (0.3 $cm^3$) is then added followed by an additional 1 hour heating at 110 °C. Next, bromo-benzene (0.5 $cm^3$) is added followed by an additional overnight heating at 110 °C.

After completion of the reaction, the reaction mixture is transferred to a flask containing 150 $cm^3$ methanol. The polymer is collected by filtration and washed with methanol (2 x 10 $cm^3$) to give a solid. The polymer is sequentially extracted by Soxhlet extraction with acetone, petroleum ether (40-60 °C), cyclohexane and chloroform and chlorobenzene. The chlorobenzene fraction is precipitated by addition to methanol (250 $cm^3$) and collected by filtration to give a black solid (27.00 mg; 13.7 %) GPC (50 °C, chlorobenzene) $M_n$ = 51.4 kg $mol^{-1}$; $M_w$ = 252.6 kg $mol^{-1}$; PDI = 4.91.

B) Use Examples

Bulk heteroiunction OPV devices for Polymers P1-P3 and P5-P9

Organic photovoltaic (OPV) devices are fabricated on pre-patterned ITO-glass substrates (13Ω/sq.) purchased from LUMTEC Corporation. Substrates were cleaned using common solvents (acetone, iso-propanol, deionized-water) in an ultrasonic bath. A conducting polymer poly(ethylene dioxythiophene) doped with poly(styrene sulfonic acid) [Clevios VPAI 4083 (H.C. Starck)] is mixed in a 1:1 ratio with deionized-water. This solution was filtered using a 0.45 μm filter before spin-coating to achieve a thickness of 20 nm. Substrates were exposed to ozone prior to the spin-coating process to ensure good wetting properties. Films were then annealed at 140 °C for 30 minutes in a nitrogen atmosphere where they were kept for the remainder of the process. Active material solutions (i.e. polymer + PCBM) were prepared and stirred overnight to fully dissolve the solutes. Thin films were either spin-coated or blade-coated in a nitrogen atmosphere to achieve active layer thicknesses between 100 and 500 nm as measured using a profilometer. A short drying period followed to ensure removal of any residual solvent.

Typically, spin-coated films were dried at 23 °C for 10 minutes and blade-coated films were dried at 70 °C for 2 minutes on a hotplate. For the last step of the device fabrication, Ca (30 nm) / Al (125 nm) cathodes were thermally evaporated through a shadow mask to define the cells. Current-voltage characteristics were measured using a Keithley

2400 SMU while the solar cells were illuminated by a Newport Solar Simulator at 100 mW.cm-2 white light. The Solar Simulator was equipped with AM1.5G filters. The illumination intensity was calibrated using a Si photodiode. All the device preparation and characterization is done in a dry-nitrogen atmosphere.

Power conversion efficiency is calculated using the following equation

$$\eta = \frac{V_{oc} \times J_{sc} \times FF}{P_{in}}$$

where FF is defined as

$$FF = \frac{V_{\max} \times J_{\max}}{V_{oc} \times J_{sc}}$$

OPV devices were prepared wherein the photoactive layer contains a blend of a polymer selected from one of the Examples P1-P3 and P5-P9 with the fullerene $PC_{60}BM$, which is coated from a solution in a solvent and at a total solid concentration as shown in Table 2 below. The OPV device characteristics are shown in Table 2.

Table 2. Photovoltaic cell characteristics.

| Polymer | Solvent | Voc/(mV) | Jsc/(mA/cm$^2$) | FF/% | PCE/% |
|---|---|---|---|---|---|
| P1 | DMA/DIO | 835 | 8.2 | 40 | 2.8 |
| P2 | o-xylene/DMA | 837 | 0.6 | 43 | 0.2 |
| P3 | DMA/DIO | 823 | 2.0 | 43 | 0.7 |
| P5 | ODCB | 800 | 1.0 | 31 | 0.3 |
| P6 | ODCB | 704 | 3.6 | 33 | 0.8 |
| P7 | DMA/DIO | 677 | 2.3 | 29 | 0.5 |
| P8 | DMA/DIO | 824 | 2.0 | 51 | 0.8 |
| P9 | ODCB | 826 | 1.2 | 33 | 0.3 |

The device data above shows that incorporation of the BBT unit into polymers does yield materials which can be used in OPV devices. The materials show high Vocs, which demonstrates that they have the potential to achieve high performances in OPV devices.

## Claims

**1.** A compound comprising one or more divalent heteroarylene units of formula I

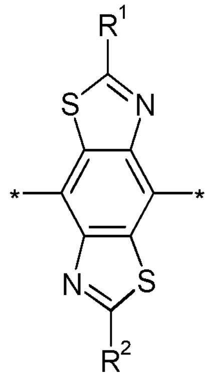

I

wherein the individual radicals, independently of each other and on each occurrence identically or differently, have the following meanings

$R^1$, $R^2$ are selected from the group consisting of -C(=O)-R,-C(=O)-OR, -OC(=O)-R, -C(=O)-NHR and -C(=O)-NRR$^n$, wherein R and R$^n$ are independently of each other straight-chain or branched alkyl with 1 to 20 C atoms, which is unsubstituted or substituted by one or more F atoms,
L F, Cl, -CN, -NC, -NCO, -NCS, -OCN, -SCN, -$R^0$, -O$R^0$, -S$R^0$,-C(=O)$X^0$, -C(=O)$R^0$, -C(=O)-O$R^0$, -O-C(=O)$R^0$, -$NH_2$, -NH$R^0$,-Nr$^0$R$^{00}$, -C(=O)NH$R^0$, -C(=O)N$R^0R^{00}$, -$SO_3$H, -$SO_2R^0$, -OH,-$NO_2$, -$CF_3$, -$SF_5$, or optionally substituted silyl, or carbyl or hydrocarbyl with 1 to 20 C atoms that is optionally substituted and optionally comprises one or more hetero atoms
R°, $R^{00}$ H or straight-chain or branched alkyl with 1 to 20 C atoms that is optionally fluorinated,

and further comprising one or more arylene or heteroarylene units that have from 5 to 20 ring atoms, are mono- or polycyclic, do optionally contain fused rings, are unsubstituted or substituted by one or more identical or different groups L, and are either selected of formula I or are structurally different from formula I, wherein all the aforementioned units are directly connected to each other,
and wherein the compound is a conjugated polymer.

2. The compound according to claim 1, wherein $R^1$ and $R^2$ in the units of formula I denote -C(=O)-OR, wherein R is straight-chain or branched alkyl with 1 to 20 C atoms that is optionally fluorinated.

3. The compound according to claim 1, wherein L denotes F or is selected from the following groups

- the group consisting of R, -OR and -SR wherein R is straight-chain or branched alkyl with 1 to 20 C atoms which is unsubstituted or substituted by one or more F atoms,
- the group consisting of -C(=O)-R, -C(=O)-OR, OC(=O)-R, -C(=O)-NHR and -C(=O)-NRR$^n$, wherein R and R$^n$ are independently of each other straight-chain or branched alkyl with 1 to 20 C atoms that is optionally fluorinated.

4. The compound according to any of claims 1 to 3, which is a conjugated polymer comprising one or more units of formula I as defined in any of claims 1 to 3, and further comprising one or more arylene or heteroarylene units that have from 5 to 20 ring atoms, are mono- or polycyclic, do optionally contain fused rings, are unsubstituted or substituted by one or more identical or different groups L, and are either selected of formula I or are structurally different from formula I, and wherein all the aforementioned units are directly connected to each other.

5. The compound according to claim 4, which comprises one or more repeating units of formula II1 or II2, and optionally one or more repeating units of formula II3:

$$-(Ar^1)_a-U-(Ar^2)_b-(Ar^3)_c-(Ar^4)_d- \qquad II1$$

$$-(Ar^1)_a-(Ar^2)_b-U-(Ar^3)_c-(Ar^4)_d- \qquad II2$$

$$-(Ar^1)_a-(Ar^2)_b-(Ar^3)_c-(Ar^4)_d- \qquad II3$$

wherein the individual radicals, independently of each other and on each occurrence identically or differently, have

the following meanings

U a unit of formula I as defined in any of claims 1 to 3,
$Ar^{1-4}$ arylene or heteroarylene that has 5 to 20 ring atoms, is mono- or polycyclic, does optionally contain fused rings, is unsubstituted or substituted by one or more identical or different groups L as defined in claim 1 or 3, and is different from formula I,
a, b, c, d 0 or 1, wherein in formula II3 a+b+c+d≥1.

**6.** The compound according to claim 4 or 5, which is selected of formula III:

$$*-\left[-(A)_x-(B)_y-\right]_n-* \qquad \text{III}$$

wherein

A is a unit of formula I, II1 or II2 as defined in any of claims 1 to 5,
B is a unit of formula I, II1, II2 or II3 as defined in any of claims 1 to 5,
x is > 0 and ≤ 1,
y is ≥ 0 and < 1,
x+y is 1, and
n is an integer ≥5.

**7.** The compound according to any of claims 4 to 6, which is selected from the following formulae

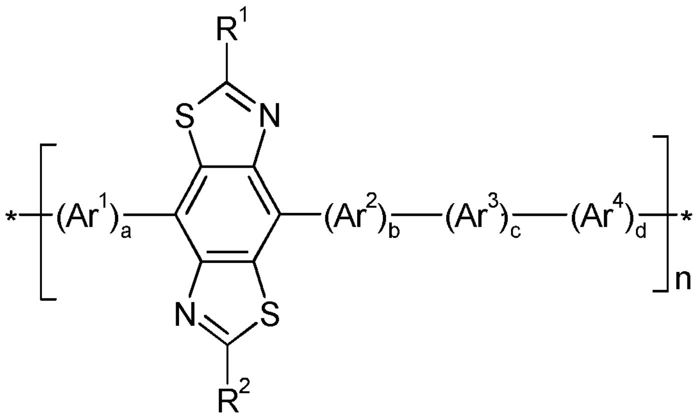

III1

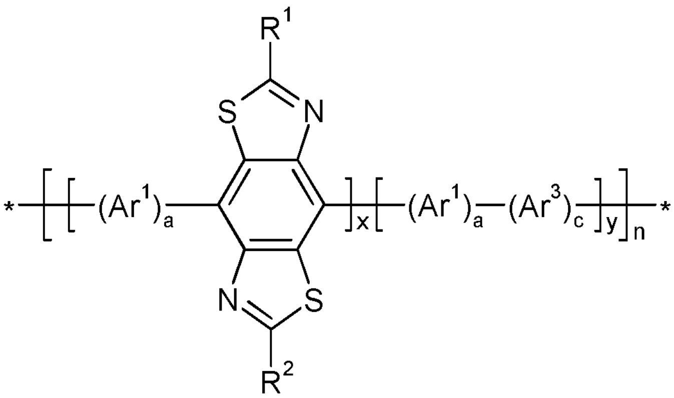

III2

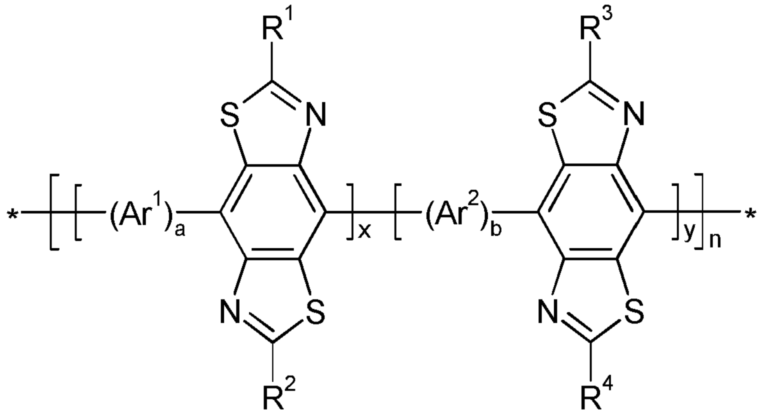

III3

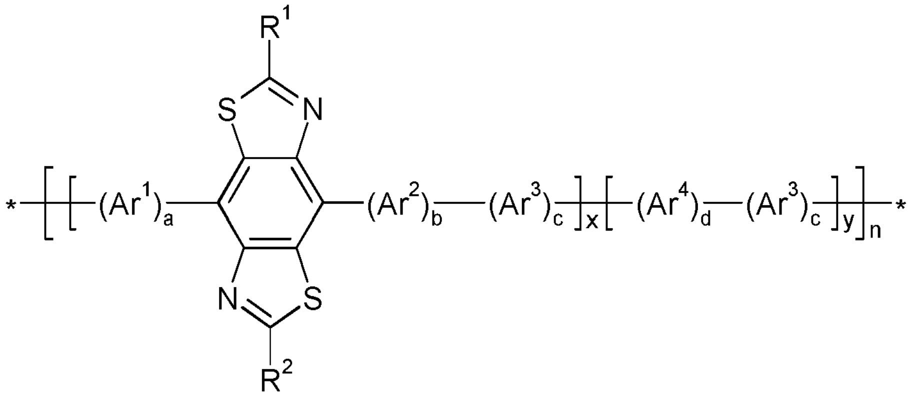

III4

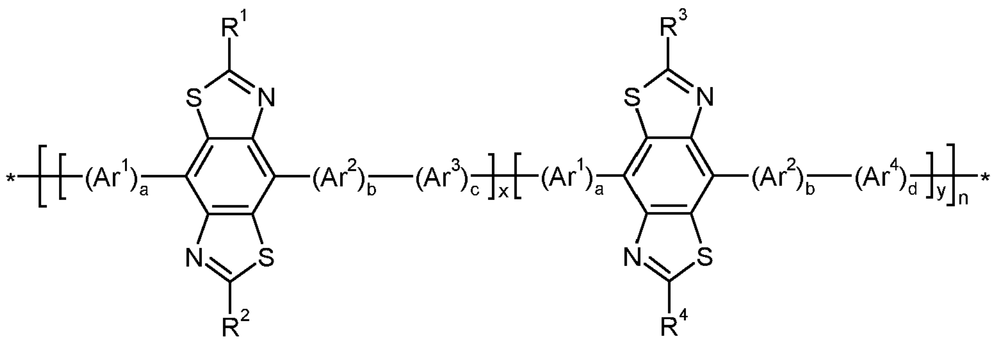

III5

wherein $R^1$ and $R^2$ have the meanings of any of claims 1 or 2, $R^3$ and $R^4$ have one of the meanings of $R^1$ as defined in claims 1 or 2, $Ar^1$, $Ar^2$, $Ar^3$, $Ar^4$, a, b, c and d have the meanings of claim 5, and x, y and n have the meanings of claim 6.

**8.** The compound according to claim 6 or 7, which is selected of formula IV

$$R^5\text{-chain-}R^6 \qquad \text{IV}$$

wherein "chain" denotes a polymer chain selected from formulae III and III1 -III5 as defined in claims 6 and 7, and $R^5$ and $R^6$ have independently of each other one of the meanings of L as defined in claim 1 or 3, or denote, independently of each other, H, F, Br, Cl, I, $-CH_2Cl$, -CHO, -CR'=CR"$_2$, -SiR'R"R'", -SiR'X'X", -SiR'R"X', -SnR'R"R'", -BR'R", -B(OR')(OR"), $-B(OH)_2$, $-O-SO_2-R'$, -C≡CH, -C≡C-SiR'$_3$, -ZnX' or an endcap group, X' and X" denote halogen, R', R" and R'" have independently of each other one of the meanings of $R^0$ given in claim 1, and two of R', R" and R'" may also form a cyclosilyl, cyclostannyl, cycloborane or cycloboronate group with 2 to 20 C atoms together with the respective hetero atom to which they are attached.

**9.** The compound according to any of claims 5 to 8, wherein one or more of $Ar^1$, $Ar^2$, $Ar^3$ and $Ar^4$ denote arylene or

heteroarylene selected from the group consisting of the following formulae

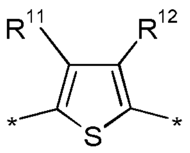
(D1)

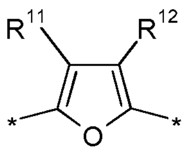
(D7)

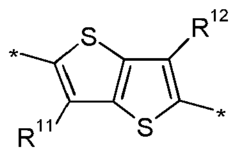
(D10)

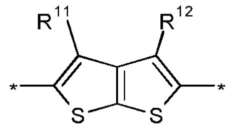
(D11)

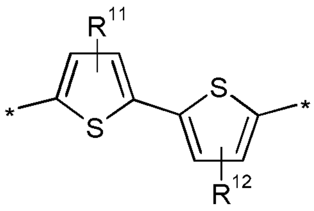
(D19)

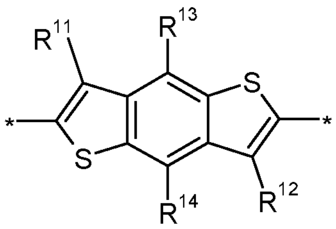
(D22)

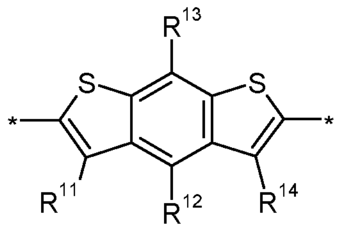
(D29)

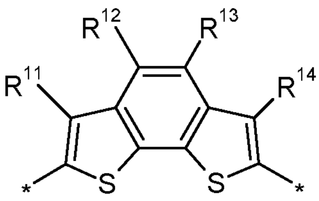
(D30)

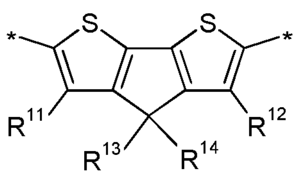
(D35)

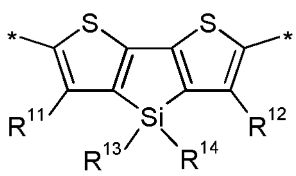
(D36)

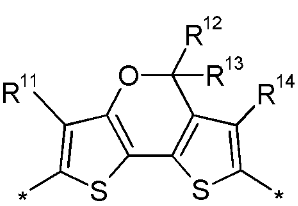
(D44)

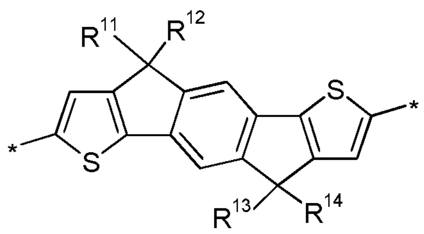
(D55)

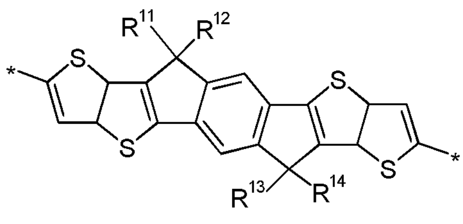
(D84)

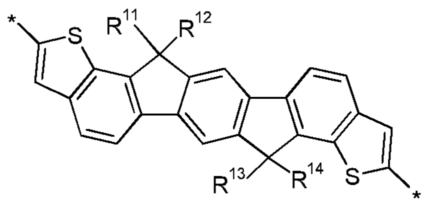
(D87)

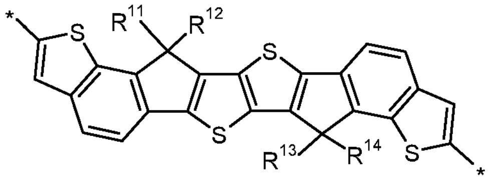

(D88)

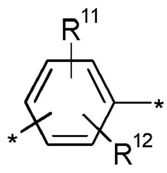

(D94)

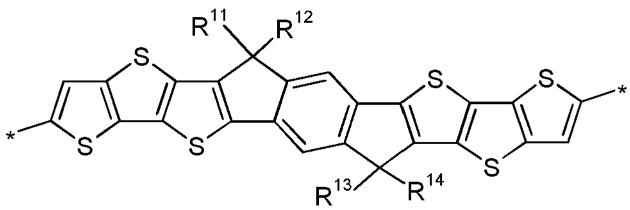

(D89)

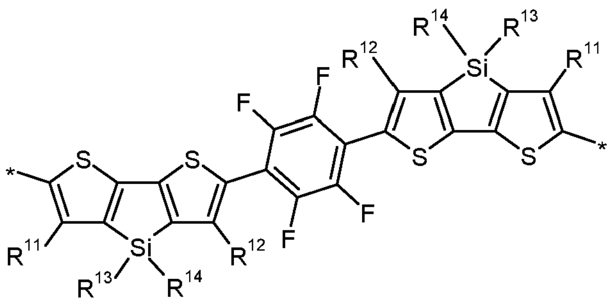

(D93)

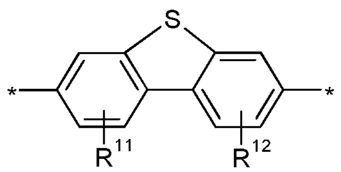

(D106)

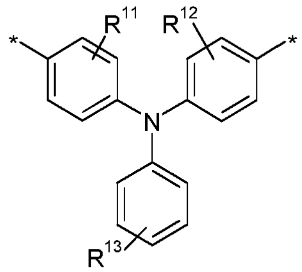

(D111)

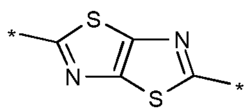

(D139)

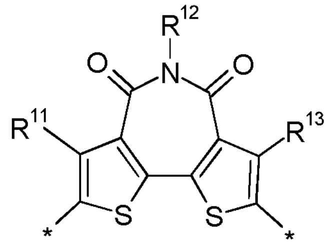

(D140)

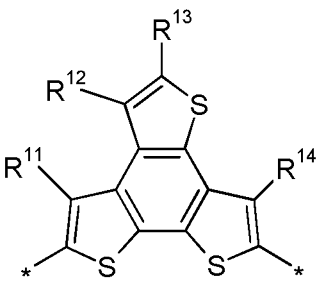

(D141)

wherein $R^{11}$, $R^{12}$, $R^{13}$, $R^{14}$ independently of each other denote H or have one of the meanings of L as defined in

claim 1 or 3.

**10.** The compound according to any of claims 5 to 9, wherein one or more of $Ar^1$, $Ar^2$, $Ar^3$ and $Ar^4$ denote arylene or heteroarylene selected from the group consisting of the following formulae

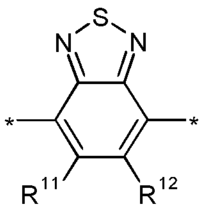
(A1)

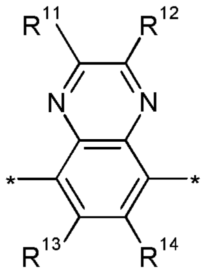
(A6)

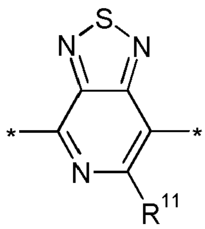
(A7)

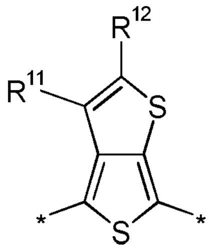
(A15)

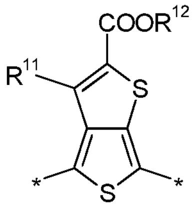
(A16)

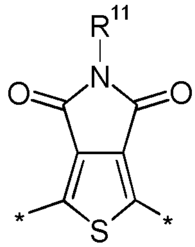
(A20)

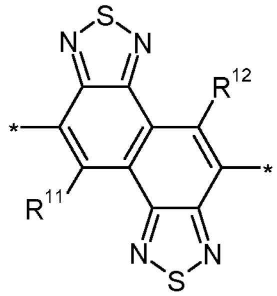
(A74)

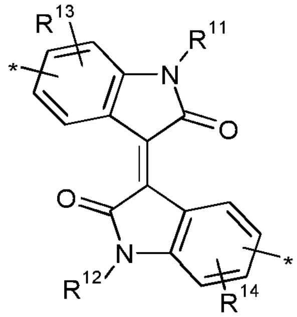
(A84)

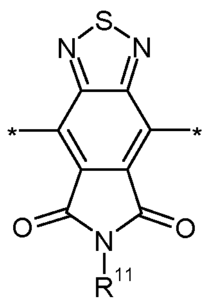
(A88)

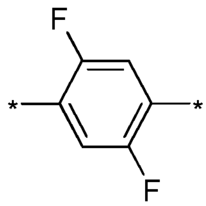
(A92)

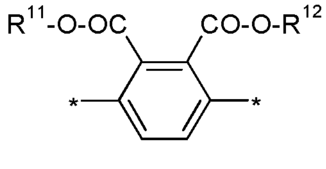
(A98)

wherein $R^{11}$, $R^{12}$, $R^{13}$, $R^{14}$ independently of each other denote H or have one of the meanings of L as defined in claim 1 or 3.

**11.** The compound according to any of claims 5 to 9, wherein one or more of $Ar^1$, $Ar^2$, $Ar^3$ and $Ar^4$ denote arylene or heteroarylene selected from the group consisting of the following formulae

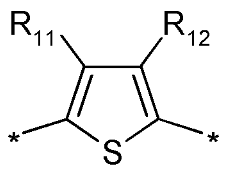

Sp1

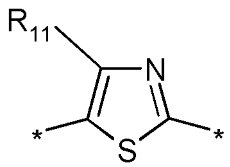

Sp2

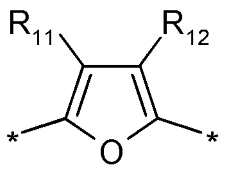

Sp3

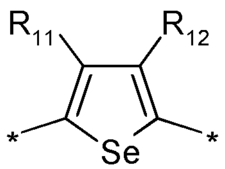

Sp4

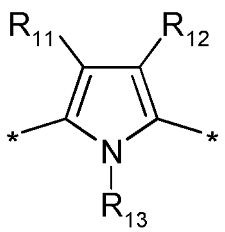

Sp5

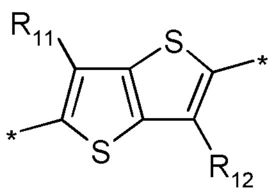

Sp6

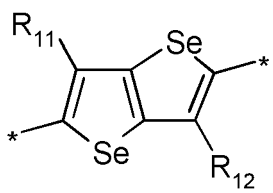

Sp7

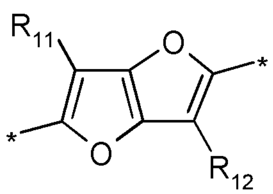

Sp8

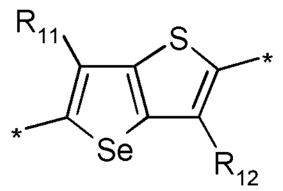

Sp9

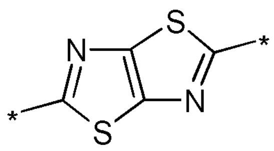

Sp10

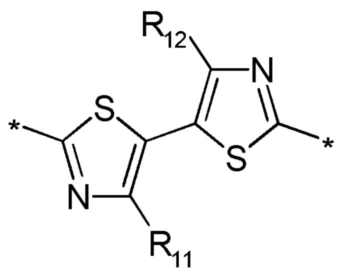

Sp11

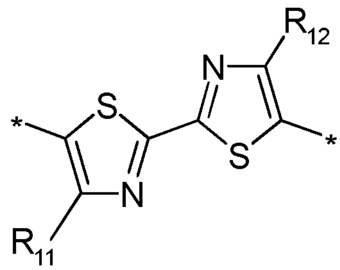

Sp12

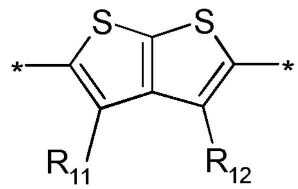

Sp13

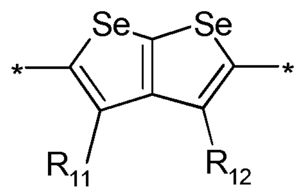

Sp14

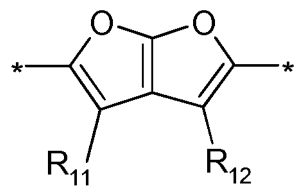

Sp15

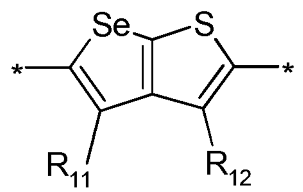

Sp16

wherein $R^{11}$ and $R^{12}$ independently of each other denote H or have one of the meanings of L as defined in claim 1 or 3.

**12.** The compound according to any of claims 5 to 11, wherein

a) one or more of $Ar^1$, $Ar^2$, $Ar^3$ and $Ar^4$ denote arylene or heteroarylene selected from the group consisting of the formulae D1, D7, D10, D11, D19, D22, D29, D30, D35, D36, D44, D55, D84, D87, D88, D89, D93, D94, D106, D111, D139, D140 and D141 as defined in claim 9, and/or
b) one or more of $Ar^1$, $Ar^2$, $Ar^3$ and $Ar^4$ denote arylene or heteroarylene selected from the group consisting of the formulae A1, A6, A7, A15, A16, A20, A74, A84, A88, A92 and A98 as defined in claim 10,
and
c) one or more of $Ar^1$, $Ar^2$, $Ar^3$ and $Ar^4$ denote arylene or heteroarylene selected from the group consisting of the formulae Sp1, Sp6, SP10 and Sp13 as defined in claim 11.

**13.** A compound of formula VI

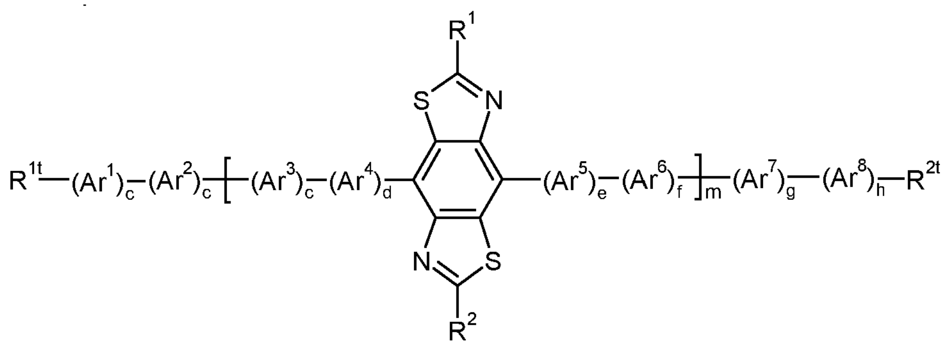

VI

wherein the individual radicals, independently of each other and on each occurrence identically or differently, have the following meanings

$Ar^{1-8}$ one of the meanings given for $Ar^1$ in claim 5, or a unit of formula I defined in claim 1, or $-CY^1=CY^2-$,
$Y^1$,$Y^2$ H, F, Cl or CN,
$R^{1t,2t}$ H, F, Cl, Br, -CN, $-CF_3$, R*, $-CF_2$-R*, -O-R*, -S-R*, $-SO_2$-R*-$SO_3$-R*, -C(=O)-R*, -C(=S)-R*, -C(=O)-$CF_2$-R*, -C(=O)-OR*, -C(=S)-OR*, -O-C(=O)-R*, -O-C(=S)-R*, -C(=O)-SR*, -S-C(=O)-R*, -C(=O)NR*R**, -NR*-C(=O)-R*, -NHR*,-NR*R**,-CR*=CR*R**, -C≡C-R*, -C≡C-SiR*R**R***, -SiR*R**R***,-CH=C(CN)-C(=O)-OR*, -CH=C(CO-OR*)$_2$, -CH=C(CO-NR*R**)$_2$, -CH=C(CN)($Ar^9$),

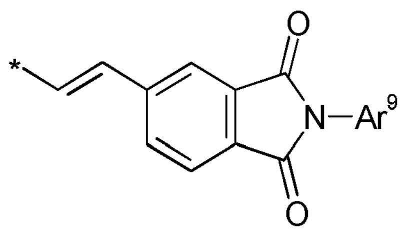

,

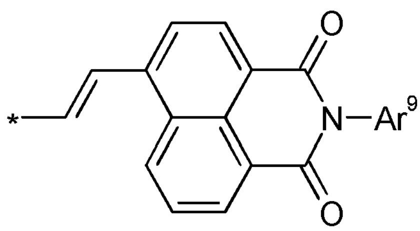

,

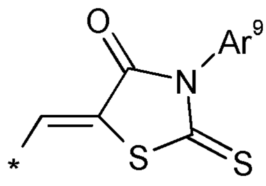 , 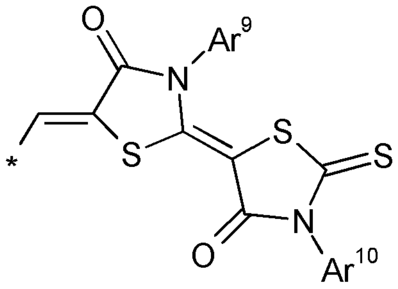 ,

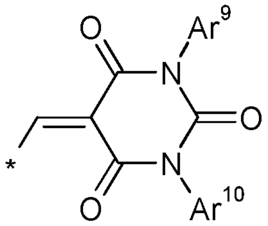 , 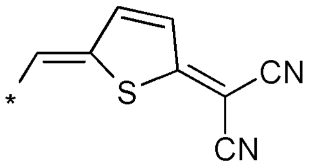 ,

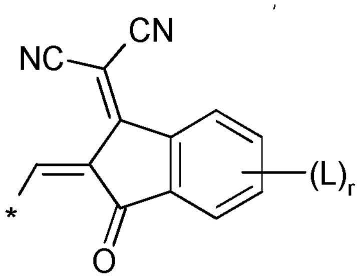 ,

$Ar^{9,10}$ aryl or heteroaryl, each having from 4 to 30 ring atoms, optionally containing fused rings and being unsubstituted or substituted with one or more groups L as defined in claim 1,
R* R**, R*** alkyl with 1 to 20 C atoms which is straight-chain, branched or cyclic, and is unsubstituted, substituted with one or more F or Cl atoms or CN groups, or perfluorinated, and in which one or more C atoms are optionally replaced by -O-,-S-, -C(O)-, -C(S)-, $-SiR^0R^{00}-$, $-NR^0R^{00}-$, $-CHR^0=CR^{00}-$ or-C≡C- such that O- and/or S-atoms are not directly linked to each other,
$R^0$, $R^{00}$ H or or straight-chain or branched alkyl with 1 to 20 C atoms that is optionally fluorinated,
a-h 0 or 1, with at least one of a-h being 1,
m 1, 2 or 3,

$R^1$ and $R^2$ are as defined in claim 1, with the proviso that m>1.

**14.** The compound of claim 13, wherein $Ar^{1-10}$ are selected from the following groups

a) the group consisting of the formulae D1, D7, D10, D11, D19, D22, D29, D30, D35, D36, D44, D55, D84, D87, D88, D89, D93, D94, D106, D111, D139, D140 and D141 as defined in claim 9,
b) the group consisting of the formulae A1, A6, A7, A15, A16, A20, A74, A84, A88, A92 and A98 as defined in claim 10,
c) the group consisting of the formulae Sp1, Sp6, Sp10 and Sp13 as defined in claim 11.

**15.** A mixture comprising one or more compounds according to any of claims 1 to 14 and one or more additional compounds having one or more of semiconducting, charge transport, hole or electron transport, hole or electron blocking, electrically conducting, photoconducting or light emitting properties.

**16.** A mixture comprising one or more compounds according to any of claims 1 to 14 and one or more n-type organic semiconductors.

17. The mixture of claim 16, wherein the n-type organic semiconductors are selected from fullerenes or substituted fullerenes.

18. A formulation comprising one or more compounds or mixtures according to any of claims 1 to 17, and further comprising one or more solvents selected from organic solvents.

19. An optical, electrooptical, electronic, electroluminescent or photoluminescent device, or a component thereof, or an assembly comprising it, which is prepared using a formulation according to claim 18.

20. Use of a compound or mixture according to any of claims 1 to 17 as semiconducting, charge transport, electrically conducting, photoconducting or light emitting material, or in an optical, electrooptical, electronic, electroluminescent or photoluminescent device, or in a component of such a device or in an assembly comprising such a device or component.

21. A semiconducting, charge transport, electrically conducting, photoconducting or light emitting material comprising a compound or mixture according to any of claims 1 to 17.

22. An optical, electrooptical, electronic, electroluminescent or photoluminescent device, or a component thereof, or an assembly comprising it, which comprises a compound or mixture according to any of claims 1 to 17, or comprises a semiconducting, charge transport, electrically conducting, photoconducting or light emitting material according to claim 21.

23. The optical, electrooptical, electronic, electroluminescent or photoluminescent device of claim 22, which is selected from organic field effect transistors (OFET), organic thin film transistors (OTFT), organic light emitting diodes (OLED), organic light emitting transistors (OLET), organic photovoltaic devices (OPV), organic photodetectors (OPD), organic solar cells, dye-sensitized solar cells (DSSC), perovskite-based solar cells, laser diodes, Schottky diodes, photoconductors and photodetectors.

24. The component of claim 22, which is selected from charge injection layers, charge transport layers, interlayers, planarising layers, antistatic films, polymer electrolyte membranes (PEM), conducting substrates and conducting patterns.

25. The assembly of claim 22, which is selected from integrated circuits (IC), radio frequency identification (RFID) tags or security markings or security devices containing them, flat panel displays or backlights thereof, electrophotographic devices, electrophotographic recording devices, organic memory devices, sensor devices, biosensors and biochips.

26. A bulk heterojunction which comprises, or is being formed from, a mixture according to any of claims 15 to 17.

27. A bulk heterojunction (BHJ) OPV device or inverted BHJ OPV device, comprising a bulk heterojunction of claim 26.

**Patentansprüche**

1. Verbindung, umfassend eine oder mehrere zweiwertige Heteroaryleneinheiten der Formel I

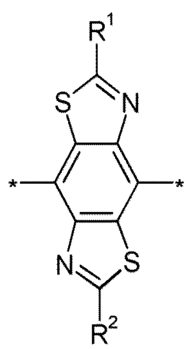

I

wobei die einzelnen Radikale unabhängig voneinander und bei jedem Auftreten identisch oder unterschiedlich

die folgenden Bedeutungen aufweisen

$R^1$, $R^2$ sind ausgewählt aus der Gruppe, bestehend aus -C(=O)-R, -C(=O)-OR, -OC-(=O)-R, -C(=O)-NHR und -C(=O)-NRR$^n$, wobei R und R$^n$ unabhängig voneinander geradkettiges oder verzweigtes Alkyl mit 1 bis 20 C-Atomen sind, das unsubstituiert oder mit einem oder mehreren F-Atomen substituiert ist,

L ist F, Cl, -CN, -NC, -NCO, -NCS, -OCN, -SCN, -$R^0$, -$OR^0$, -$SR^0$, -C(=O)$X^0$, -C(=O)-$R^0$, - C(=O)-$OR^0$, -O-C(=O)$R^0$, -$NH_2$, -$NHR^0$, $NR^0R^{00}$, -C(=O)$NHR^0$, -C(=O)$NR^0R^{00}$, -$SO_3H$, -$SO_2R^0$, - OH, -$NO_2$, -$CF_3$, -$SF_5$ oder wahlweise substituiertes Silyl oder Carbyl oder Hydrocarbyl mit 1 bis 20 C-Atomen, das wahlweise unsubstituiert ist oder wahlweise ein oder mehrere Heteroatome umfasst,

$R^0$, $R^{00}$ sind H oder geradkettiges oder verzweigtes Alkyl mit 1 bis 20 C-Atomen, das wahlweise fluoriert ist,

und weiter umfassend eine oder mehrere Arylen- oder Heteroaryleneinheiten, die 5 bis 20 Ringatome aufweisen, mono- oder polyzyklisch sind, wahlweise kondensierte Ringe enthalten, unsubstituiert oder mit einer oder mehreren identischen oder unterschiedlichen L-Gruppen substituiert sind und entweder aus Formel I ausgewählt sind oder strukturell verschieden von Formel I sind, wobei alle zuvor genannten Einheiten direkt miteinander verbunden sind,

und wobei die Verbindung ein konjugiertes Polymer ist.

**2.** Verbindung nach Anspruch 1, wobei $R^1$ und $R^2$ in den Einheiten der Formel I -C(=O)-OR bezeichnen, wobei R geradkettiges oder verzweigtes Alkyl mit 1 bis 20 C-Atomen ist, das wahlweise fluoriert ist.

**3.** Verbindung nach Anspruch 1, wobei L F bezeichnet oder aus den folgenden Gruppen ausgewählt ist

- der Gruppe, bestehend aus R, -OR und -SR, wobei R geradkettiges oder verzweigtes Alkyl mit 1 bis 20 C-Atomen ist, das unsubstituiert oder mit einem oder mehreren F-Atomen substituiert ist,
- der Gruppe, bestehend aus -C(=O)-R, -C(=O)-OR, -OC(=O)-R, -C(=O)-NHR und -C(=O)-NRR$^n$, wobei R und R$^n$ unabhängig voneinander geradkettiges oder verzweigtes Alkyl mit 1 bis 20 C-Atomen sind, das wahlweise fluoriert ist.

**4.** Verbindung nach einem der Ansprüche 1 bis 3, die ein konjugiertes Polymer ist, das eine oder mehrere Einheiten der Formel I wie in einem der Ansprüche 1 bis 3 definiert umfasst und weiter eine oder mehrere Arylen- und Heteroaryleneinheiten umfasst, die die 5 bis 20 Ringatome aufweisen, mono- oder polyzyklisch sind, wahlweise kondensierte Ringe enthalten, unsubstituiert oder mit einer oder mehreren identischen oder unterschiedlichen L-Gruppen substituiert sind und entweder aus Formel I ausgewählt sind oder strukturell verschieden von Formel I sind, wobei alle zuvor genannten Einheiten direkt miteinander verbunden sind.

**5.** Verbindung nach Anspruch 4, die eine oder mehrere Wiederholungseinheiten der Formel II1 oder II2 und wahlweise eine oder mehrere Wiederholungseinheiten der Formel II3 umfasst:

$$-(Ar^1)_a-U-(Ar^2)_b-(Ar^3)_c-(Ar^4)_d- \qquad 111$$

$$-(Ar^1)_a-(Ar^2)_b-U-(Ar^3)_c-(Ar^4)_d- \qquad II2$$

$$-(Ar^1)_a-(Ar^2)_b-(Ar^3)_c-(Ar^4)_d- \qquad II3$$

wobei die einzelnen Radikale unabhängig voneinander und bei jedem Auftreten identisch oder unterschiedlich die folgenden Bedeutungen aufweisen

U ist eine Einheit der Formel I wie in einem der Ansprüche 1 bis 3 definiert,

$Ar^{1-4}$ sind Arylen oder Heteroarylen, das 5 bis 20 Ringatome aufweist, mono- oder polyzyklisch ist, wahlweise kondensierte Ringe enthält, unsubstituiert oder mit einer oder mehreren identischen oder unterschiedlichen L-Gruppen wie in Anspruch 1 oder 3 definiert substituiert ist und verschieden von Formel I ist,

a, b, c, d sind 0 oder 1, wobei in Formel II3 $a+b+c+d \geq 1$ sind.

**6.** Verbindung nach Anspruch 4 oder 5, die ausgewählt ist aus Formel III:

$$*\!-\!\left[-(A)_x-(B)_y-\right]_n\!-\!* \qquad III$$

wobei

A eine Einheit der Formel I, II1 oder II2 wie in einem der Ansprüche 1 bis 5 definiert ist,
B eine Einheit der Formel I, II1, II2 oder II3 wie in einem der Ansprüche 1 bis 5 definiert ist,
$x > 0$ und $\leq 1$ ist,
$y \geq 0$ und $< 1$ ist,
x+y 1 sind und
n eine ganze Zahl $\geq 5$ ist.

**7.** Verbindung nach einem der Ansprüche 4 bis 6, die aus den folgenden Formeln ausgewählt ist

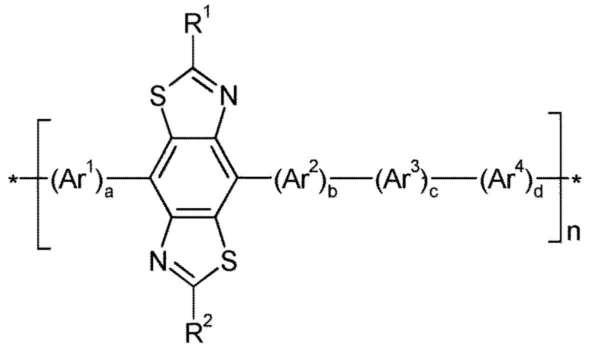

III1

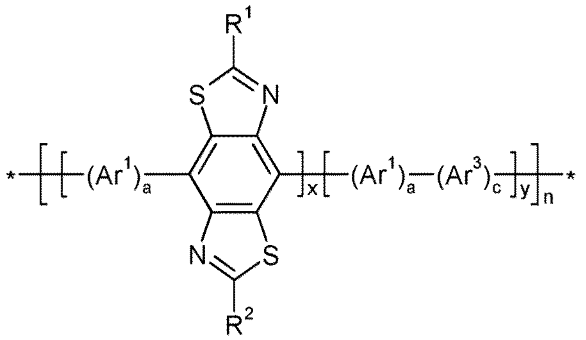

III2

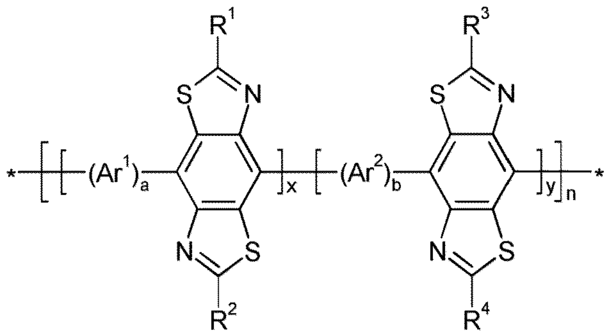

III3

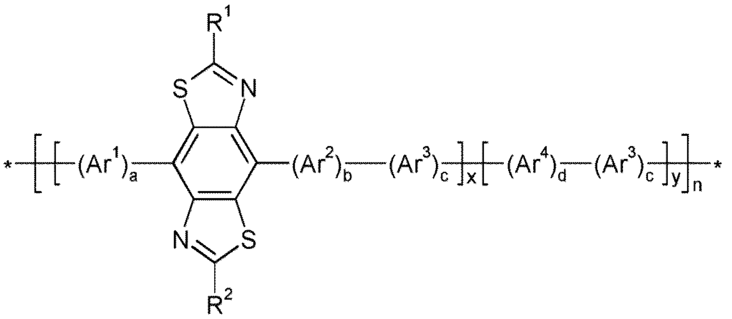

III4

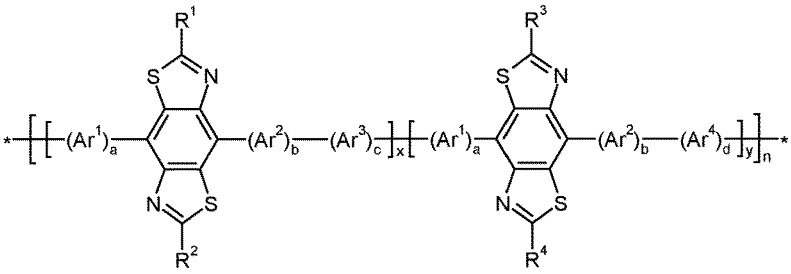

III5

wobei $R^1$ und $R^2$ die Bedeutungen nach einem der Ansprüche 1 oder 2 aufweisen, $R^3$ und $R^4$ eine der Bedeutungen von $R^1$ wie in Anspruch 1 oder 2 definiert aufweisen, $Ar^1$, $Ar^2$, $Ar^3$, $Ar^4$, a, b, c und d die Bedeutungen nach Anspruch 5 aufweisen und x, y und n die Bedeutungen nach Anspruch 6 aufweisen.

**8.** Verbindung nach Anspruch 6 oder 7, die aus der Formel IV ausgewählt ist

$R^5$-Kette-$R^6$ IV

wobei "Kette" eine Polymerkette bezeichnet, die aus den Formeln III und III1-III5 wie in den Ansprüchen 6 und 7 definiert ausgewählt ist, und $R^5$ und $R^6$ unabhängig voneinander eine der Bedeutungen von L wie in Anspruch 1 oder 3 definiert aufweisen oder unabhängig voneinander H, F, Br, Cl, I, $-CH_2Cl$, -CHO, -CR'=CR"$_2$, -SiR'R"R‴, -SiR'X'X", -SiR'R"X', -SnR'R"R‴, -BR'R", -B(OR')(OR"), $-B(OH)_2$, $-O-SO_2-R'$, -C≡CH, -C≡C-SiR'$_3$, -ZnX' oder eine Endkappengruppe bezeichnen, X' und X" Halogen bezeichnen, R', R"und R‴ unabhängig voneinander eine der Bedeutungen von $R^0$ nach Anspruch 1 aufweisen und zwei von R', R" und R‴ außerdem eine Cyclosilyl-, Cyclostannyl-, Cycloboran- oder Cycloboranatgruppe mit 2 bis 20 C-Atomen zusammen mit dem jeweiligen Heteroatom, an dem sie hängen, bilden können.

**9.** Verbindung nach einem der Ansprüche 5 bis 8, wobei eines oder mehrere von $Ar^1$, $Ar^2$, $Ar^3$ und $Ar^4$ Arylen oder Heteroarylen bezeichnen, ausgewählt aus der Gruppe, bestehend aus den folgenden Formeln

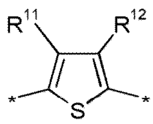

(D1)

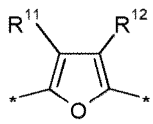

(D7)

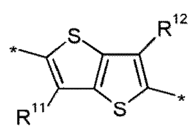

(D10)

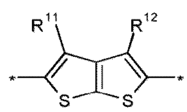

(D11)

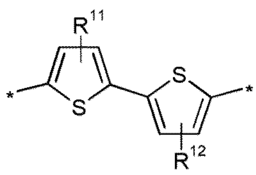

(D19)

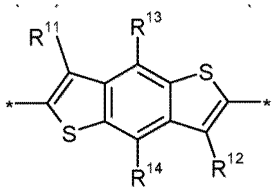

(D22)

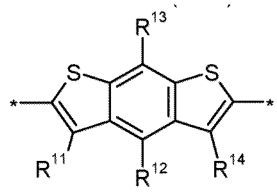

(D29)

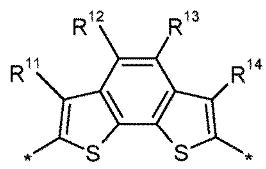

(D30)

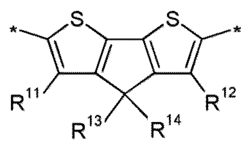

(D35)

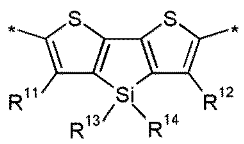

(D36)

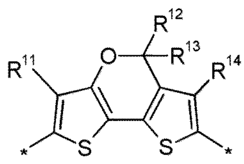

(D44)

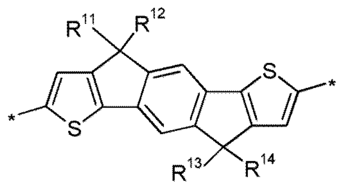

(D55)

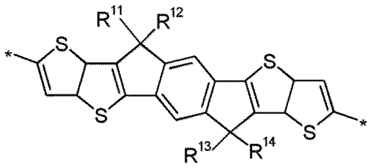

(D84)

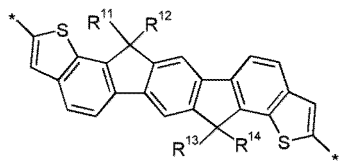

(D87)

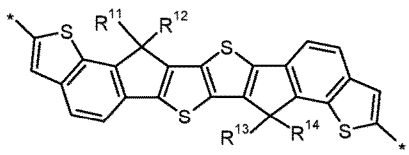

(D88)

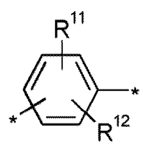

(D94)

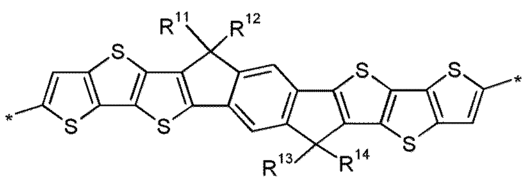

(D89)

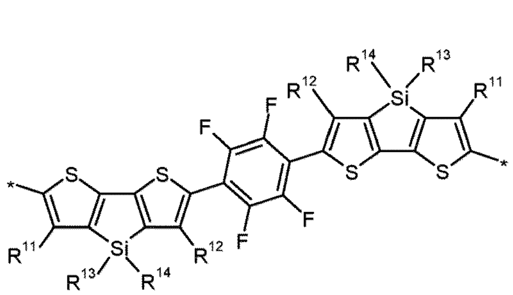

(D93)

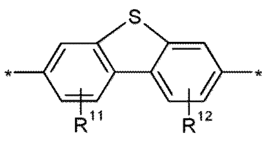 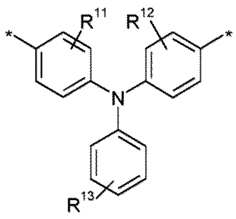 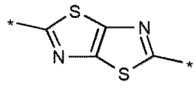

(D106) (D111) (D139)

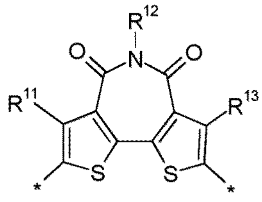 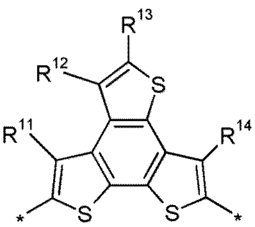

(D140) (D141)

wobei $R^{11}$, $R^{12}$, $R^{13}$, $R^{14}$ unabhängig voneinander H bezeichnen oder eine der Bedeutungen von L wie in Anspruch 1 oder 3 definiert aufweisen.

**10.** Verbindung nach einem der Ansprüche 5 bis 9, wobei eines oder mehrere von $Ar^1$, $Ar^2$, $Ar^3$ und $Ar^4$ Arylen oder Heteroarylen bezeichnen, ausgewählt aus der Gruppe, bestehend aus den folgenden Formeln

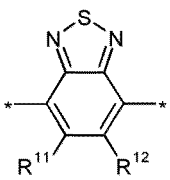 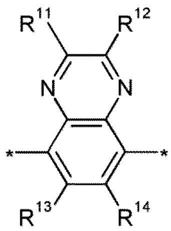 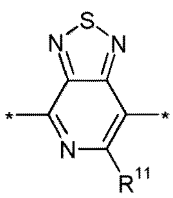 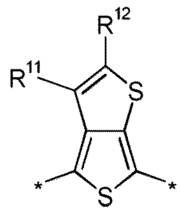

(A1) (A6) (A7) (A15)

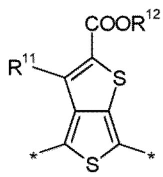 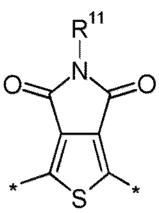 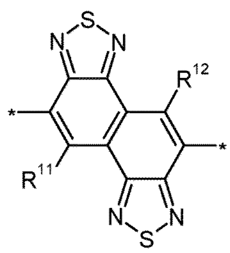

(A16) (A20) (A74)

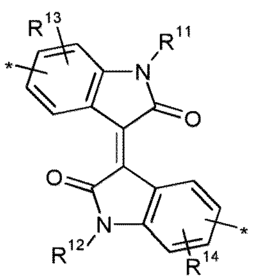 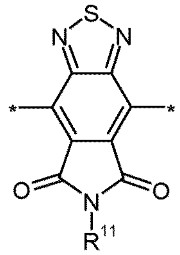 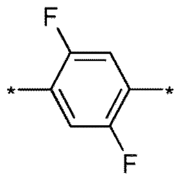

(A84) (A88) (A92)

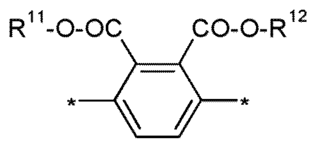

(A98)

wobei $R^{11}$, $R^{12}$, $R^{13}$, $R^{14}$ unabhängig voneinander H bezeichnen oder eine der Bedeutungen von L wie in Anspruch 1 oder 3 definiert aufweisen.

**11.** Verbindung nach einem der Ansprüche 5 bis 9, wobei eines oder mehrere von $Ar^1$, $Ar^2$, $Ar^3$ und $Ar^4$ Arylen oder Heteroarylen bezeichnen, ausgewählt aus der Gruppe, bestehend aus den folgenden Formeln

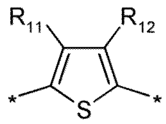

Sp1

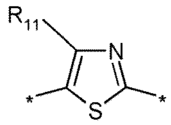

Sp2

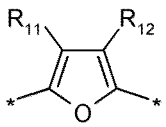

Sp3

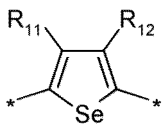

Sp4

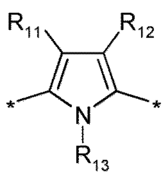

Sp5

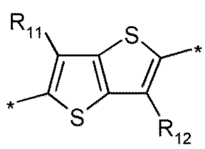

Sp6

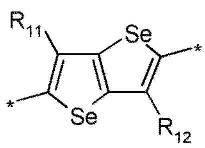

Sp7

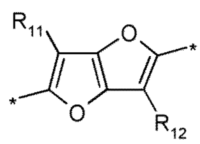

Sp8

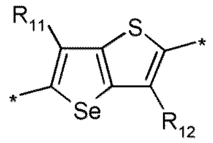

Sp9

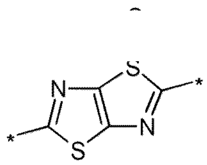

Sp10

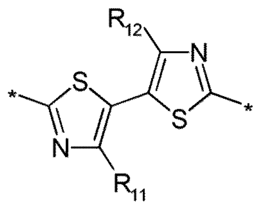

Sp11

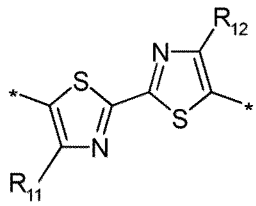

Sp12

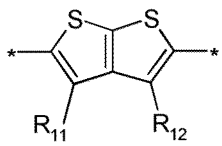

Sp13

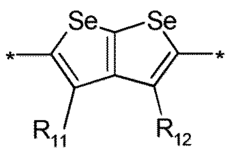

Sp14

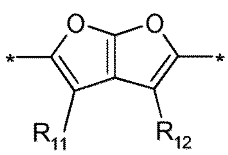

Sp15

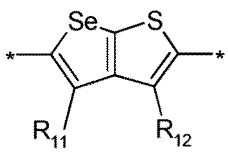

Sp16

wobei $R^{11}$ und $R^{12}$ unabhängig voneinander H bezeichnen oder eine der Bedeutungen von L wie in Anspruch 1 oder 3 definiert aufweisen.

**12.** Verbindung nach einem der Ansprüche 5 bis 11, wobei

a) ein oder mehrere von $Ar^1$, $Ar^2$, $Ar^3$ und $Ar^4$ Arylen oder Heteroarylen bezeichnen, ausgewählt aus der Gruppe, bestehend aus den Formeln D1, D7, D10, D11, D19, D22, D29, D30, D35, D36, D44, D55, D84, D87, D88, D89, D93, D94, D106, D111, D139, D140 und D141 wie in Anspruch 9 definiert, und/oder

b) ein oder mehrere von $Ar^1$, $Ar^2$, $Ar^3$ und $Ar^4$ Arylen oder Heteroarylen bezeichnen, ausgewählt aus der Gruppe, bestehend aus den Formeln A1, A6, A7, A15, A16, A20, A74, A84, A88, A92 und A98 wie in Anspruch 10 definiert, und

c) ein oder mehrere von $Ar^1$, $Ar^2$, $Ar^3$ und $Ar^4$ Arylen oder Heteroarylen bezeichnen, ausgewählt aus der Gruppe, bestehend aus den Formeln Sp1, Sp6, Sp10 und Sp13 wie in Anspruch 11 definiert.

**13.** Verbindung der Formel VI

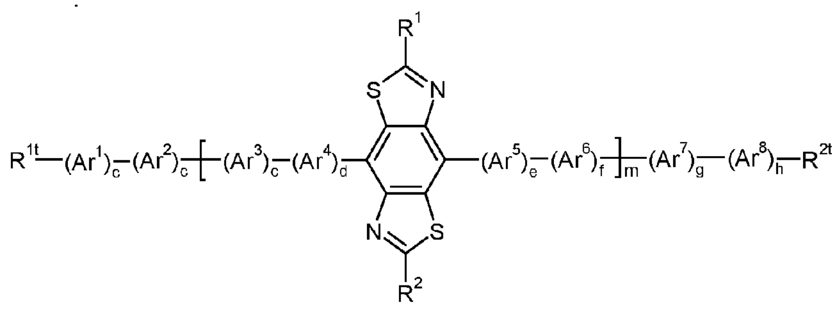

VI

wobei die einzelnen Radikale unabhängig voneinander und bei jedem Auftreten identisch oder unterschiedlich die folgenden Bedeutungen aufweisen

$Ar^{1-8}$ weisen eine der Bedeutungen von $Ar^1$ in Anspruch 5 auf oder sind eine Einheit der Formel I wie in Anspruch 1 definiert oder sind $-CY^1=CY^2-$,

$Y^1$, $Y^2$ sind H, F, Cl oder CN,

$R^{1t,2t}$ sind H, F, Cl, Br, -CN, $-CF_3$, R*, $-CF_2-R^*$, -O-R*, -S-R*, $-SO_2R^*$, $-SO_3-R^*$, -C(=O)-R*, -C(=S)-R*, $-C(=O)-CF_2R^*$, -C(=O)-OR*, -C(=S)-OR*, -O-C(=O)-R*, -O-C(=S)-R*, -C(=O)-SR*, -S-C(=O)-R*, C(=O)NR*R**, -NR*-C(=O)-R*, -NHR*, -NR*R**, CR*=CR*R**, -C≡C-R*, -C≡C-SiR*R**R***, -SiR*R**R***, -CH=C(CN)-C(=O)-OR*, $-CH=C(CO-OR^*)_2$, $-CH=C(CO-NR^*R^{**})_2$, $-CH=C(CN)(Ar^9)$,

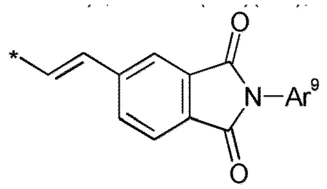

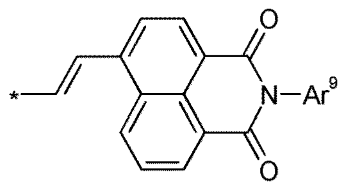

,

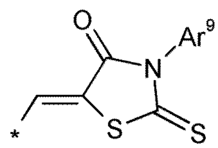

,

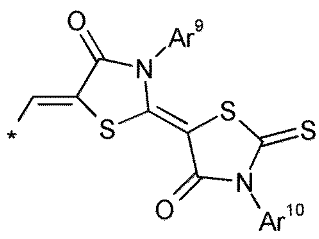

,

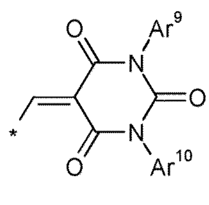

.

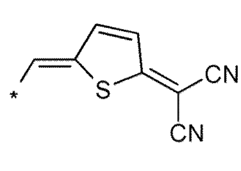

.

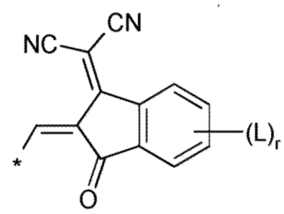

,

$Ar^{9,10}$ sind Aryl oder Heteroaryl, das jeweils 4 bis 30 Ringatome aufweist, wahlweise kondensierte Ringe enthält und unsubstituiert ist oder mit einer oder mehreren L-Gruppen wie in Anspruch 1 definiert substituiert ist,
$R^*$, $R^{**}$, $R^{***}$ sind Alkyl mit 1 bis 20 C-Atomen, das geradkettig, verzweigt oder zyklisch und unsubstituiert ist, mit einem oder mehreren F- oder Cl-Atomen oder CN-Gruppen substituiert oder perfluoriert ist und bei dem ein oder mehrere C-Atome wahlweise durch -O-, -S-, -C(O)-, -C(S)-, $-SiR^0R^{00}-$, $-NR^0R^{00}-$, $-CHR^0=CR^{00}-$ oder -C≡C- ersetzt sind, sodass O- und/oder S-Atome nicht direkt miteinander verbunden sind,
$R^0$, $R^{00}$ sind H oder geradkettiges oder verzweigtes Alkyl mit 1 bis 20 C-Atomen, das wahlweise fluoriert ist,
a-h sind 0 oder 1, wobei mindestens eines von a-h 1 ist,
m ist 1, 2 oder 3,
$R^1$ und $R^2$ sind wie in Anspruch 1 definiert,

mit der Maßgabe, dass m>1 ist.

**14.** Verbindung nach Anspruch 13, wobei $Ar^{1-10}$ aus den folgenden Gruppen ausgewählt sind

a) der Gruppe, bestehend aus den Formeln D1, D7, D10, D11, D19, D22, D29, D30, D35, D36, D44, D55, D84, D87, D88, D89, D93, D94, D106, D111, D139, D140 und D141 wie in Anspruch 9 definiert,
b) der Gruppe, bestehend aus den Formeln A1, A6, A7, A15, A16, A20, A74, A84, A88, A92 und A98 wie in Anspruch 10 definiert,
c) der Gruppe, bestehend aus den Formeln Sp1, Sp6, Sp10 und Sp13 wie in Anspruch 11 definiert.

**15.** Mischung, umfassend eine oder mehrere Verbindungen nach einem der Ansprüche 1 bis 14 und eine oder mehrere zusätzliche Verbindungen, die eine oder mehrere von halbleitenden, Ladungstransport-, Loch- oder Elektronentransport-, Loch- oder Elektronenblockade-, elektrisch leitenden, lichtleitenden oder lichtemittierenden Eigenschaften aufweisen.

**16.** Mischung, umfassend eine oder mehrere Verbindungen nach einem der Ansprüche 1 bis 14 und einen oder mehrere organische Halbleiter vom n-Typ.

**17.** Mischung nach Anspruch 16, wobei die organischen Halbleiter vom n-Typ aus Fullerenen oder substituierten Fullerenen ausgewählt sind.

**18.** Formulierung, umfassend eine oder mehrere Verbindungen oder Mischungen nach einem der Ansprüche 1 bis 17 und weiter umfassend ein oder mehrere Lösemittel, ausgewählt aus organischen Lösemitteln.

**19.** Optische, elektrooptische, elektronische, elektrolumineszierende oder fotolumineszierende Vorrichtung oder Bauteil davon oder sie umfassende Baugruppe, die unter Verwendung einer Formulierung nach Anspruch 18 hergestellt wird.

**20.** Verwendung einer Verbindung oder Mischung nach einem der Ansprüche 1 bis 17 als halbleitendes, Ladungstransport-, elektrisch leitendes, lichtleitendes oder lichtemittierendes Material oder in einer optischen, elektrooptischen, elektronischen, elektrolumineszierenden oder fotolumineszierenden Vorrichtung oder in einem Bauteil einer solchen Vorrichtung oder in einer Baugruppe, die eine solche Vorrichtung oder Baugruppe umfasst.

**21.** Halbleitendes, Ladungstransport-, elektrisch leitendes, lichtleitendes oder lichtemittierendes Material, das eine Verbindung oder Mischung nach einem der Ansprüche 1 bis 17 umfasst.

**22.** Optische, elektrooptische, elektronische, elektrolumineszierende oder fotolumineszierende Vorrichtung oder Bauteil davon oder sie umfassende Baugruppe, die eine Verbindung oder Mischung nach einem der Ansprüche 1 bis 17 umfasst oder ein halbleitendes, Ladungstransport-, elektrisch leitendes, lichtleitendes oder lichtemittierendes Material nach Anspruch 21 umfasst.

**23.** Optische, elektrooptische, elektronische, elektrolumineszierende oder fotolumineszierende Vorrichtung nach Anspruch 22, die ausgewählt ist aus organischen Feldeffekttransistoren (OFET), organischen Dünnschichttransistoren (OTFT), organischen lichtemittierenden Dioden (OLED), organischen lichtemittierenden Transistoren (OLET), organischen Fotovoltaikvorrichtungen (OPV), organischen Fotodetektoren (OPD), organischen Solarzellen, farbstoffsensibilisierten Solarzellen (DSSC), perowskitbasierten Solarzellen, Laserdioden, Schottky-Dioden, Fotoleitern und Fotodetektoren.

**24.** Bauteil nach Anspruch 22, das ausgewählt ist aus Ladungsinjektionsschichten, Ladungstransportschichten, Zwischenschichten, Planarisierungsschichten, antistatischen Folien, Polymerelektrolytmembranen (PEM), leitenden Substraten und leitenden Mustern.

**25.** Baugruppe nach Anspruch 22, die ausgewählt ist aus integrierten Schaltungen (IC), Radiofrequenzidentifikations (RFID)-Tags oder Sicherheitskennzeichnungen oder sie enthaltenden Sicherheitsvorrichtungen, Flachbildschirmanzeigen oder deren Hintergrundbeleuchtungen, elektrofotografischen Vorrichtungen, elektrofotografischen Aufzeichnungsvorrichtungen, organischen Speichervorrichtungen, Sensorvorrichtungen, Biosensoren und Biochips.

**26.** Bulk-Heteroübergang, der eine Mischung nach einem der Ansprüche 15 bis 17 umfasst oder daraus gebildet ist.

**27.** Bulk-Heteroübergang (BHJ)-OPV-Vorrichtung oder invertierte BHJ-OPV-Vorrichtung, umfassend einen Bulk-Heteroübergang nach Anspruch 26.

## Revendications

**1.** Composé comprenant une ou plusieurs unités hétéroarylène divalentes de formule I

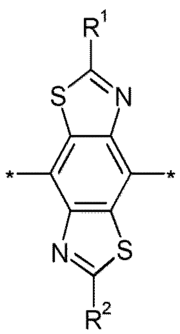

I

dans lequel les radicaux individuels, indépendamment les uns des autres et à chaque occurrence de manière identique ou différente, présentent les significations suivantes
$R^1$, $R^2$ sont sélectionnés à partir du groupe consistant en -C(=O)-R, -C(=O)-OR, - OC(=O)-R, -C(=O)-NHR et -C(=O)-NRR$^n$, dans lequel R et R$^n$ sont, indépendamment l'un de l'autre, un alkyle linéaire ou ramifié avec 1 à 20 atomes de C, qui est non substitué ou substitué avec un ou plusieurs atomes de F,
L est F, Cl, -CN, -NC, -NCO, -NCS, -OCN, -SCN, -$R^0$, -$OR^0$, -$SR^0$, -C(=O)$X^0$, -C(=O)$R^0$, - C(=O)-$OR^0$, -O-C(=O)$R^0$, -$NH_2$, -$NHR^0$, -$NR^0R^{00}$, -C(=O)$NHR^0$, -C(=O)$NR^0R^{00}$, -$SO_3H$, -$SO_2R^0$, - OH, -$NO_2$, -$CF_3$, $SF_5$, ou un silyle facultativement substitué, ou un carbyle ou un hydrocarbyle avec 1 à 20 atomes de C qui est facultativement substitué et comprend facultativement un ou plusieurs hétéroatomes,
$R^0$, $R^{00}$ sont H ou un alkyle linéaire ou ramifié avec 1 à 20 atomes de C qui est facultativement fluoré,
et comprenant en outre une ou plusieurs unités arylène ou hétéroarylène qui présentent de 5 à 20 atomes de cycle, sont mono- ou polycycliques, contiennent facultativement des cycles condensés, sont non substituées ou substituées avec un ou plusieurs groupes L identiques ou différents, et sont soit sélectionnées de formule I, soit sont structurellement différentes de la formule I, dans lequel toutes les unités susmentionnées sont directement liées les unes aux autres,
et dans lequel le composé est un polymère conjugué.

**2.** Composé selon la revendication 1, dans lequel $R^1$ et $R^2$ dans les unités de formule I désignent -C(=O)-OR, dans

lequel R est un alkyle linéaire ou ramifié avec 1 à 20 atomes de C qui est facultativement fluoré.

3. Composé selon la revendication 1, dans lequel L désigne F ou est sélectionné à partir des groupes suivants

   - le groupe consistant en R, -OR et -SR, dans lequel R est un alkyle linéaire ou ramifié avec 1 à 20 atomes de C qui est non substitué ou substitué avec un ou plusieurs atomes de F,
   - le groupe consistant en -C(=O)-R, -C(=O)-OR, OC(=O)-R, -C(=O)-NHR et -C(=O)-NRR$^n$, dans lequel R et R$^n$ sont, indépendamment l'un de l'autre, un alkyle linéaire ou ramifié avec 1 à 20 atomes de C qui est facultativement fluoré.

4. Composé selon l'une quelconque des revendications 1 à 3, qui est un polymère conjugué comprenant une ou plusieurs unités de formule I telles que définies selon l'une quelconque des revendications 1 à 3, et comprenant en outre une ou plusieurs unités arylène ou hétéroarylène qui présentent de 5 à 20 atomes de cycle, sont mono- ou polycycliques, contiennent facultativement des cycles condensés, sont non substituées ou substituées avec un ou plusieurs groupes L identiques ou différents, et sont soit sélectionnées de formule I, soit sont structurellement différentes de la formule I, et dans lequel toutes les unités susmentionnées sont directement liées les unes aux autres.

5. Composé selon la revendication 4, qui comprend une ou plusieurs unités de répétition de formule II1 ou II2, et facultativement une ou plusieurs unités de répétition de formule II3 :

$$-(Ar^1)_a-U-(Ar^2)_b-(Ar^3)_c-(Ar^4)_d- \qquad \text{II1}$$

$$-(Ar^1)_a-(Ar^2)_b-U-(Ar^3)_c-(Ar^4)_d- \qquad \text{II2}$$

$$-(Ar^1)_a-(Ar^2)_b-(Ar^3)_c-(Ar^4)_d- \qquad \text{II3}$$

dans lequel les radicaux individuels, indépendamment les uns des autres et à chaque occurrence de manière identique ou différente, présentent les significations suivantes

U est une unité de formule I telle que définie selon l'une quelconque des revendications 1 à 3,
Ar$^{1-4}$ sont un arylène ou un hétéroarylène qui présente 5 à 20 atomes de cycle, est mono- ou polycyclique, contient facultativement des cycles condensés, est non substitué ou substitué avec un ou plusieurs groupes L identiques ou différents tels que définis selon la revendication 1 ou 3, et est différent de la formule I,
a, b, c, d sont 0 ou 1, dans lequel, dans la formule II3, $a + b + c + d \geq 1$.

6. Composé selon la revendication 4 ou 5, qui est sélectionné de formule III :

$$*\!-\!\left[-(A)_x-(B)_y-\right]_n\!-\!* \qquad \text{III}$$

dans lequel

A est une unité de formule I, II1 ou II2 telle que définie selon l'une quelconque des revendications 1 à 5,
B est une unité de formule I, II1, II2 ou II3 telle que définie selon l'une quelconque des revendications 1 à 5,
x est $> 0$ et $\leq 1$,
y est $\geq 0$ et $< 1$,
$x + y$ est 1, et
n est un nombre entier $\geq 5$.

7. Composé selon l'une quelconque des revendications 4 à 6, qui est sélectionné parmi les formules suivantes

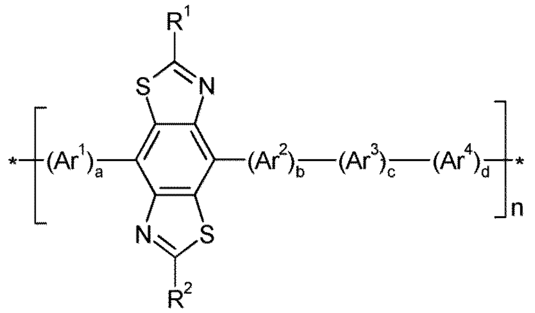

III1

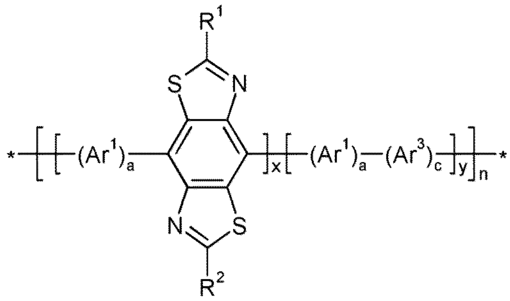

III2

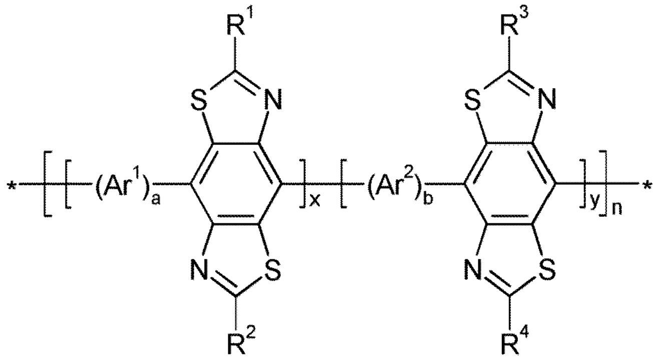

III3

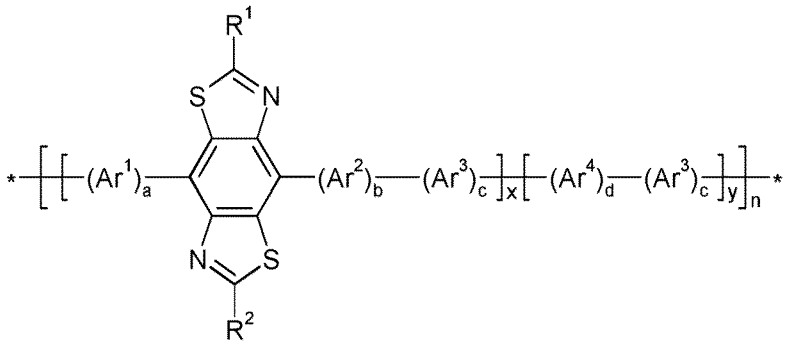

III4

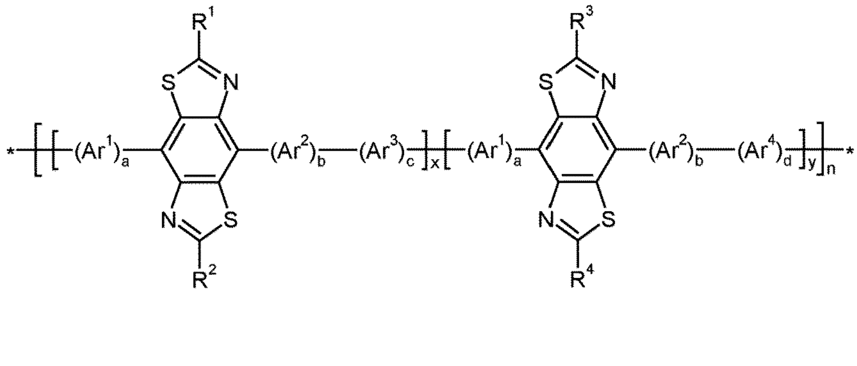

III5

dans lequel $R^1$ et $R^2$ présentent les significations selon l'une quelconque des revendications 1 ou 2, $R^3$ et $R^4$ présentent une des significations de $R^1$ tel que défini selon les revendications 1 ou 2, $Ar^1$, $Ar^2$, $Ar^3$, $Ar^4$, a, b, c et d présentent les significations selon la revendication 5, et x, y et n présentent les significations selon la revendication 6.

**8.** Composé selon la revendication 6 ou 7, qui est sélectionné de formule IV

$R^5$-chaîne-$R^6$ IV

dans lequel « chaîne » désigne une chaîne de polymère sélectionnée parmi les formules III et III1-III5 telles que définies selon les revendications 6 et 7, et $R^5$ et $R^6$ présentent, indépendamment l'un de l'autre, une des significations de L tel que défini selon la revendication 1 ou 3, ou désignent, indépendamment l'un de l'autre, H, F, Br, Cl, I, $-CH_2Cl$, -CHO, -CR'=CR"$_2$, -SiR'R"R‴, -SiR'X'X", -SiR'R"X', -SnR'R"R‴, -BR'R", - B(OR')(OR"), $-B(OH)_2$, $-O-SO_2$-R', -C≡CH, -C≡C-SiR'$_3$, -ZnX' ou un groupe d'extrémité, X' et X" désignent un halogène, R', R" et R‴ présentent, indépendamment les uns des autres, une des significations de $R^0$ données dans la revendication 1, et deux de R', R" et R‴ peuvent également former un groupe cyclosilyle, cyclostannyle, cycloborane ou cycloboronate avec 2 à 20 atomes de C conjointement avec l'hétéroatome respectif auquel ils sont liés.

**9.** Composé selon l'une quelconque des revendications 5 à 8, dans lequel un ou plusieurs parmi $Ar^1$, $Ar^2$, $Ar^3$ et $Ar^4$ désignent un arylène ou un hétéroarylène sélectionné à partir du groupe consistant en les formules suivantes

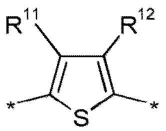
(D1)

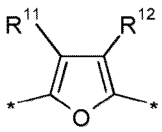
(D7)

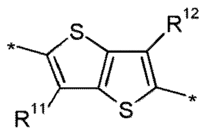
(D10)

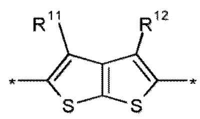
(D11)

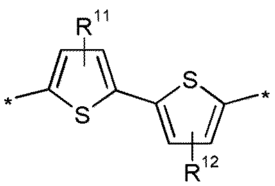
(D19)

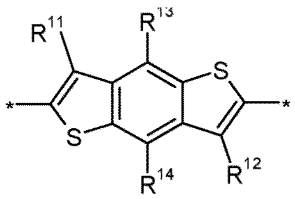
(D22)

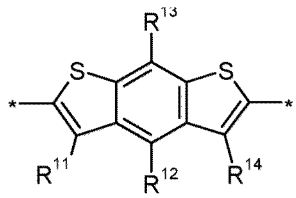
(D29)

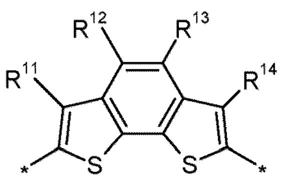
(D30)

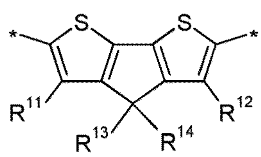
(D35)

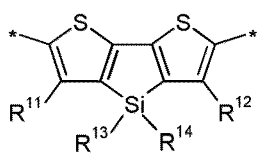
(D36)

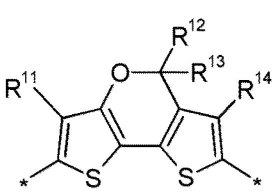
(D44)

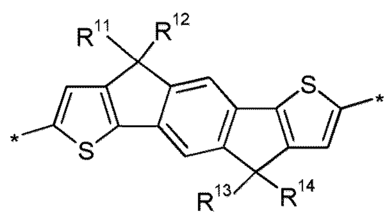
(D55)

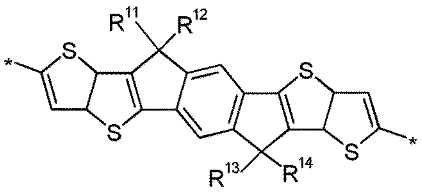

(D84)

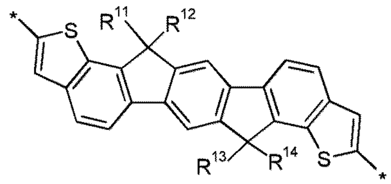

(D87)

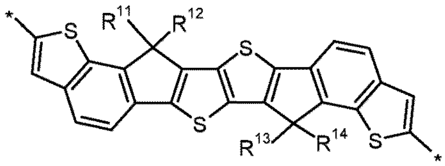

(D88)

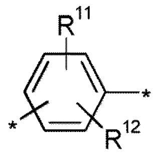

(D94)

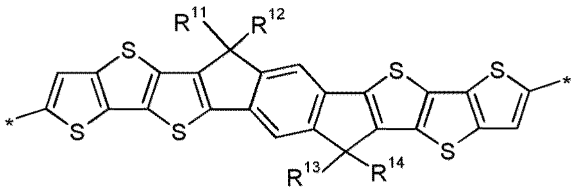

(D89)

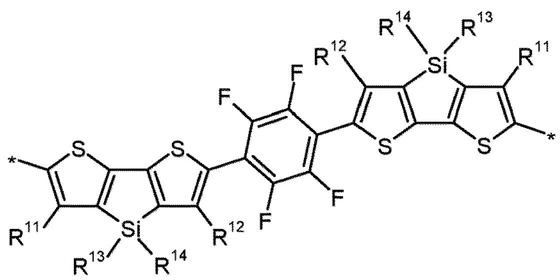

(D93)

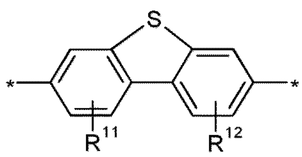

(D106)

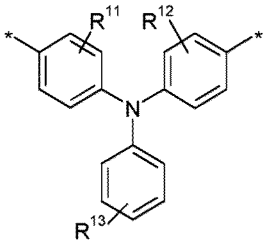

(D111)

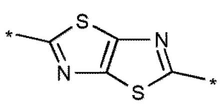

(D139)

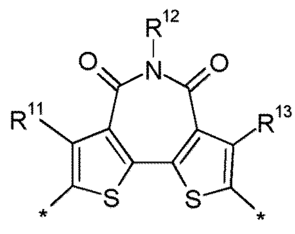

(D140)

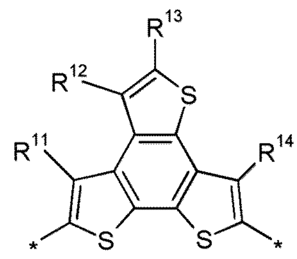

(D141)

dans lequel $R^{11}$, $R^{12}$, $R^{13}$, $R^{14}$, indépendamment les uns des autres, désignent H ou présentent une des significations de L tel que défini selon la revendication 1 ou 3.

**10.** Composé selon l'une quelconque des revendications 5 à 9, dans lequel un ou plusieurs parmi $Ar^1$, $Ar^2$, $Ar^3$ et $Ar^4$ désignent un arylène ou un hétéroarylène sélectionné à partir du groupe consistant en les formules suivantes

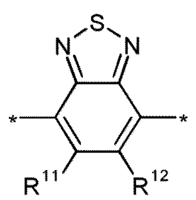

(A1)

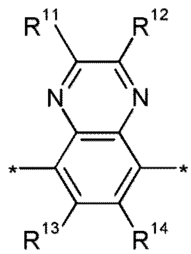

(A6)

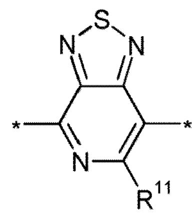

(A7)

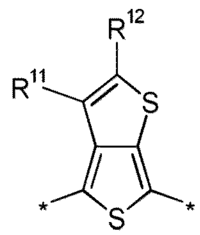

(A15)

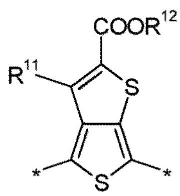

(A16)

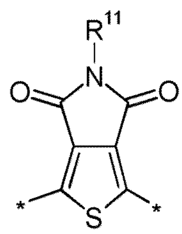

(A20)

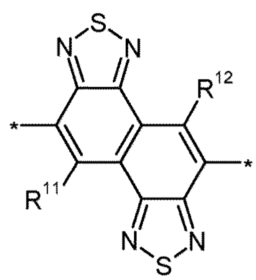

(A74)

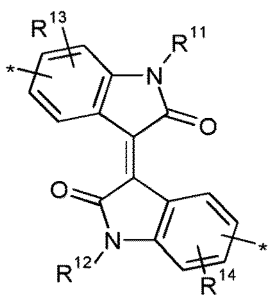

(A84)

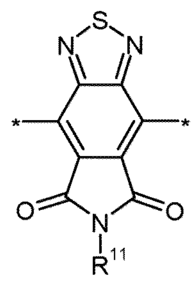

(A88)

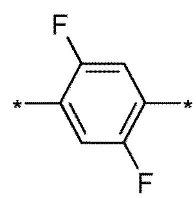

(A92)

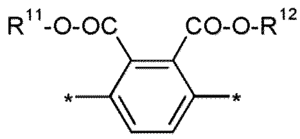

(A98)

dans lequel $R^{11}$, $R^{12}$, $R^{13}$, $R^{14}$, indépendamment les uns des autres, désignent H ou présentent une des significations de L tel que défini selon la revendication 1 ou 3.

**11.** Composé selon l'une quelconque des revendications 5 à 9, dans lequel un ou plusieurs parmi $Ar^1$, $Ar^2$, $Ar^3$ et $Ar^4$ désignent un arylène ou un hétéroarylène sélectionné à partir du groupe consistant en les formules suivantes

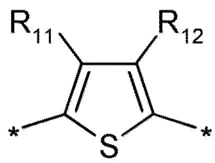

Sp1

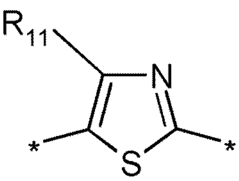

Sp2

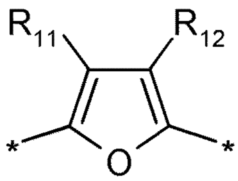

Sp3

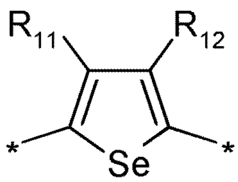

Sp4

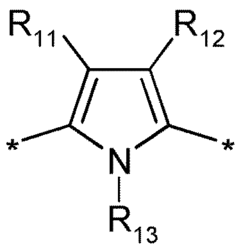

Sp5

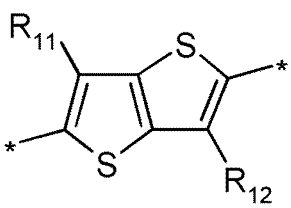

Sp6

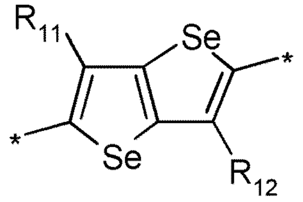

Sp7

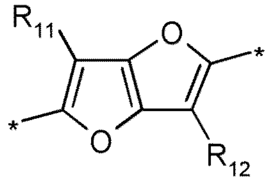

Sp8

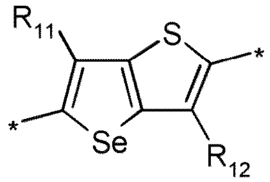

Sp9

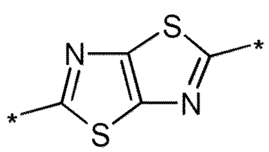

Sp10

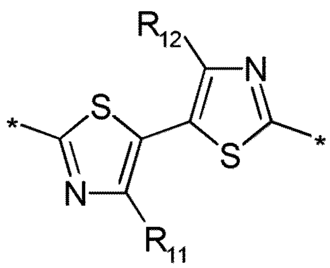

Sp11

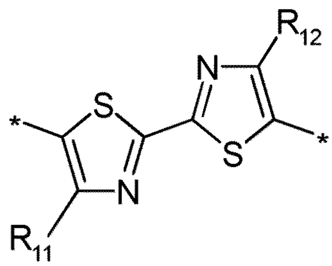

Sp12

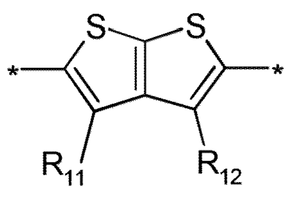

Sp13

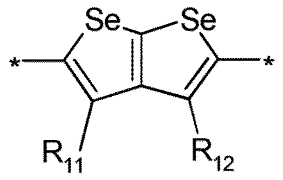

Sp14

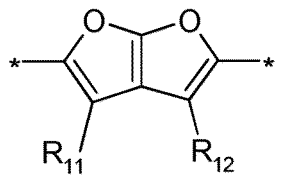

Sp15

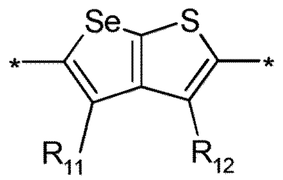

Sp16

dans lequel $R^{11}$ et $R^{12}$, indépendamment l'un de l'autre, désignent H ou présentent une des significations de L tel que défini selon la revendication 1 ou 3.

**12.** Composé selon l'une quelconque des revendications 5 à 11, dans lequel

a) un ou plusieurs parmi $Ar^1$, $Ar^2$, $Ar^3$ et $Ar^4$ désignent un arylène ou un hétéroarylène sélectionné à partir du groupe consistant en les formules D1, D7, D10, D11, D19, D22, D29, D30, D35, D36, D44, D55, D84, D87, D88, D89, D93, D94, D106, D111, D139, D140 et D141 telles que définies selon la revendication 9, et/ou
b) un ou plusieurs parmi $Ar^1$, $Ar^2$, $Ar^3$ et $Ar^4$ désignent un arylène ou un hétéroarylène sélectionné à partir du groupe consistant en les formules A1, A6, A7, A15, A16, A20, A74, A84, A88, A92 et A98 telles que définies selon la revendication 10,
et
c) un ou plusieurs parmi $Ar^1$, $Ar^2$, $Ar^3$ et $Ar^4$ désignent un arylène ou un hétéroarylène sélectionné à partir du groupe consistant en les formules Sp1, Sp6, Sp10 et Sp13 telles que définies selon la revendication 11.

**13.** Composé de formule VI

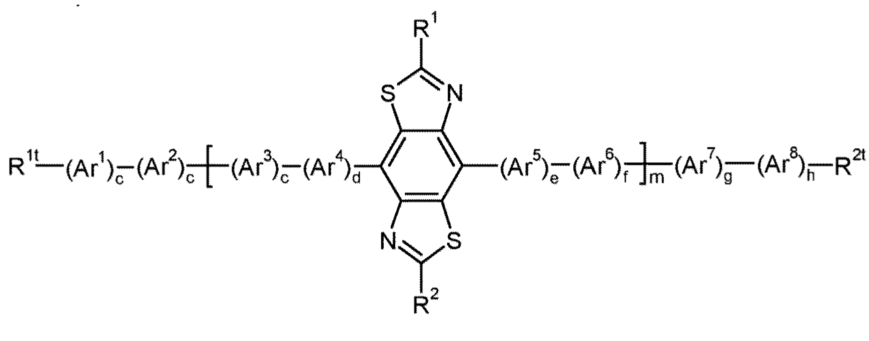

VI

dans lequel les radicaux individuels, indépendamment les uns des autres et à chaque occurrence de manière identique ou différente, présentent les significations suivantes
$Ar^{1-8}$ sont une des significations données pour $Ar^1$ dans la revendication 5, ou une unité de formule I définie selon la revendication 1, ou $-CY^1=CY^2-$,
$Y^1$, $Y^2$ sont H, F, Cl ou CN,
$R^{1t,2t}$ sont H, F, Cl, Br, -CN, $-CF_3$, R*, $-CF_2R^*$, -O-R*, -S-R*, $-SO_2-R^*$, $-SO_3-R^*$, -C(=O)-R*, -C(=S)-R*, $-C(=O)-CF_2R^*$, -C(=O)-OR*, -C(=S)-OR*, -O-C(=O)-R*, -O-C(=S)-R*, -C(=O)-SR*, -S-C(=O)-R*, -C(=O)NR*R**, -NR*-C(=O)-R*, -NHR*, -NR*R**, -CR*=CR*R**, -C≡C-R*, -C≡C-SiR*R**R***, -SiR*R**R***,

-CH=C(CN)-C(=O)-OR*, -CH=C(CO-OR*)$_2$, -CH=C(CO-NR*R**)$_2$, -CH=C(CN)(Ar$^9$),

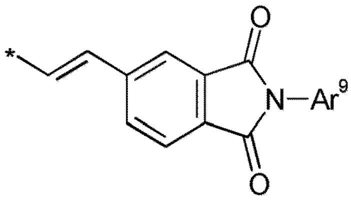
,
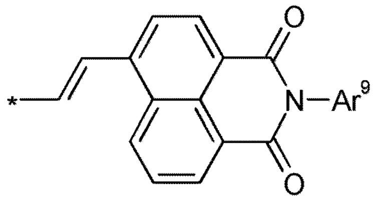
,

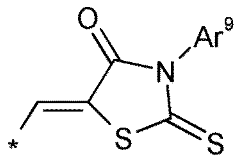
,
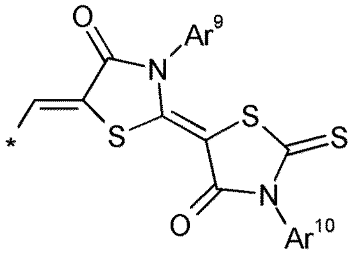
,

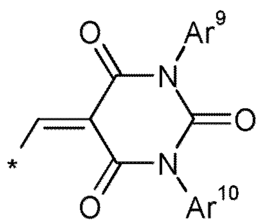
,
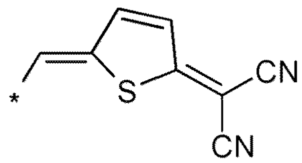
,

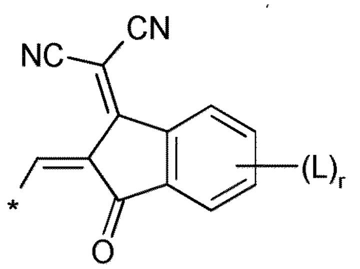
,

Ar$^{9,10}$ sont un aryle ou un hétéroaryle, chacun présentant de 4 à 30 atomes de cycle, facultativement contenant des cycles condensés et étant non substitué ou substitué avec un ou plusieurs groupes L tels que définis selon la revendication 1,

R*, R**, R*** sont un alkyle avec 1 à 20 atomes de C qui est linéaire, ramifié ou cyclique, et est non substitué, substitué avec un ou plusieurs atomes de F ou Cl ou groupes CN, ou perfluoré, et dans lequel un ou plusieurs atomes de C sont facultativement remplacés par -O-, -S-, -C(O)-, -C(S)-, -SiR$^0$R$^{00}$-, -NR$^0$R$^{00}$-, -CHR$^0$=CR$^{00}$- ou - C≡C- de sorte que des atomes O et/ou S ne sont pas directement liés les uns aux autres,

R$^0$, R$^{00}$ sont H ou un alkyle linéaire ou ramifié avec 1 à 20 atomes de C qui est facultativement fluoré,

a-h sont 0 ou 1, avec au moins l'un de a-h étant 1,

m est 1, 2 ou 3,

R$^1$ et R$^2$ sont tels que définis selon la revendication 1,

à condition que m > 1.

**14.** Composé selon la revendication 13, dans lequel Ar$^{1-10}$ sont sélectionnés à partir des groupes suivants

a) le groupe consistant en les formules D1, D7, D10, D11, D19, D22, D29, D30, D35, D36, D44, D55, D84, D87, D88, D89, D93, D94, D106, D111, D139, D140 et D141 telles que définies selon la revendication 9,

b) le groupe consistant en les formules A1, A6, A7, A15, A16, A20, A74, A84, A88, A92 et A98 telles que définies

selon la revendication 10,
c) le groupe consistant en les formules Sp1, Sp6, Sp10 et Sp13 telles que définies selon la revendication 11.

**15.** Mélange comprenant un ou plusieurs composés selon l'une quelconque des revendications 1 à 14 et un ou plusieurs composés supplémentaires présentant une ou plusieurs parmi des propriétés de semi-conductivité, de transport de charge, de transport de trous ou d'électrons, de blocage de trous ou d'électrons, de conduction électrique, de photoconduction ou d'électroluminescence.

**16.** Mélange comprenant un ou plusieurs composés selon l'une quelconque des revendications 1 à 14 et un ou plusieurs semiconducteurs organiques de type n.

**17.** Mélange selon la revendication 16, dans lequel les semiconducteurs organiques de type n sont sélectionnés parmi les fullerènes ou les fullerènes substitués.

**18.** Formulation comprenant un ou plusieurs composés ou mélanges selon l'une quelconque des revendications 1 à 17, et comprenant en outre un ou plusieurs solvants sélectionnés parmi les solvants organiques.

**19.** Dispositif optique, électro-optique, électronique, électroluminescent ou photoluminescent, ou composant de celui-ci, ou ensemble le comprenant, qui est préparé en utilisant une formulation selon la revendication 18.

**20.** Utilisation d'un composé ou d'un mélange selon l'une quelconque des revendications 1 à 17 comme matériau semi-conducteur, de transport de charge, de conduction électrique, de photoconduction ou d'électroluminescence, ou dans un dispositif optique, électro-optique, électronique, électroluminescent ou photoluminescent, ou dans un composant d'un tel dispositif ou dans un ensemble comprenant un tel dispositif ou composant.

**21.** Matériau semi-conducteur, de transport de charge, de conduction électrique, de photoconduction ou d'électroluminescence comprenant un composé ou un mélange selon l'une quelconque des revendications 1 à 17.

**22.** Dispositif optique, électro-optique, électronique, électroluminescent ou photoluminescent, ou composant de celui-ci, ou ensemble le comprenant, qui comprend un composé ou un mélange selon l'une quelconque des revendications 1 à 17, ou comprend un matériau semi-conducteur, de transport de charge, de conduction électrique, de photoconduction ou d'électroluminescence selon la revendication 21.

**23.** Dispositif optique, électro-optique, électronique, électroluminescent ou photoluminescent selon la revendication 22, qui est sélectionné parmi les transistors organiques à effet de champ (OFET), les transistors organiques à couche mince (OTFT), les diodes électroluminescentes organiques (OLED), les transistors électroluminescents organiques (OLET), les dispositifs photovoltaïques organiques (OPV), les photodétecteurs organiques (OPD), les cellules solaires organiques, les cellules solaires à pigment photosensible (DSSC), les cellules solaires à base de pérovskite, les diodes laser, les diodes Schottky, les photoconducteurs et les photodétecteurs.

**24.** Composant selon la revendication 22, qui est sélectionné parmi les couches d'injection de charge, les couches de transport de charge, les couches intermédiaires, les couches planarisantes, les films antistatiques, les membranes électrolytes polymères (PEM), les substrats conducteurs et les motifs conducteurs.

**25.** Ensemble selon la revendication 22, qui est sélectionné parmi les circuits intégrés (IC), les étiquettes de radio-identification (RFID) ou les marquages de sécurité ou les dispositifs de sécurité les contenant, les affichages à écran plat ou les rétroéclairages de ceux-ci, les dispositifs électrophotographiques, les dispositifs d'enregistrement électrophotographique, les dispositifs à mémoire organique, les dispositifs de détection, les biocapteurs et les biopuces.

**26.** Hétérojonction en volume qui comprend, ou est formée à partir de, un mélange selon l'une quelconque des revendications 15 à 17.

**27.** Dispositif OPV à hétérojonction en volume (BHJ) ou dispositif OPV à BHJ inversée, comprenant une hétérojonction en volume selon la revendication 26.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007238530 B
- KR 2010088764
- WO 2011098113 A1
- WO 2012156022 A1
- WO 2013013765 A1
- WO 2015122321 A1
- WO 2016125822 A1
- WO 2016121589 A1
- WO 0053656 A1
- WO 2004022626 A1
- WO 03048225 A2
- WO 2005014688 A2
- WO 2005055248 A1
- WO 2013142841 A1
- WO 2012095796 A1
- WO 2013021971 A1
- US 5892244 A
- US 5998804 A
- US 6723394 B
- US 20070102696 A1
- US 7095044 B
- EP 0889350 A1
- EP 0528662 A
- US 5198153 A
- WO 9621659 A
- US 20030021913 A
- US 20030021913 A1


### Non-patent literature cited in the description


- **KIM et al.** *Synth. Met.,* 2006, vol. 156 (1), 38-41
- **KIM et al.** *Pol. Preprints,* 2003, vol. 44 (1), 931-932
- *Pure Appl. Chem.,* 1996, vol. 68, 2291
- *International Union of Pure and Applied Chemistry, Compendium of Chemical Technology, Gold Book,* 19 August 2012, 477, , 480
- **J. THEWLIS.** Concise Dictionary of Physics. Pergamon Press, 1973
- *Pure Appl. Chem.,* 1994, vol. 66, 1134
- **J. M. G. COWIE.** Polymers: Chemistry & Physics of Modern Materials. Blackie, 1991
- *J. Chem. Soc., Chem. Commun.,* 1977, 683-684
- **T. YAMAMOTO et al.** *Prog. Polym. Sci.,* 1993, vol. 17, 1153-1205
- **Z. BAO et al.** *J. Am. Chem. Soc.,* 1995, vol. 117, 12426-12435
- **M. LECLERC et al.** *Angew. Chem. Int. Ed.,* 2012, vol. 51, 2068-2071
- **MALIKA JEFFRIES-EL.** *J. Org. Chem.,* 2010, vol. 75 (2), 495-497
- **PERSEPHONIS et al.** *Org. Lett.,* 2014, vol. 16 (24), 6358-6361
- **CROWLEY, J.D. ; TEAGUE, G.S. JR ; LOWE, J.W. JR.** *Journal of Paint Technology,* 1966, vol. 38 (496), 296
- **W.H.ELLIS.** Solvents. Federation of Societies for Coatings Technology, 1986, 9-10
- *Science,* 1995, vol. 270, 1789
- **COAKLEY, K. M. ; MCGEHEE, M. D.** *Chem. Mater.,* 2004, vol. 16, 4533
- *Adv. Mater.,* 2013, vol. 25 (17), 2385-2396
- *Adv. Ener. Mater.*
- *Adv. Mater.,* 2013, vol. 25 (48), 7038-7044
- *J. Mater. Chem.,* 2011, vol. 21, 12331
- *J. Appl. Phys.,* 2013, vol. 113, 124509
- *Adv. Energy Mater.,* 2012, vol. 2, 82-86
- **WALDAUF et al.** *Appl. Phys. Lett.,* 2006, vol. 89, 233517
- **DENNLER et al.** *Proceedings of the IEEE,* 2005, vol. 93 (8), 1429
- **HOPPE et al.** *Adv. Func. Mater,* 2004, vol. 14 (10), 1005
- **J. PEET et al.** *Nat. Mater.,* 2007, vol. 6, 497
- **FRÉCHET et al.** *J. Am. Chem. Soc.,* 2010, vol. 132, 7595-7597
- **MÜLLER et al.** *Synth. Metals,* 2000, vol. 111-112, 31-34
- **ALCALA.** *J. Appl. Phys.,* 2000, vol. 88, 7124-7128
- **C. WEDER et al.** *Science,* 1998, vol. 279, 835-837
- **KOLLER et al.** *Nat. Photonics,* 2008, vol. 2, 684
- **L. CHEN ; D. W. MCBRANCH ; H. WANG ; R. HELGESON ; F. WUDL ; D. G. WHITTEN.** *Proc. Natl. Acad. Sci. U.S.A.,* 1999, vol. 96, 12287
- **D. WANG ; X. GONG ; P. S. HEEGER ; F. RININSLAND ; G. C. BAZAN ; A. J. HEEGER.** *Proc. Natl. Acad. Sci. U.S.A.,* 2002, vol. 99, 49

- **N. DICESARE ; M. R. PINOT ; K. S. SCHANZE ; J. R. LAKOWICZ.** *Langmuir,* 2002, vol. 18, 7785
- **D. T. MCQUADE ; A. E. PULLEN ; T. M. SWAGER.** *Chem. Rev.,* 2000, vol. 100, 2537